# EUROPEAN PATENT SPECIFICATION

(11) **EP 1 920 775 B1**
(45) Date of publication and mention of the grant of the patent: **19.12.2012**
(21) Application number: 06021271.9
(22) Date of filing: 10.10.2006
(51) Int. Cl.: C12N 15/117, A61K 31/7115, A61P 37/04, A61P 31/12, A61P 31/04, A61P 35/00, A61P 37/06, A61P 37/08, C07H 21/00

(54) **5'Triphosphate oligonucleotide induces anti-viral response**
5' Triphosphat -oligonukleotid induziert eine antivirale Reaktion
Oligonucleotide 5'triphosphate induisant une réaction antivirale

(43) Date of publication of application: 14.05.2008
(60) Divisional application: 10015841.9
(73) Proprietor: Hartmann, Gunther, Prof. Dr., 53127 Bonn (DE)
(72) Inventor: Hartmann, Gunther, Prof. Dr., 53347 Alfter (Impekoven) (DE); Hornung, Veit, Dr., 82049 Pullach (DE)
(74) Representative: Zimmer, Franz-Josef

(56) References cited:
- EP-A2- 0 031 285
- WO-A-96/40159
- US-A1- 2006 178 334
- KIM DONG-HO ET AL: "Interferon induction by siRNAs and ssRNAs synthesized by phage polymerase" NAT. BIOTECHNOL.; NATURE BIOTECHNOLOGY MARCH 2004, vol. 22, no. 3, March 2004 (2004-03), pages 321-325, XP002428031
- SCHLEE M ET AL: "siRNA and isRNA: two edges of one sword" MOLECULAR THERAPY, ACADEMIC PRESS, SAN DIEGO, CA,, US, vol. 14, no. 4, 2006, pages 463-470, XP002416372 ISSN: 1525-0016
- HORNUNG VEIT ET AL: "5'-Triphosphate RNA is the ligand for RIG-I." SCIENCE (NEW YORK, N.Y.) 10 NOV 2006, vol. 314, no. 5801, 10 November 2006 (2006-11-10), pages 994-997, XP002428032 ISSN: 1095-9203
- PICHLMAIR ANDREAS ET AL: "RIG-I-mediated antiviral responses to single-stranded RNA bearing 5'-phosphates." SCIENCE (NEW YORK, N.Y.) 10 NOV 2006, vol. 314, no. 5801, 10 November 2006 (2006-11-10), pages 997-1001, XP009081711 ISSN: 1095-9203
- BOWIE A.G. ET AL: "RIG - I : tri-ing to discriminate between self and non-self RNA." TRENDS IN IMMUNOLOGY, 28/4 (147-150). REFS: 40. CODEN: TIRMA ISSN: 1471-4906 PUI - S 1471-4906(07)00028-2, April 2007 (2007-04), XP009081714

## Description

### Field of the Invention

The present invention relates to the field of immunotherapy and drug discovery. The present invention provides oligonucleotides which are capable of inducing an anti-viral or an anti-bacterial response, in particular, the production of type I IFN, IL-18 and/or IL-1β, and their *in vitro* as well as therapeutic uses.

### Background of the Invention

The vertebrate immune system established different ways to detect invading pathogens based on certain characteristics of their microbial nucleic acids. Detection of microbial nucleic acids alerts the immune system to mount the appropriate type of immune response that is required for the defense against the respective type of pathogen detected. Detection of viral nucleic acids leads to the production of type I interferon (IFN) including IFN-α and IFN-β, the key cytokines for anti-viral defense.

IFN-α was the first type of interferon to be identified and commercialized; it is widely used clinically in the treatment of a variety of tumors (e.g., hairy cell leukemia, cutaneous T cell leukemia, chronic myeloid leukemia, non-Hodgkin's lymphoma, AIDS-related Kaposi's sarcoma, malignant melanoma, multiple myeloma, renal cell carcinoma, bladder cell carcinoma, colon carcinoma, cervical dysplasia) and viral diseases (e.g., chronic hepatitis B, chronic hepatitis C). IFN-α products that are currently in clinical use include the recombinant protein and the highly purified natural protein, both of which have high production costs. Therefore, there is a need for more economical ways of providing IFN-α to patients in need. Furthermore, IFN-α is currently administrated systematically and causes a broad spectrum of side effects (e.g. fatigue, flu-like symptoms, diarrhea). Most alarmingly, IFN-α causes a decrease in bone marrow function which leads to increased susceptibility to life-threatening infections, anemia and bleeding problems. Therefore, there is a need for ways of providing IFN-α in a more localized (i.e., target-specific) matter to reduce the occurrence of side effects.

Receptor-mediated detection of pathogen-derived nucleic acids assists in protecting the host genome from invading foreign genetic material. A new picture is evolving in which the ability of biological systems to detect viral nucleic acids via protein receptor-nucleic acid ligand interactions is crucial for maintaining the integrity of the genome and for survival.

A number of receptor proteins have evolved that take part in nucleic acid recognition. Recent studies indicate that one of the most important protein receptors for antiviral defense is the retinoic-acid-inducible protein I (RIG-I), a member of the helicase family containing two caspase-recruitment domains (CARDs) and a DExD/H-box helicase domain (M. Yoneyama et al., Nat Immunol 5, 730 (Jul, 2004)). RIG-I-mediated recognition of a specific set of RNA viruses (flaviviridae, paramyxoviridae, orthomyxoviridae and rhabdoviridae) (M. Yoneyama et al., Nat Immunol 5, 730 (Jul, 2004); R. Sumpter, Jr. et al., J Virol 79, 2689 (Mar, 2005); H. Kato et al., Nature 441, 101 (Apr 9, 2006)) has a critical role in antiviral host defense *in vitro* and *in vivo.* A second member of the the helicase family, MDA-5, is responsible for the antiviral defense against a reciprocal set of RNA viruses (picornaviridae)(H. Kato et al., Nature 441(7089):101-105, Apr 9, 2006).

In addition to RIG-I and MDA-5, the four members of the Toll-like receptor (TLR) family, TLR3, TLR7, TLR9 and TLR9, are also known to be involved in viral nucleic acid recognition. RIG-I and MDA-5 differ from the TLRs in their subcellular localization, expression patem, signal transduction pathways and ligands.

While RIG-I and MDA-5 are cytosolic receptors, TLR3, TLR7, TLR8 and TLR9 are located in the endosomal membrane.

While TLRs are mainly expressed on certain defined immune cell subsets (i.e. TLR9 restricted to PDC and B cells), RIG-I and MDA-5 are expressed in both immune and non-immune cells (H. Kato et al., Immunity 23, 19 (Jul, 2005)).

Besides distinct expression profiles and cellular localization, signalling of endosomal TLRs and the two cytoplasmic receptors RIG-I and MDA-5 differs. While TLR3 signals via TRIF and TLR7, TLR8 and TLR9 signal via MyD88, RIG-I recruits a CARD-containing adaptor, IPS-1 (T. Kawai et al., Nat Immunol 6, 981 (Oct, 2005)) (also known as MAVS (R. B. Seth et al., Cell 122, 669 (Sep 9, 2005)), VISA (L. G. Xu et al., Mol Cell 19, 727 (Sep 16, 2005)) or Cardif (E. Meylan et al., Nature 437, 1167 (Oct 20, 2005))). IPS-1 relays the signal to the kinases TBK1 and IKK-i, which phosphorylate interferon-regulatory factor-3 (IRF-3) and IRF-7, transcription factors essential for the expression of type-I interferons. As a consequence, *in vivo,* endosomal and cytoplasmic nucleic acid receptors induce different cytokine patterns. For example, both TLR3 and MDA-5 contribute to IL-12 production in reponse to poly(I:C), while MDA-5 but not TLR3 is responsible for IFN-α induction (H. Kato et al., Nature 441, 101 (Apr 9, 2006)).

The ligand for TLR3 is long dsRNA such as poly(I:C) (L. Alexopoulou, et al., Nature 413, 732 (Oct 18, 2001)), for TLR7 ssRNA (S. S. Diebold et al., Science 303, 1529 (Mar 5, 2004); F. Heil et al., Science 303, 1526 (Mar 5, 2004)) and short dsRNA with certain sequence motifs (i.e., the immunostimulatory RNA, isRNA) (V. Homung et al., Nat Med 11, 263 (Mar, 2005)), and for TLR9 CpG DNA (A. M. Krieg et al., Nature 374, 546 (Apr 6, 1995); H.. Hemmi et al., Nature 408, 740 (Dec 7, 2000)).

In several studies, long double-stranded RNA was proposed to be the ligand for MDA-5 and RIG-I (M. Yoneyama et al., Nat Immunol 5, 730 (Jul, 2004); H. Kato et al., Nature 441, 101 (Apr 9, 2006); S: Rothenfusser et al., J Immunol 175, 5260 (Oct 15, 2005)). A synthetic mimic of long dsRNA is poly(I:C). Recent data showed that poly(I:C) is a ligand for MDA-5, while it is not recognized by RIG-I (H. Kato et al., Nature 441, 101 (Apr 9, 2006)). On the other hand, long dsRNA was found to activate RIG-I but not MDA-5 (H. Kato et al., Nature 441, 101 (Apr 9, 2006)). This discrepancy of long dsRNA and poly(I:C) activity suggests that there is more to cytoplasmic RNA recognition than long dsRNA.

In general, compartimentalization and different molecular structure are believed to contribute to the detection of foreign nucleic acids. DNA (G. M. Barton et al., Nat Immunol 7, 49 (Jan, 2006)) and RNA (F. Heil et al., Science 303, 1526 (Mar 5, 2004)) localized in the endosome or DNA localized in the cytoplasm (K. J. Ishii et al., Nat Immunol 7, 40 (Jan, 2006)) are recognized and thus interpreted as foreign. The frequency of so-called CpG motifs in microbial DNA serves as a molecular feature further improving distinction of self and non-self DNA in the endosome. Although RNA recognition in the endosome is sequence dependent (F. Heil et al., Science 303, 1526 (Mar 5, 2004); V. Homung et al., Nat Med 11, 263 (Mar, 2005)), no sequence motifs have been defined so far that serve as a molecular basis to improve distinction of self and non-self RNA (i.e. motifs that are more frequent in viral than in self RNA) in the cytoplasm. Instead, the molecular characteristic of double-strandedness seems to allow distinction of self and non-self RNA. In fact, in the endosome, long double-stranded RNA and its mimic poly(I:C), but not single-stranded RNA, are recognized via TLR3 (L. Alexopoulou, et al., Nature 413, 732 (Oct 18, 2001)). In the cytoplasm, abundant self RNA complicates our understanding of the recognition of non-self RNA. Nevertheless, the concept that long dsRNA in the cytoplasm is detected as non-self has never been questioned since the discovery of type I IFN.

Unlike in the absence of RIG-I and MDA-5, antiviral defense is largely maintained in the absence of TLRs (A. Krug et al., Immunity 21, 107 (Jul, 2004); K. Tabeta et al., Proc Natl Acad Sci U S A 101, 3516 (Mar 9, 2004); T. Delale et al., J Immunol 175, 6723 (Nov 15, 2005); K. Yang et al., Immunity 23, 465 (Nov, 2005)), underscoring the critical role of RIG-I and MDA-5 in antiviral responses.

It is therefore an object of the present invention to provide polynucleotides/oligonucleotides which are capable of stimulating an anti-viral response, in particular, a type I IFN response. It is another object of the present invention to provide a pharmaceutical composition capable of inducing an anti-viral response, in particular, type I IFN production, in a patient for the prevention and treatment of diseases and disorders such as viral infection and tumor.

A recent study demonstrated that *in vitro* transcribed siRNA (small-interfering RNA), but not synthetic siRNA, stimulated the production of IFN-α from selected cell lines (D. H. Kim et al., Nat Biotechnol 22, 321 (Mar, 2004)). However, the physiological relevance of this induction and the mechanism of detection remain unclear.

US2006/0178334 A1 discloses pharmaceutical compositions comprising 5' triphosphate-RNA for inducing interferon and inducing an antiviral response in vitro or in vivo. The therapy and prevention of diseases including viral infections and cancer are also disclosed

### Summary of the Invention

The invention relates to the following embodiments as defined in items 1 to 25
1. A pharmaceutical composition comprising an oligonucleotide wherein the oligonucleotide comprises a 5' triphosphate, wherein the oligonucleotide comprises at least 1, preferably at least 3, more preferably at least 6 ribonucleotide(s) at the 5' end, and wherein the oligonucleotide is at least 12, preferably at least 18, more preferably at least 20, and even more preferably at least 21 nucleotides in length, wherein the first ribonucleotide at the 5' end of the oligonucleotide comprises an adenine, wherein the oligonucleotide is free of modifications, said modifications being 2'-O-methylated NTP.
2. The pharmaceutical composition of item 1, wherein the sequence of the first 4 nucleotides at the 5' end of the oligonucleotide is selected from: AAGU, AAAG, AUGG, AUUA, AACG, AUGA, AGUU, AUUG, AACA, AGAA, AGCA, AACU, AUCG, AGGA, AUCA, AUGC, AGUA, AAGC, AACC, AGGU, AAAC, AUGU, ACUG, ACGA, ACAG, AAGG, ACAU, ACGC, AAAU, ACGG, AUUC, AGUG, ACAA, AUCC, AGUC, wherein the sequence listing is in the 5' -> 3' direction.
3. The pharmaceutical composition of any of items 1-2, wherein the oligonucleotide is furthermore free of pseudouridine, 2-thiouridine, and 2'-Fluorine-dNTP modifications.
4. The pharmaceutical composition of any of items 1-3, further comprising a complexation agent.
5. The pharmaceutical composition of any of items 1-3, wherein the oligonucleotide is comprised in a viral vector.
6. A combined preparation comprising an oligonucleotide as defined in any of items 1-3 and an immunostimulatory agent, wherein the oligonucleotide and the agent are for simultaneous, separate or sequential administration.
7. The combined preparation of item 6, further comprising an anti-viral agent and/or anti-bacterial agent and/or anti-tumor agent.
8. A combined preparation comprising an oligonucleotide as defined in any of items 1-3 and an anti-viral agent and/or anti-bacterial agent and/or anti-tumor agent, wherein the oligonucleotide and the agent are for simultaneous, separate or sequential administration.
9. The combined preparation of any of items 6-8, further comprising retinoic acid and/or type I IFN.
10. The combined preparation of any of items 6-9, further comprising a complexation agent.
11. The combined preparation of any of items 6-9, wherein the oligonucleotide is comprised in a viral vector.
12. A pharmaceutical package comprising the pharmaceutical composition of any of items 1-7 or the combined preparation of any of items 6-11 and an instruction for use.
13. An oligonucleotide defined in any of items 1-3 for use in stimulating an anti-viral response or an anti-bacterial response in a vertebrate animal.
14. The oligonucleotide for use of item 13, wherein the anti viral response or anti-bacterial response comprises type I IFN production, IL-18 production, and/or IL-1β production.
15. An oligonucleotide as defined in any of items 1-3 for use in preventing and/or treating diseases and/or disorders selected from the group consisting of viral infection, bacterial infection, tumor, immunosuppression and immunodeficiency in a vertebrate animal.
16. The oligonucleotide for use of item 15, wherein the oligonucleotide or the precursor thereof is to be used in combination with an anti-viral agent and/or an anti-bacterial agent and/or an anti-tumor agent and/or an anti-tumor therapy.
17. An oligonucleotide defined in any of items 1-3 and at least one antigen for use in inducing an immune response against the at least one antigen in a vertebrate animal.
18. The oligonucleotide for use of item 17, wherein the oligonucleotide or the precursor thereof is covalently linked to the at least one antigen.
19. An oligonucleotide as defined in any of items 1-3 for use in inducing apoptosis of a tumor cell.
20. The oligonucleotide for use of any of items 13-19, wherein the oligonucleotide or the precursor thereof is used in combination with a complexing agent.
21. The oligonucleotide for use of any of items 13-19, wherein the oligonucleotide or the precursor thereof is comprised in a viral vector.
22. The olionucleotide for use of any of items 13-21, wherein the oligonucleotide or precursor thereof is to be used in combination with an immunostimulatory agent.
23. The oligonucleotide for use of any of items 13-22, wherein the oligonucleotide is to be used in combination with retinoic acid and/or type I IFN.
24. An in vitro method for stimulating an anti-viral or an anti-bacterial response in a cell, comprising the steps of:
   (a) mixing an oligonucleotide as defined in any of items 1-3 with a complexation agent; and
   (b) contacting a cell with the mixture of (a), wherein the cell expresses RIG-I and/or NALP-3.
25. The in vitro method of item 24, wherein the anti-viral response comprises type I IFN production, IL-18 production, and/or IL-1β production.

In the following the invention is defined by the embodiments of the appended claims.

### Brief Description of the Figures

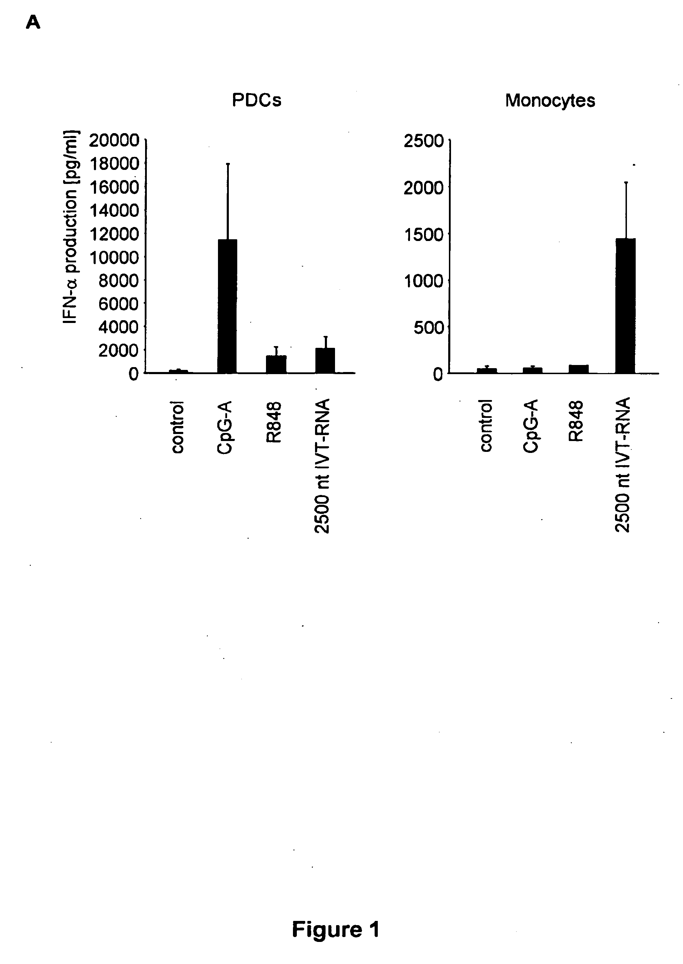

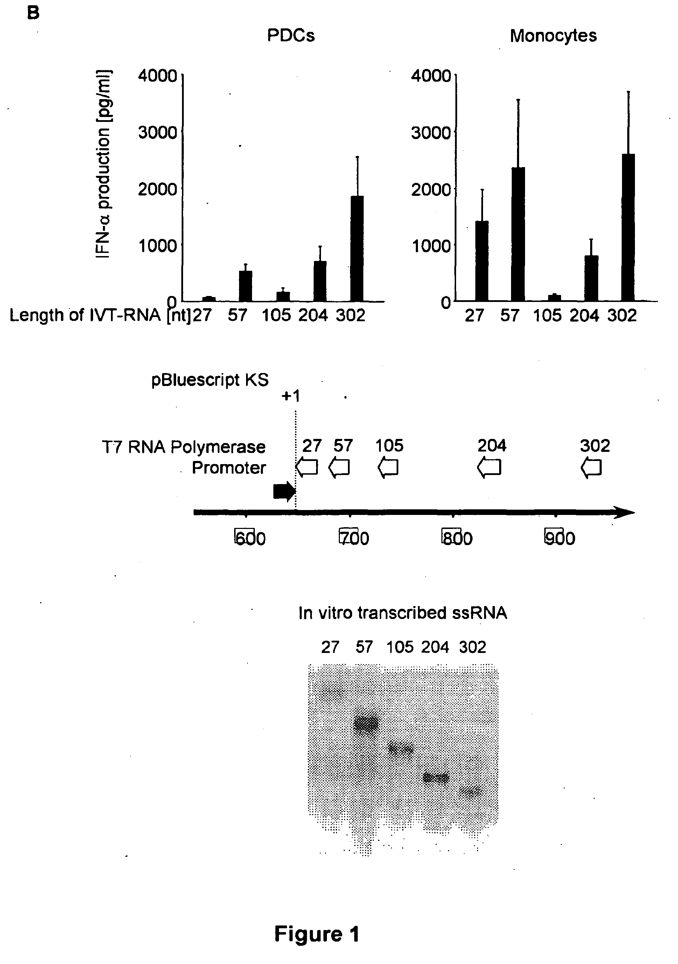

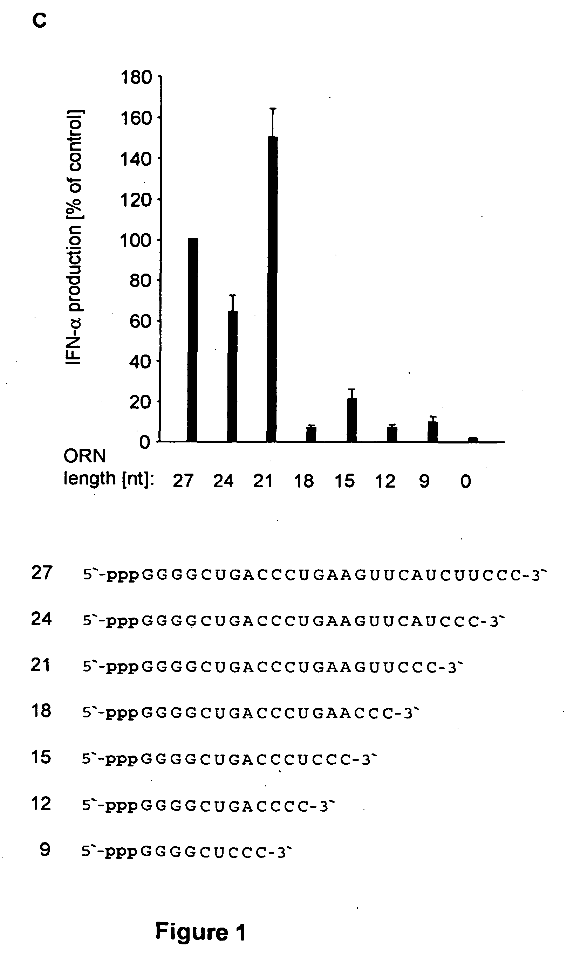

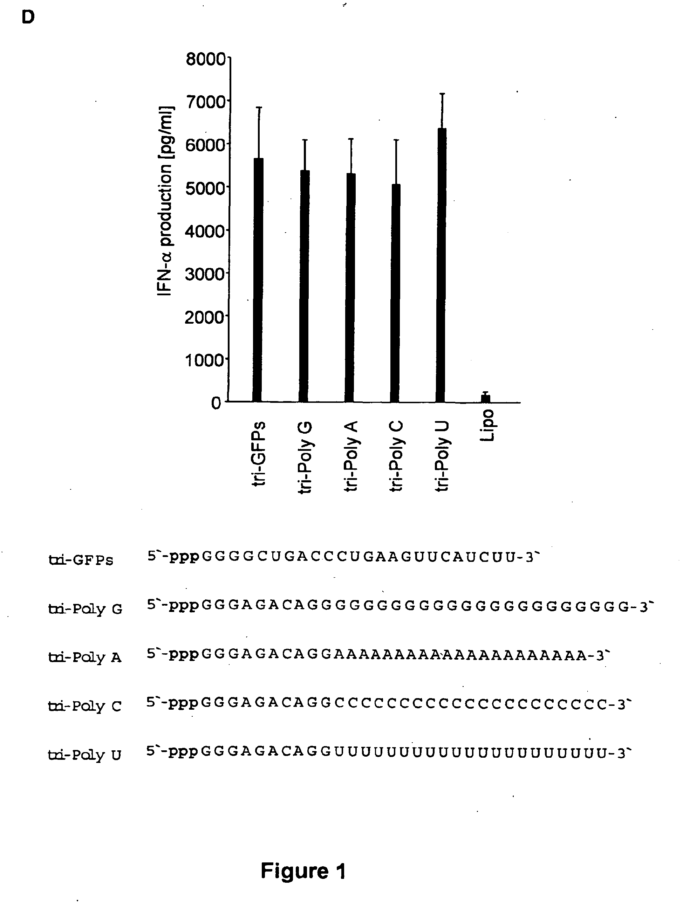

### Figure 1: In vitro transcribed RNA induces a potent IFN-α response in human monocytes

(A) PDC and monocytes were plated in 96-well plates and transfected with 200 ng in vitro transcribed RNA (2500 nucleotides). CpG-A (3 µg/ ml) and R848 (10 µM) were used as control stimuli for TLR9- or TLR7-mediated IFN-α induction in PDC. Supernatant was harvested 24 hours after stimulation and IFN-α production was assessed via ELISA. Data of two independent donors were summarized and are depicted as mean values ± SEM.
(B) pBluescript KS was used to generate DNA templates of various lengths for in vitro transcription (lower panel). In vitro transcribed RNAs were analyzed on a 4 % denaturing agarose gel prior to transfection. Subsequently in vitro generated RNAs were transfected in purified PDC and monocytes plated in 96-well plate. 24 hours after transfection supernatants were analyzed for IFN-α production. Data of two independent donors were summarized and are depicted as mean values ± SEM.
(C) A set of RNA oligonucleotides was generated ranging from 27 to 9 nucleotides by gradually shortening a 27-mer oligonucleotide from the 3' end in steps of three nucleotides. Purified monocytes were transfected with the respective oligonucleotides and IFN-α production was analyzes 24 hours after stimulation. Data of five independent donors were normalized to the IFN-α induction level of the 27 nucleotides oligonucleotide (5876 ± 1785 pg/ ml) and summarized as mean values ± SEM.
(D) Purified monocytes were transfected with 200 ng in vitro transcribed RNA with different homopolymeric 3' tails. Tri-GFPs was included as a positive control. 24 hours after transfection, supernatants were collected and IFN-α production was assessed via ELISA. Data of four independent donors were summarized and are depicted as mean values ± SEM.

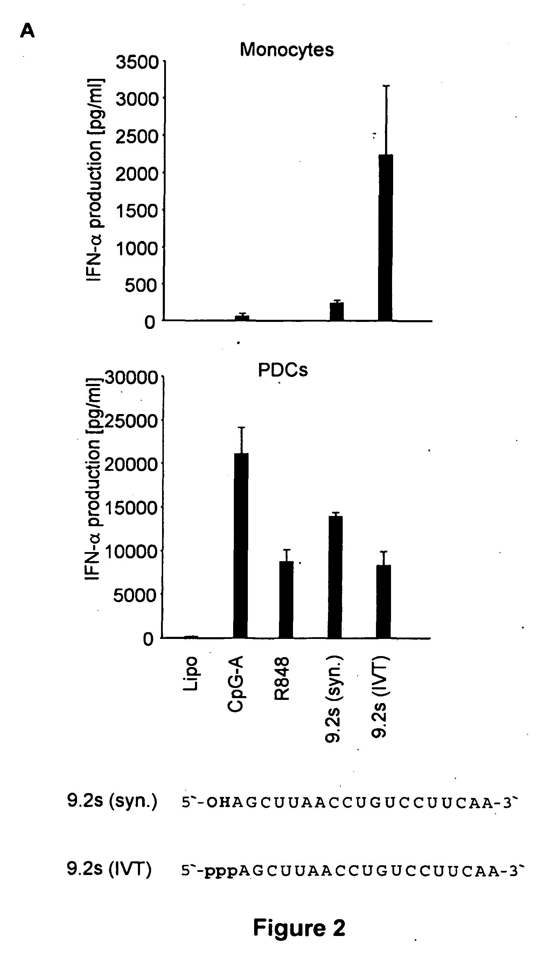

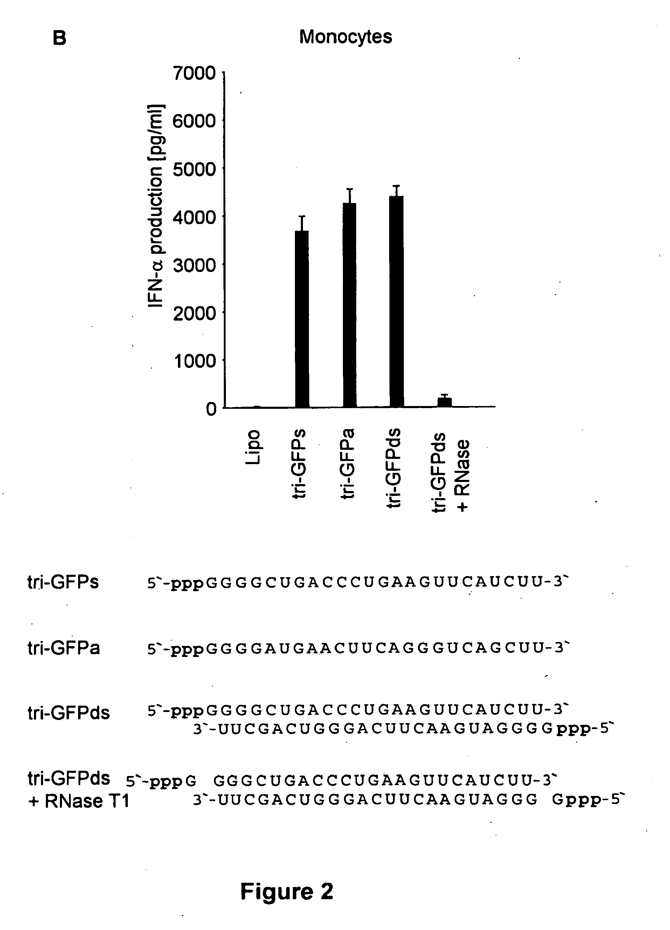

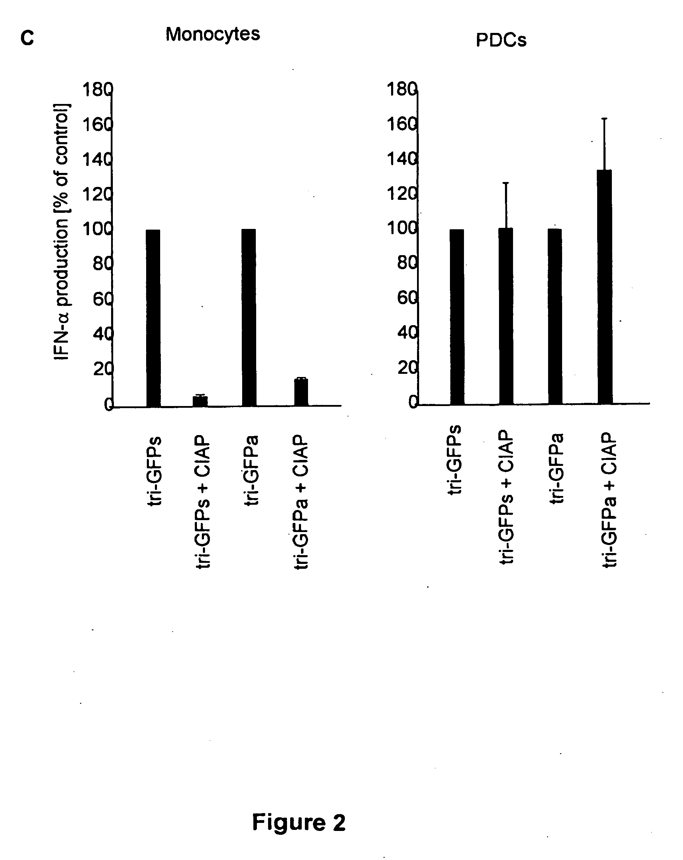

### Figure 2: 5' phosphorlyated, but not synthetic RNA oligonucleotides are potent inducers of IFN-α in human monocytes

(A) Synthetically synthesized or enzymatically transcribed RNA9.2s (200 ng) was transfected into purified monocytes or PDCs. CpG-A (3 µg/ ml) and R848 (10 µM) were included as positive control stimuli for TLR9- or TLR7-mediafed IFN-α induction in PDC. Data of two (monocytes) or three (PDCs) independent donors were summarized and are depicted as mean values ± SEM.
(B) The sense (tri-GFPs) and the antisense (tri-GFPa) strand of an established anti-GFP siRNA were transcribed using in vitro transcription. Both the single stranded components and the annealed dsRNA molecule (all 200 ng) were transfected into purified monocytes. In addition the dsRNA molecule was incubated with RNase T1 to remove the overhanging 5' ends from both strands. Data from two independent donors are depicted as mean values ± SEM.
(C) Calf intestine alkaline phosphatase (CIAP) was used to dephosphorylate tri-GFPs and tri-GFPa. Untreated or dephosphorylated RNA oligonuceltoides were subsequently transfected into monocytes and PDC. Data from two independent donors were normalized to the respective untreated control oligonucelotide and are depicted as mean values ± SEM.

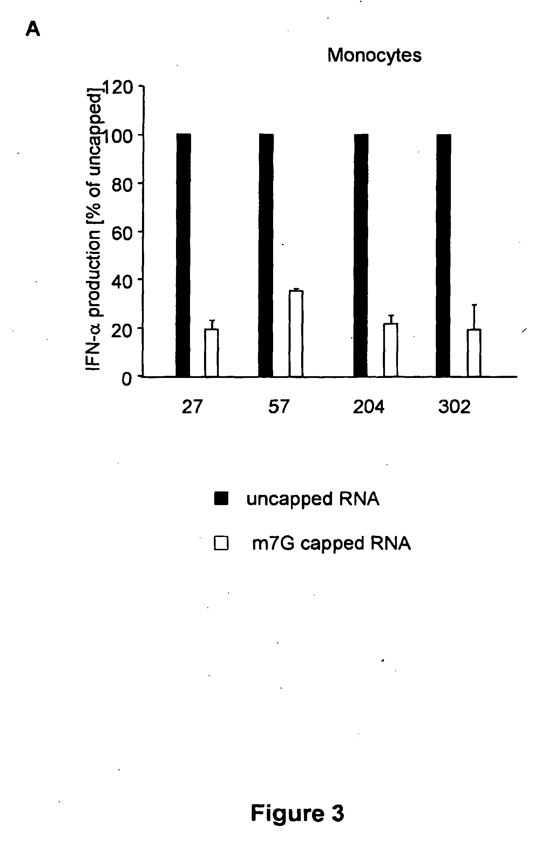

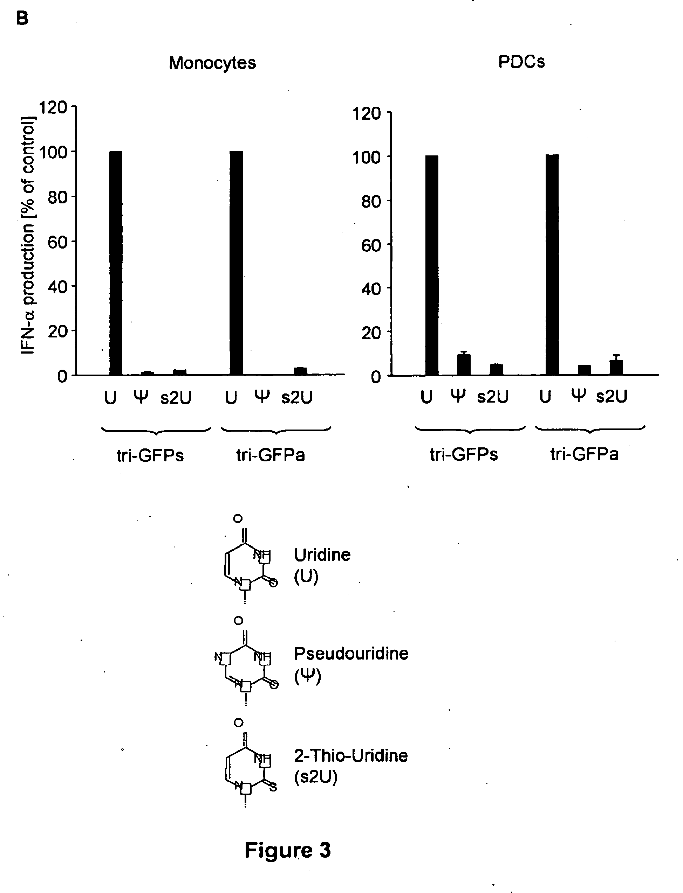

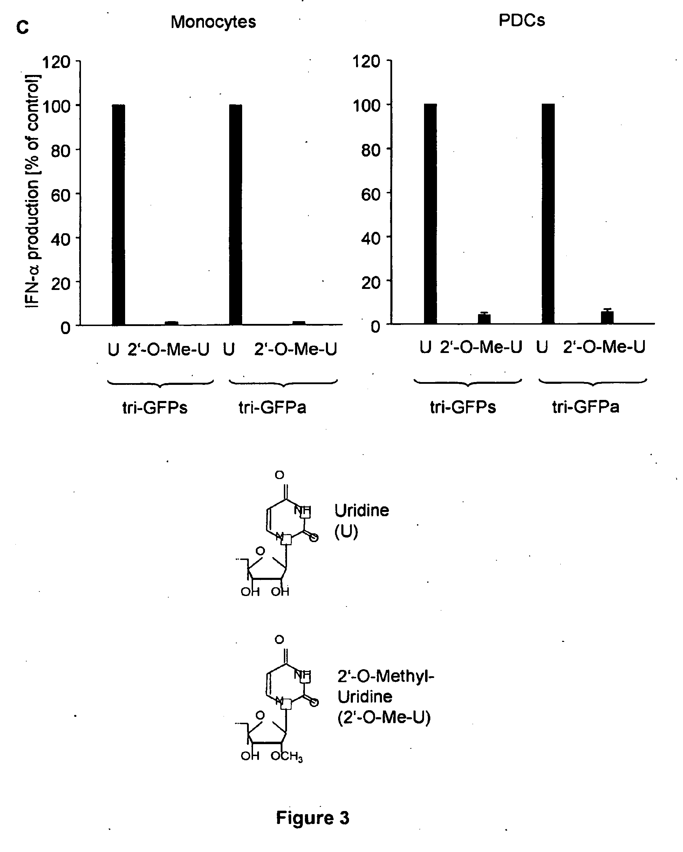

### Figure 3: 7-methyl-guanosine capping and eukaryotic-specific base modifications abolish IFN-α induction via 5'triphosphate RNA

(A) RNA molecules of various length (27 nucleotides - 302 nucleotides) dervied from pBKS as a template (see Table 1 B) were transcribed in the presence of the cap analogue N-7 methyl GpppG (m7G capped RNA) or using standard NTPs (uncapped RNA). Purified monocytes were transfected with either m7G capped or uncapped RNAs (200 ng each) and IFN-α production was assessed 24 hours after stimulation. For each RNA transcript, data of two independent donors were normalized to the uncapped RNA value and summarized as mean values ± SEM. The absolute values for the respective RNA transcripts were 1401, 2351, 91, 797 and 2590 pg/ml, respectively.
(B) & (C) Tri-GFPs and tri-GFPa were synthesized via in vitro transcription in the presence of either uridine-5'-triphosphate, pseudouridine-5'-triphosphate (ψ), 2-thiouridine-5'-triphosphate (s2U) (all B) or 2'-O-methyluridine-5'-triphosphate (C). Subsequently purified monocytes and PDCs were transfected with the respective oligonucleotides and IFN-a production was assessed 24 hours after stimulation. For each RNA transcript, data of two (B) or three (C) independent donors were normalized to the value of the RNA oligonucleotide transcribed in the presence of uridine-5'-triphosphate and summarized as mean values ± SEM.

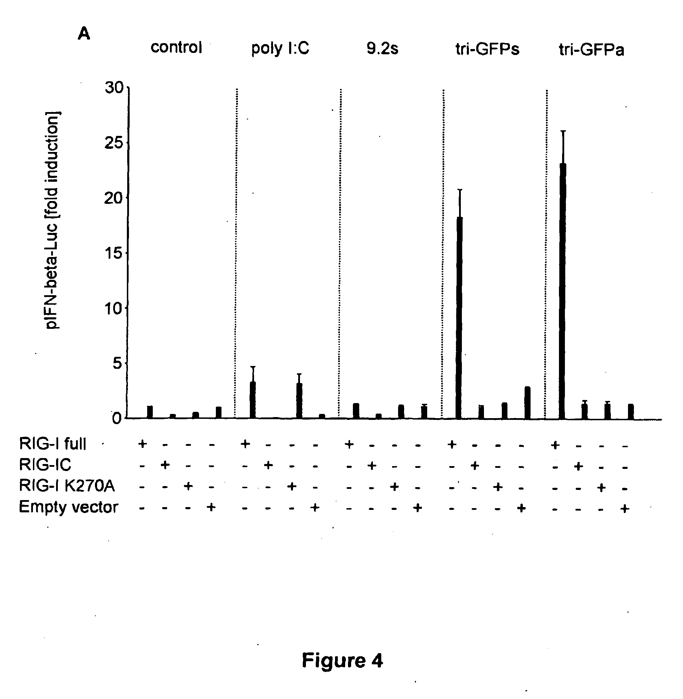

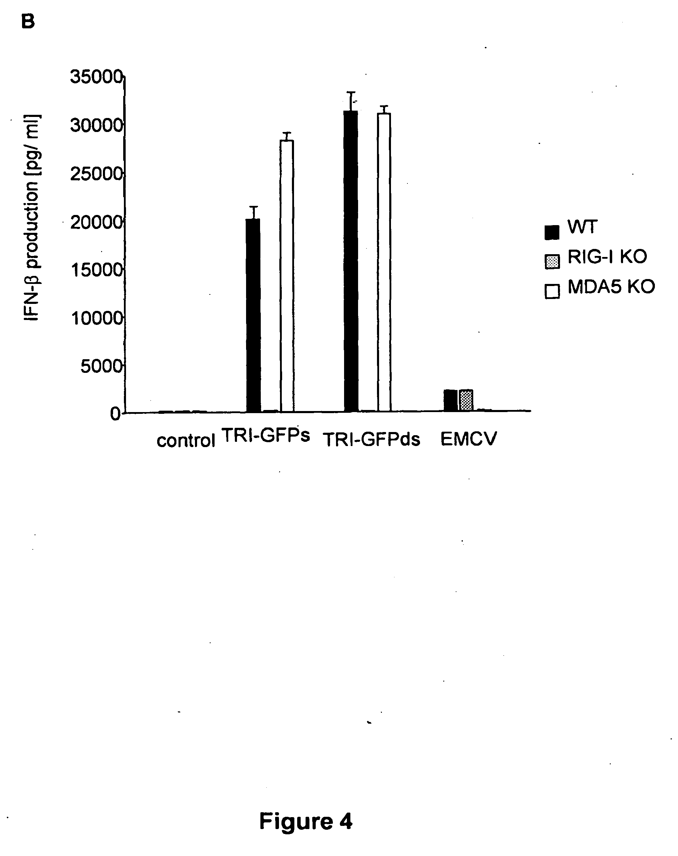

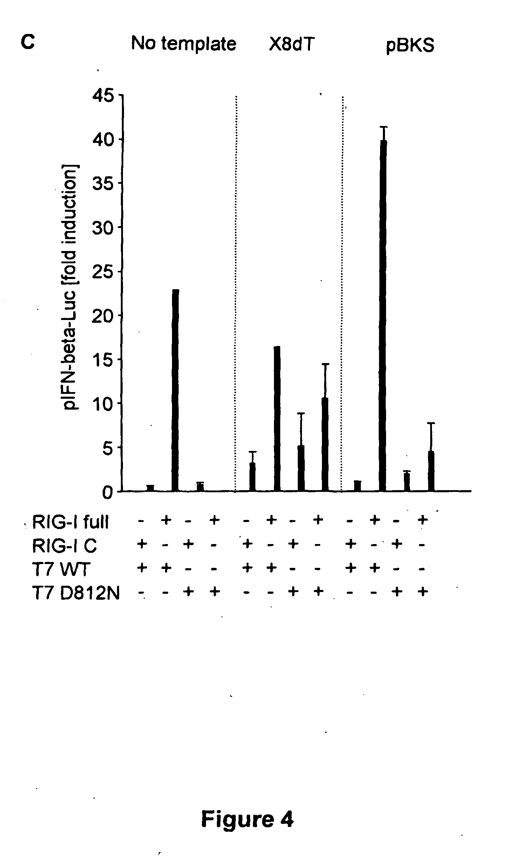

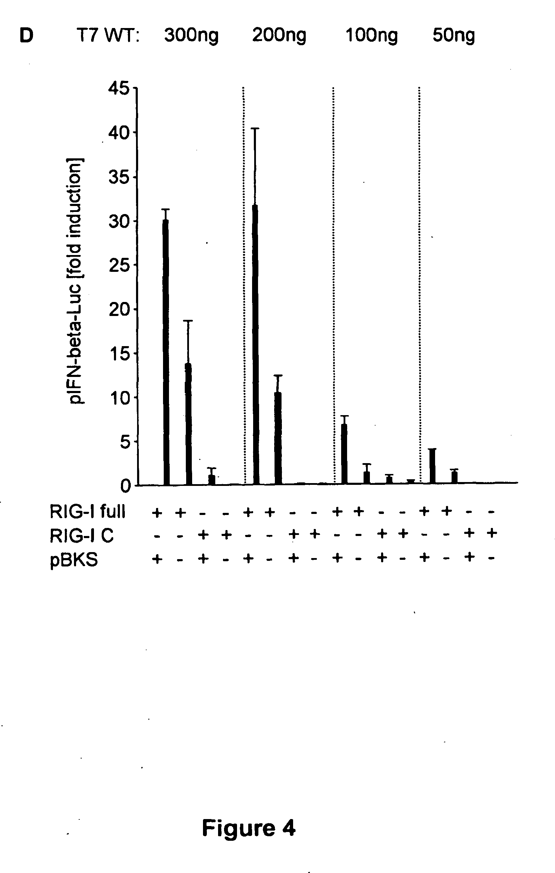

### Figure 4: Triphosphate-mediated IFN-α induction requires RIG-I but not MDA5

(A) HEK 293 cells were transfected with either RIG-I full, RIG-IC, RIG-I K270A or the corresponding empty vector (all 200 ng each) in the presence of pIFN-beta-Luc (300 ng) and pSV-beta Galactosidase (400 ng). In addition either nothing, poly I:C, synthetic RNA9.2s, tri-GFPs or tri-GFPa (all 200 ng) were included. 24 hours after transfection pIFN-beta-Luc reporter activity was assessed. Data from one representative experiment out of three were normalized to the empty vector condition and are depicted as mean values of duplicates ± SEM.
(B) MEFs from mice devoid of either RIG-I or MDA5 or respective wild type MEFs were transfected with tri-GFPs or tri-GFPds. In addition MEFs were infected with EMCV at a M.O.I. of 1. 24 hours after stimulation supernatants were collected and assayed for IFN-β production. Data from one representative experiment out of three are depicted.
(C) In addition, HEK 293 cells were transfected with either RIG-I full or RIG-IC (200 ng each) and T7 RNA polymerase or the transcriptionally defective point mutant T7 RNA polymerase D812N (300 ng each) in the presence of pIFN-beta-Luc (300 ng) and pSV-beta Galactosidase (400 ng). In addition either nothing, X8dt (vector based on the pBKS backbone without T7 RNA polymerase promoter) or pBKS (all 300 ng) were included. 24 hours after transfection pIFN-beta-Luc reporter activity was assessed.
(D) In addition HEK 293 cells were transfected with decreasing doses of T7 RNA polymerase in the presence of either RIG-I full or RIG-IC (200 ng) with nothing or pBKS (300 ng), while pIFN-beta-Luc (300 ng) and pSV-beta Galactosidase (400 ng) were included. 24 hours after transfection pIFN-beta-Luc reporter activity was assessed. Data from one representative experiment out of three were normalized to the RIG-IC / pBKS / T7 RNA polymerase (300 ng) condition and are depicted as mean values of duplicates ± SEM.

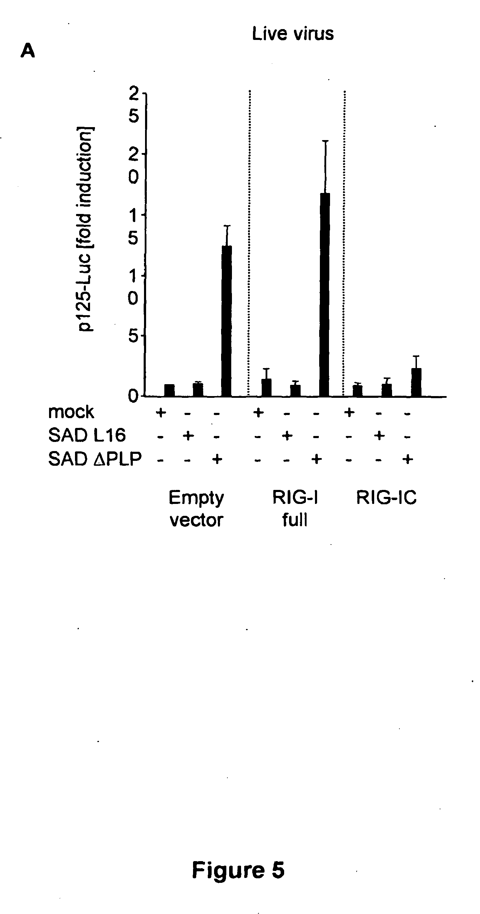

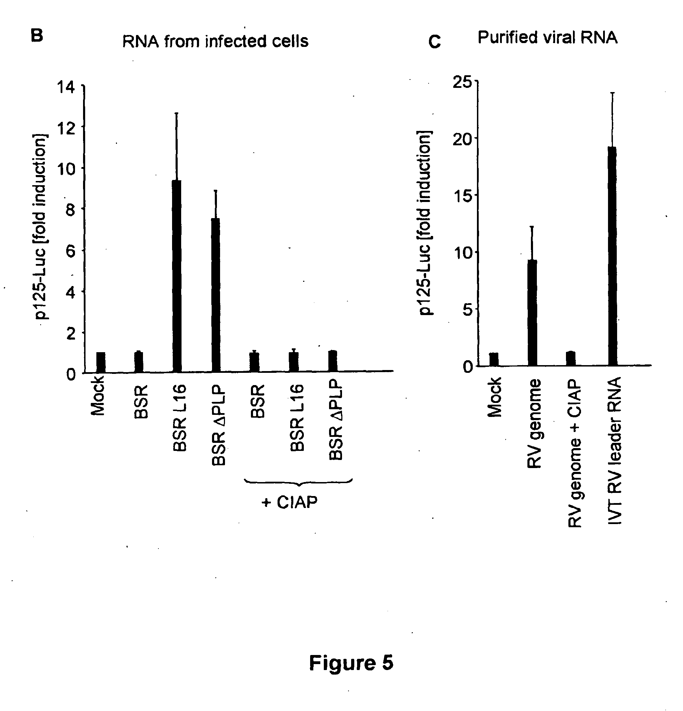

### Figure 5: Viral RNA induces IFN-induction via RIG-I depending on its 5' end phosphorylation status

(A) Vero cells were transfected with either empty vector, RIG-I full or RIG-IC in the presence of the reporter plasmid p125-Luc. 6 hours later, the cells were either mock-infected or infected with RV SAD L16 or RV SAD ΔPLP at a MOI of 3. p125-Luc reporter activity was assessed 48h after DNA transfection. Average data from two experiments done in duplicates are shown as mean fold values (mock = 1) ± SEM.
(B) HEK 293T cells were either mock-transfected with PEI, or with 1 µg total RNA isolated from non-infected BSR cells or total RNA isolated from BSR cells infected with RV L16 or RV □PLP. RNA isolates of non-infected BSR-cells, BSR cells infected with SAD L16 (BSR L16) and SAD ΔPLP (BSR dPLP) were additionally treated with CIAP and transfected accordingly. 48h after transfection p125-Luc reporter activity was assessed. Data are shown as mean fold values (mock = 1) of triplicates ± SEM.
(C) Either mock, RNA isolated from gradient-purified virions (RV L16) or CIAP-treated RNA from purified virions was used to stimulate HEK 293T cells. As a positive control, an in vitro transcribed RNA oligonucleotide corresponding to the 5' terminal leader sequence (58 nt) of the RV SAD L16 cRNA was used to stimulate HEK 293T cells. 48h after stimulation p125-Luc reporter activity was assessed. Data from the experiment are shown as mean fold values (mock = 1) of triplicates ± SEM.

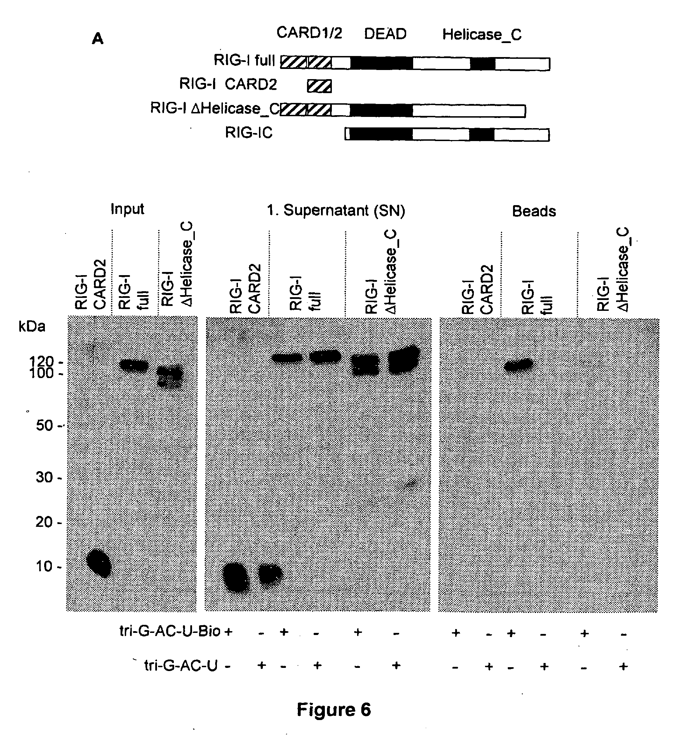

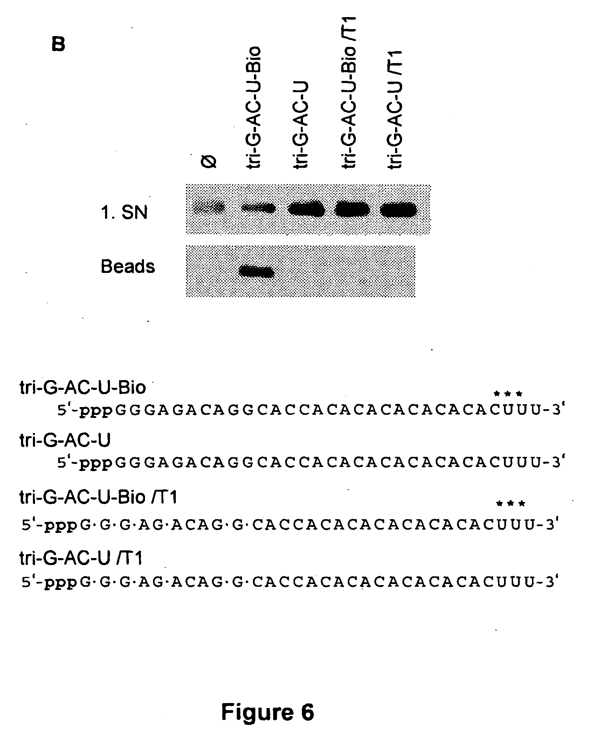

### Figure 6: Triphosphate RNA directly binds to RIG-I

(A) HEK 293 cells were transiently transfected with full length RIG-I, RIG-I CARD2 or RIG-I ΔHELIc. 36 hours after transfection cells were lysed and conincubated with the indicated RNA oligonucleotides (0.375 µg; lower right panel) for two hours at 4 °C. Next, streptavidin-agarose-beads were added for an additional period of one hour. Beads were collected by centrifugation and washed four consecutive times. After all washing steps, supernatants were collected and after four washes streptavidin-agarose beads were collected by centrifugation and boiled in Laemmli buffer. For one representative experiment out of two, the input (A, left panel), the supernatants of the first wash (1. SN) (A, middle panel) and the bead-bound fraction (A, right panel) are depicted (no or little signal was seen in the supernatant of the second, third and fourth wash; data not shown). All preparations were run on the same gel and the membranes were exposed for the same time period.
(B) RIG-IC was immunoprecipitated using Flag-agarose-beads and subsequently eluted via Flag-peptide. In analogy to above experiments, the depicted RNA oligonucleotides were added to purified RIG-IC and subsequently conicubated with streptavidin-agarose beads. If inidicated, RNase T1 was used to remove the 5' portion of the oligonucleotide containing the triphosphate group. Beads were washed four consecutive times and the first supernatant and the bead-bound fraction were analyzed by western blotting. One representative experiment out of three is shown.

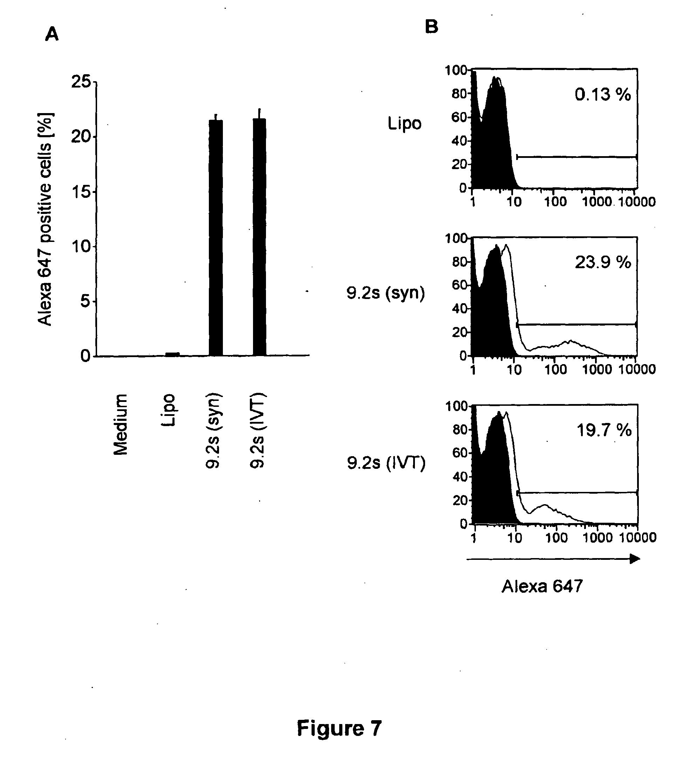

### Figure 7: No difference in uptake of synthetic and triphosphate RNA oligonucleotides in monocytes

(A) Synthetic or *in vitro* transcribed RNA oligonucleotides of the sequence 9.2s were chemically labelled with Alexa 647 fluorophores, resulting in a base : dye ratio of 81 and 71 respectively. Subsequently purified monocytes were transfected with labeled RNA oligonucleotides (all 50 ng). Two hours after transfection cells were harvested and vigorously washed with 10 mM EDTA in PBS twice. Uptake of the fluorescently labeled oligonucleotides were assessed by flow cytometry. Untreated monocytes were used to set the threshold level for positive cells. Data from two independent donors were summarized and are depicted as as mean values ± SEM.
(B) Histogram plots from one representative donor are depicted.

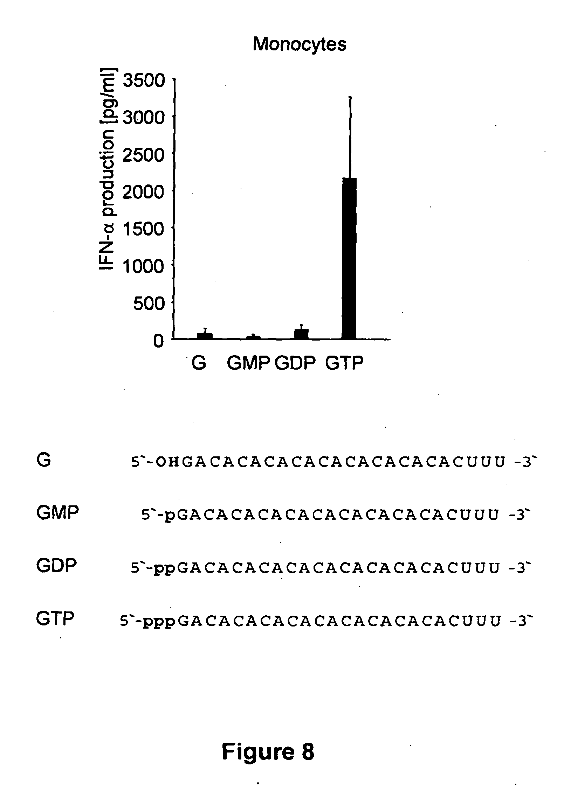

### Figure 8: Only guanosine triphosphate, but not guanosine diphosphate, guanosine monophosphate or guanosine initiated RNA oligonucleotides induce a potent IFN-α response in human monocytes

Using a T7 RNA polymerase template coding for a 24-mer RNA oligonucleotide with only one initial guanosine, RNA oligonucleotides were generated via in vitro transcription in the presence of ATP, CTP and UTP and either only guanosine, guanosine-5'-monophosphate, guanosine-5'-diphosphate or guanosine-5'-triphosphate. Subsequently purified monocytes were transfected with the respective RNA oligonucleotides (all 200 ng) and IFN-α production was analyzed 24 hours after stimulation. Data from two independent donors were summarized and are depicted as mean values ± SEM.

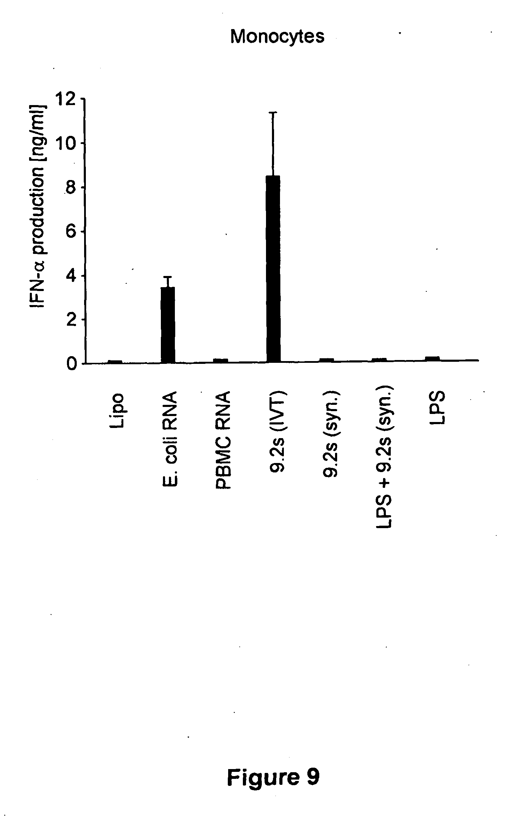

### Figure 9: Prokaryotic RNA, but not eukaryotic RNA induces IFN-a production in monocytes

Total RNA was isolated from *E*. *coli* bacteria strain DH10B and human PBMC. Subsequently monocytes were transfected with *E. coli* RNA, PBMC RNA, synthetic 9.2s RNA or in vitro transcribed 9.2s (all 200 ng). In addition LPS (100 ng/ ml) was added either exogenously or combined with cationic lipid complexed synthetic 9.2s RNA to stimulate monocytes. IFN-α production was analyzed 24 hours after stimulation. Data from two independent donors were summarized and are depicted as mean values ± SEM.

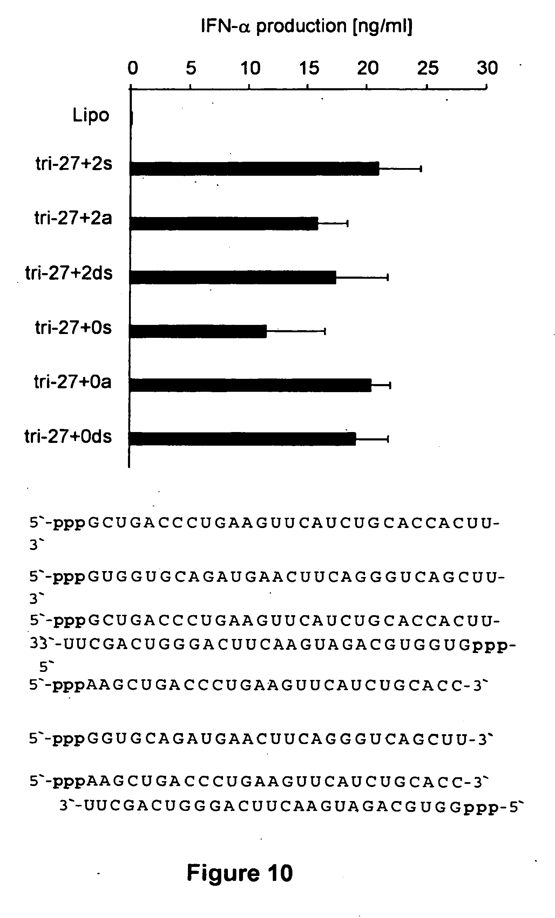

### Figure 10: 3' overhangs of double stranded triphosphate RNA oligonucleotides do not impact on the immunostimulatory activity

Purified monocytes were transfected with either tri-27+2s, tri-27+2a, tri-27+0s, tri-27+0a or the respective double stranded oligonucleotides (all 200 ng). IFN-α production was analyzed 24 hours after stimulation. Data from three independent donors were summarized and are depicted as mean values ± SEM.

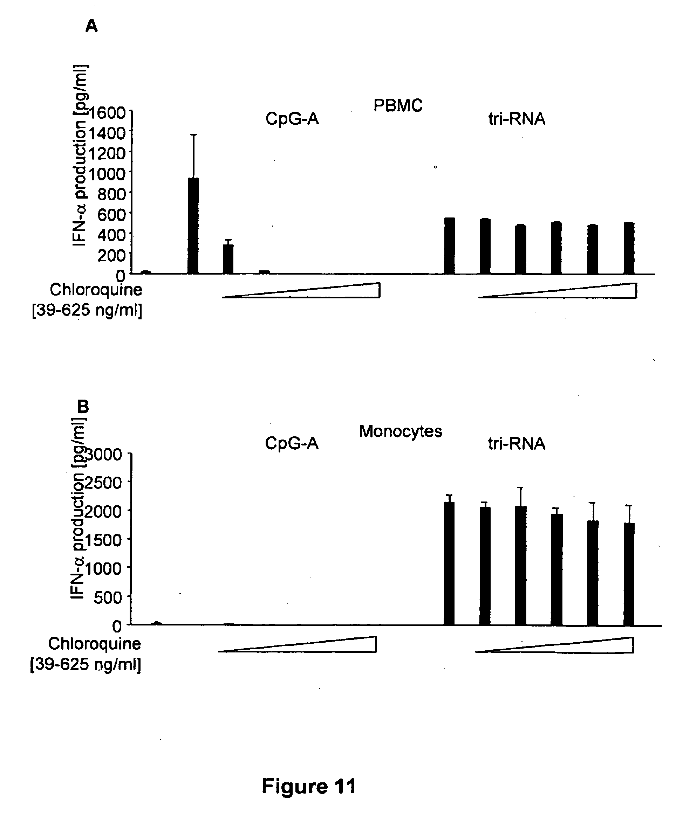

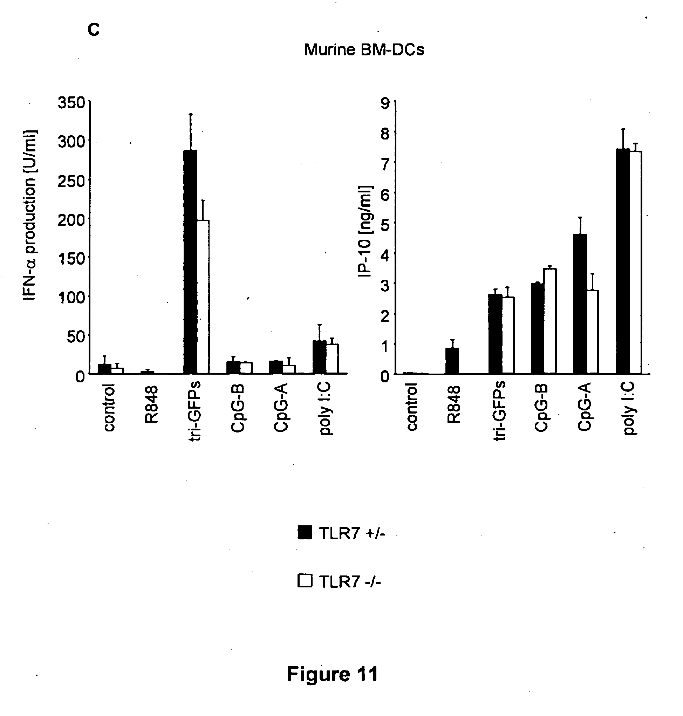

### Figure 11: Triphosphate RNA-mediated IFN-α induction is independent of endosomal maturation and of TLR7

(A) & (B) Purified PDCs (A) and monocytes (B) were preincubated with two-fold ascending doses of chloroquine (39 - 625 ng/ ml) and subsequently cells were either stimulated with CpG-A (3 µg/ ml) or transfected with 200 ng tri-GFPa. 24 hours after incubation supernatants were collected and IFN-α production was assessed via ELISA. Data from two independent donors were summarized as mean values ± SEM.
(C) Murine MDC were generated from bone marrow cells from either TLR7 knock out mice (TLR7 -/-) or respective control animals (TLR7 +/-). Subsequently BM-MDC were transfected with 200 ng tri-GFPs or stimulated with either R848 (10 µM), CpG-B (3 µg/ ml), CpG-A (3 µg/ ml) or poly I:C (25 µg/ ml). 24 hours after incubation supernatants were analyzed for IFN-α and IP-10 production. One representative experiment (mean of duplicates ± SEM) out of three is depicted.

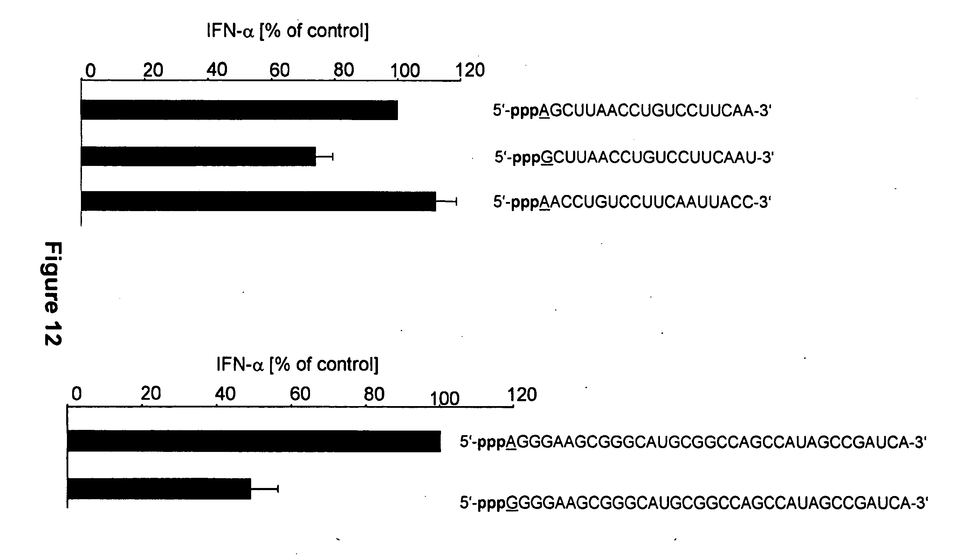

### Figure 12: 5' adenosine-initiated triphosphate transcripts are superior to 5' guanosine initiated transcripts in terms of IFN-α induction

(A) Purified monocytes were transfected with either RNA9.2-0A, RNA9.2s-1G or RNA9.2s-5A (all 200 ng) and IFN-α production was analyzed 24 hours after stimulation. Data from two independent donors were summarized and are depicted as mean values ± SEM.
(B) RNA transcripts derived from either the AΦ6.5-35n or the GΦ6.5-35n template were transfected into purified monocytes and IFN-α induction was assessed 24 hours after transfection. Data from three independent donors were summarized and are depicted as mean values ± SEM.

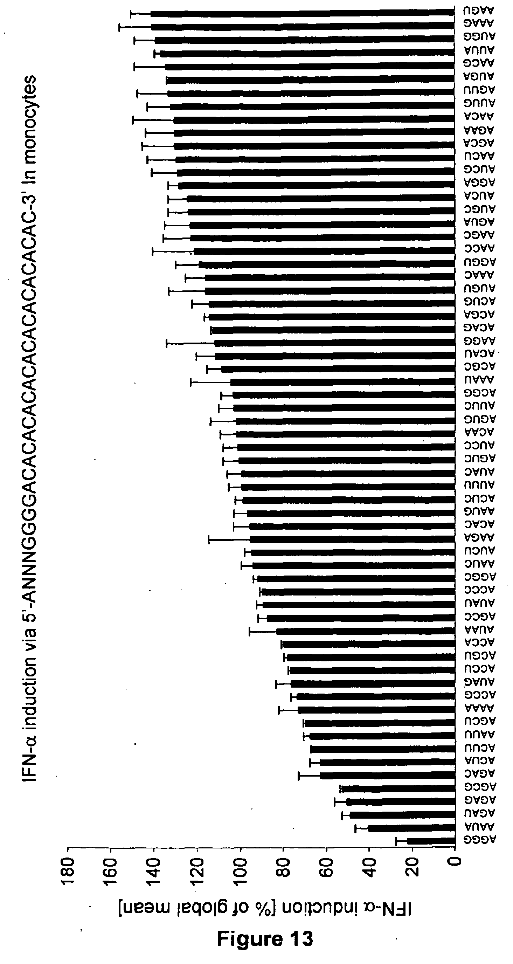

### Figure 13: 5' sequence of adenosine-initiated 5'-triphosphate RNA oligonucleotides dictates IFN-α inducing activity.

Adenosine-initiated triphosphate RNA oligonucleotides with all possible base permutations (A, C, G and U) of the 2nd, 3rd and 4th position of the sequence (5'-> 3') were generated via in vitro transcription (see Table 2). Subsequently monocytes from three independent donors were isolated and transfected with the respective RNA oligonucleotides. 36 hours after transfection, supernatants were analyzed for IFN-a production. The obtained IFN-α induction levels of all oligonucleotides were normalized to the mean induction level of all oligonucleotides (= 100%). The obtained normalized induction levels of all three donors were summarized as mean values ± SEM.

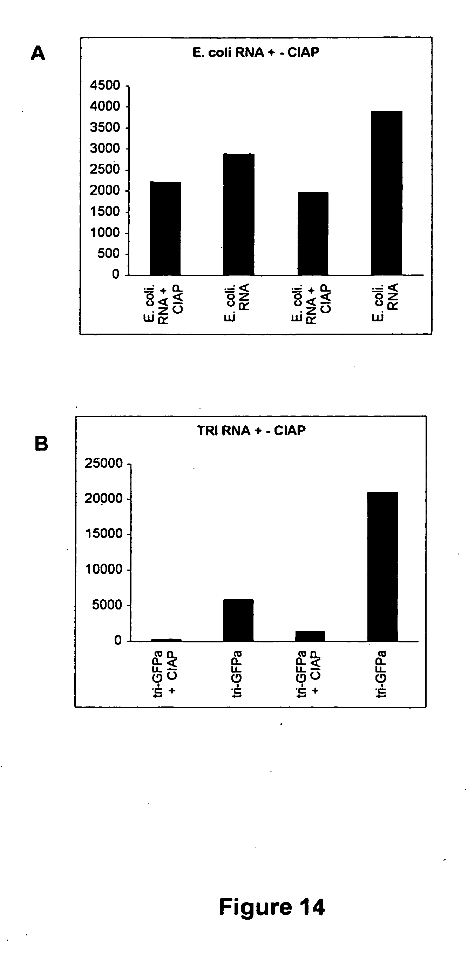

### Figure 14: Prokaryotic RNA, but not in vitro transcribed RNA induces IFN-α in human monocytes after 5' dephosphorylation.

Tri-GFPa was prepared via *in vitro* transcription (A), and in addition total RNA was isolated from *E. coli* bacteria strain DH10B (B). Subsequently the respective RNA preparations were treated with CIAP to dephosphorylate the 5' end and transfected into purified monocytes (200 ng of RNA). IFN-α production was analyzed 24 hours after stimulation. Data from two independent donors are depicted.

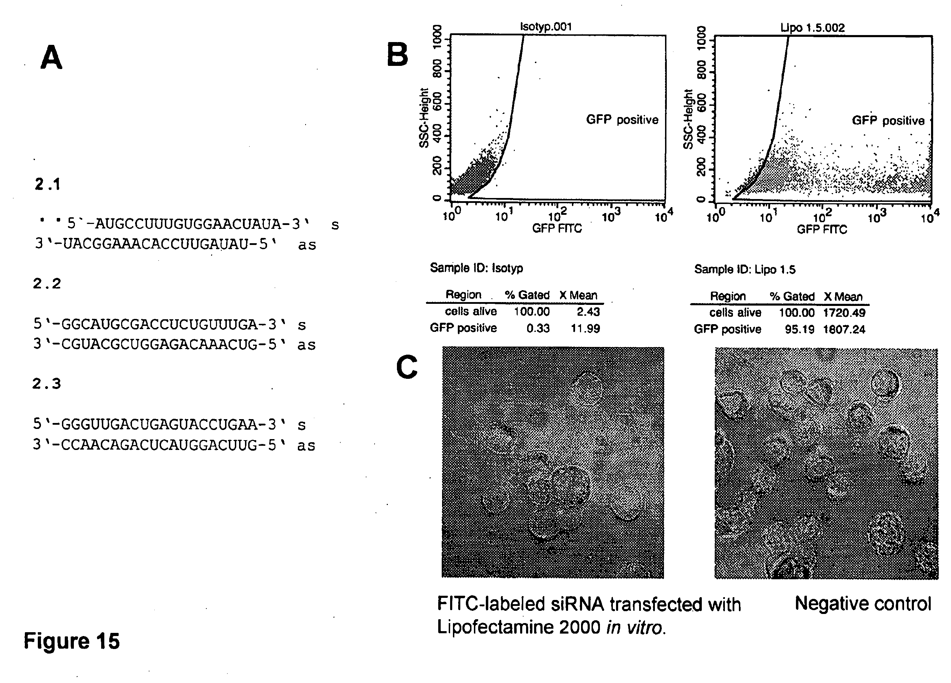

### Figure 15: Transfection efficiency of murine B16 cells.

(A) SiRNAs directed against murine BCL-2 are depicted. SiRNAs were designed using an algorithm provided by Dharmacon Research (Reynolds et al. 2005). Additionally, the immunostimulatory capability of the selected siRNAs was predicted according to an algorithm established by Homung et al. (European patent application no. 05 020 020.3; PCT/EP2006/008980). Only siRNAs with highly immunogenic potential were selected.
(B and C) Murine B16 cells (5 x 10⁵ cells/ml) were seeded in 24 well plates and transfected at a confluency of 70% with 200 ng of a GFP-plasmid or 200 ng of FITC-labelled siRNA. GFP-Plasmid and FITC-labelled siRNA were both complexed to Lipofectamine according to the manufacturer's instructions. After 24 hours transfection efficiency was analysed by FACS (B) and internalisation of the FITC-labelled siRNA was analyzed by confocal microscopy (C). Data are representative of 3 independent experiments.

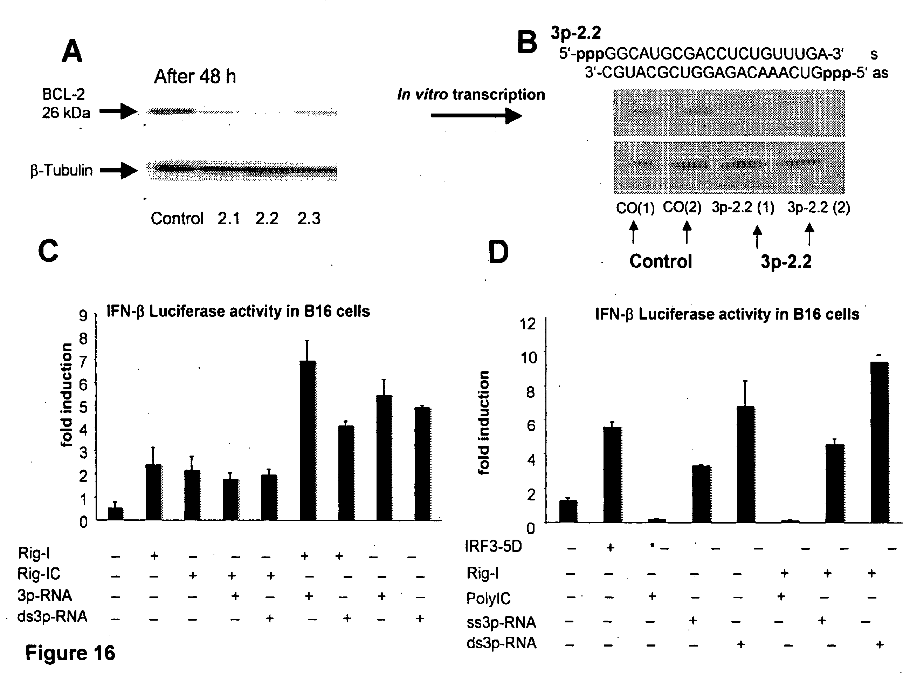

### Figure 16: Target knockdown and IFN-β induction by siRNA and 5'-triphosphate siRNA

(A) At a confluency of 70 %, B16 cells were transfected with the selected siRNA using 1,2 µg/ml siRNA. B16 cells were transfected using Lipofectamine (1,5 µl). 48 hours after transfection, "knockdown" of murine BCL-2 was analysed by Western blot analysis. SiRNA against TLR2 served as the negative control.
(B) siRNA 2.2 was produced by *in vitro* transcription and contained 5' triphosphate (now termed 3p-2.2). Two 3p-2.2 siRNA preparations of the same nucleotide sequence were tested for their ability to induce gene silencing as in (A).
(C and D) To monitor transient IFN-β activation by 3p-2.2, murine B16 cells were seeded in 24-well plates. At a confluency of 70 %, B16 cells were transfected using PEI with 500 ng of a reporter plasmid (pIFNß-luc DAM/DCM), 50 ng of a normalisation plasmid (expressing Renilla-Luc) and the indicated expression plasmids giving a total of 1,5 µg DNA/ well. As positive control, cells were transfected with IRF3-5D which drives the expression of a constitutively active IRF3, a transcription factor which drives IFN-α and IFN-β expression. 24 h after transfection, cells were analyzed for luciferase activity with a microplate luminometer (LUMIstar, BMGLabtechnologies). Data are shown as means ± SEM of 3 independent experiments.

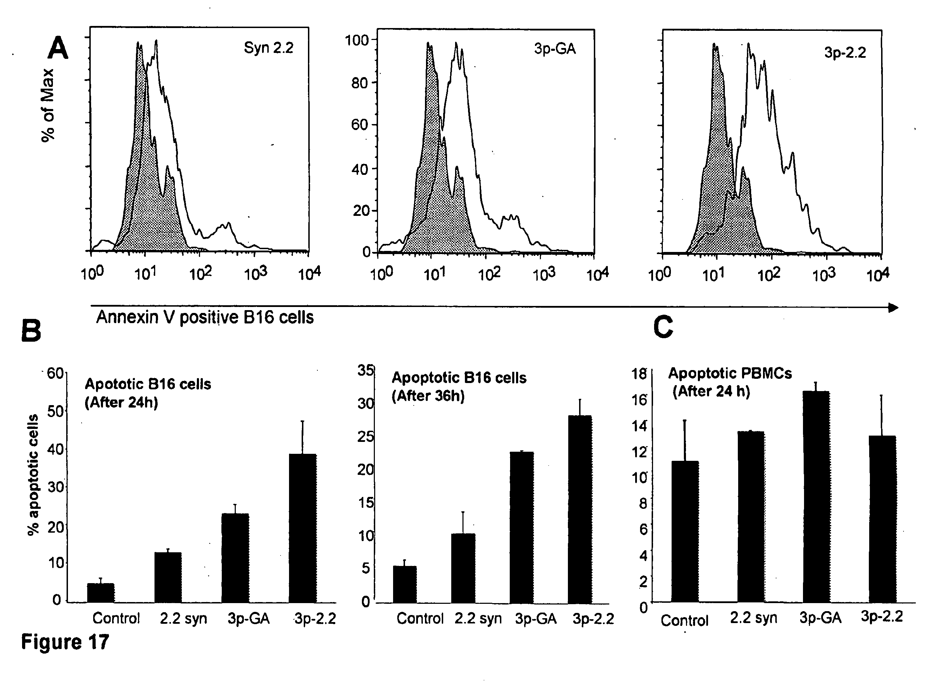

### Figure 17: 5' triphosphate-siRNA induces apoptosis in tumor cells, but not in primary non-tumor cells

To analyze apoptosis of murine B16 cells and primary human PBMCs induced by 3p-2.2, cells were seeded in 24-well plates and transfected as described previously. 24 h and 36 h after transfection, cells were analyzed by flow cytometry for early apoptosis by Annexin V staining. Annexin-V positive and PI-positve cells (i.e., late apoptotic or dead cells) were excluded.
(A) One representative histogramm of 2 independent experiments is shown. Syn 2.2 stands for synthetic 2.2 siRNA which does not contain the 5' triphophate. 3p-GA is a control 5' triphosphate RNA having the sequence 5'-TRI-GGGGGGGGGGGAAAAAAAAAAAA-3').
(B) Murine B16 cells were seeded in 24-well plates and transfected as described above. 24 h and 36 h after transfection, cells were analyzed for early apoptosis. Data are shown as means ± SEM of 2 independent experiments.
(C) Primary human PBMC were seeded in 24-well plates and transfected as described above 24 h after transfection, cells were analyzed for early apoptosis. Data are shown as means ± SEM of 2 independent experiments.

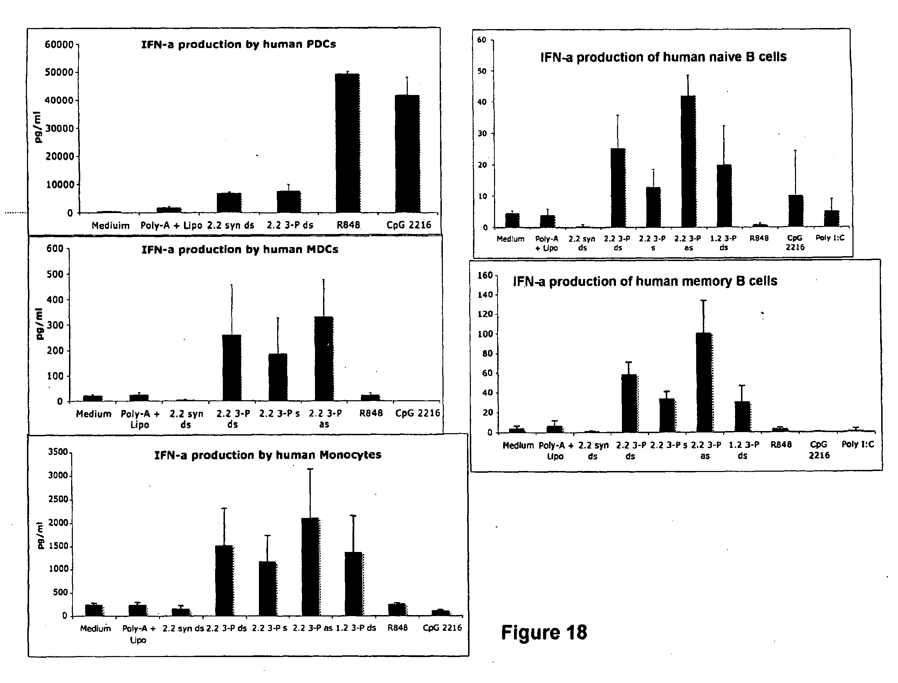

### Figure 18: 5' triphophate siRNA induces IFN-α secretion from distinct human immune subsets

To assess immunostimulatory capability of 5' triphophate siRNA, human immune cells were isolated and stimulated with 200 ng of 3p-2.2 siRNA, 3p-2.2s, or 3p-2.2as and 0,5 µl Lipofectamine in a 96 well plate. CpG2216 (3µg/ml) and R848 (1µM) served as a positive control. 2.2 syn ds served as a negative control. After 24 hours, the amount of secreted IFN-a in tissue culture supernatant was determined by ELISA. Data are shown as means ± SEM of 3 independent experiments.

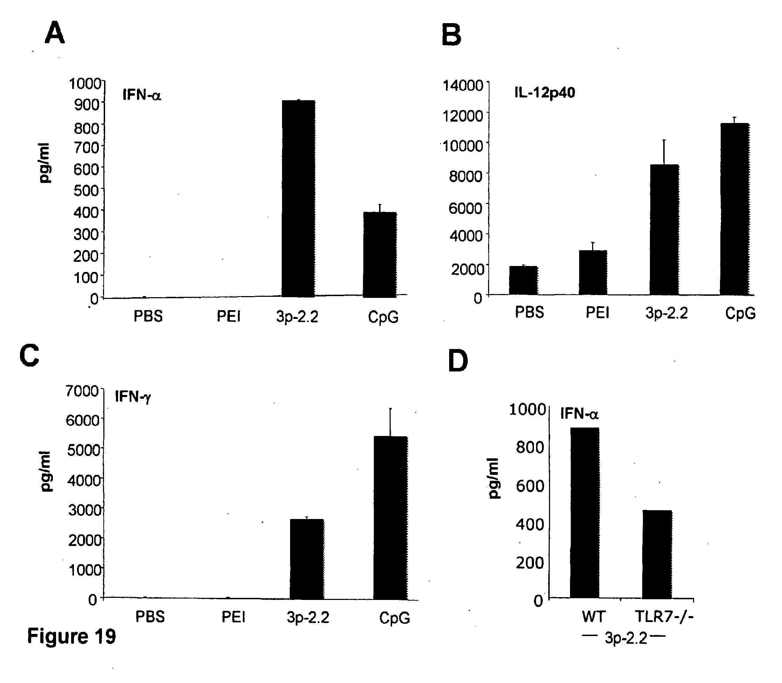

### Figure 19: 5' triphophate siRNA induces TLR7-independent immune activation in vivo

(A-C) C57/BL6 mice were injected with 200 µl of 3p-2.2 (50 µg/Mouse) complexed with jet-PEI™ in the retro-orbital vein. Serum was collected after 6 h unless indicated otherwise. Whole blood was obtained by tail clipping at the indicated time points. Cytokine levels of IFN-α (A), IL-12p40 (B) and IFN-γ (C) were determined by ELISA. CpG1826 served as a positive control. Data are shown as means ± SEM of 6 independent experiments.
(D) C57/BL6 and TLR7-/- mice were injected intravenously with 5' triposphate siRNA (50 µg) complexed to in vivo-jetPEI™ (Polypus transfection). After 6h mice were sacrificed and serum was analysed for IFN-α production by ELISA. Data are shown as means ± SEM of 2 independent experiments.

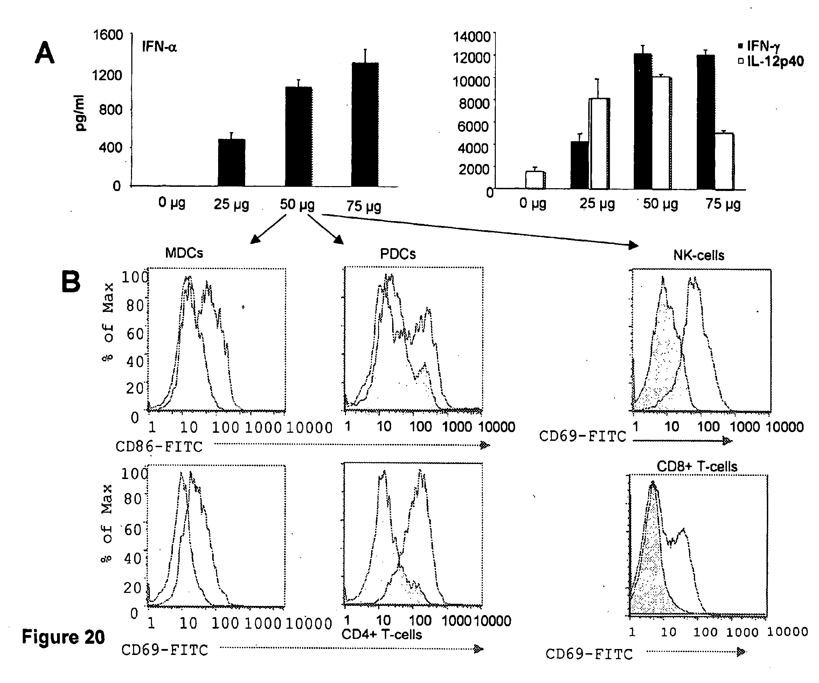

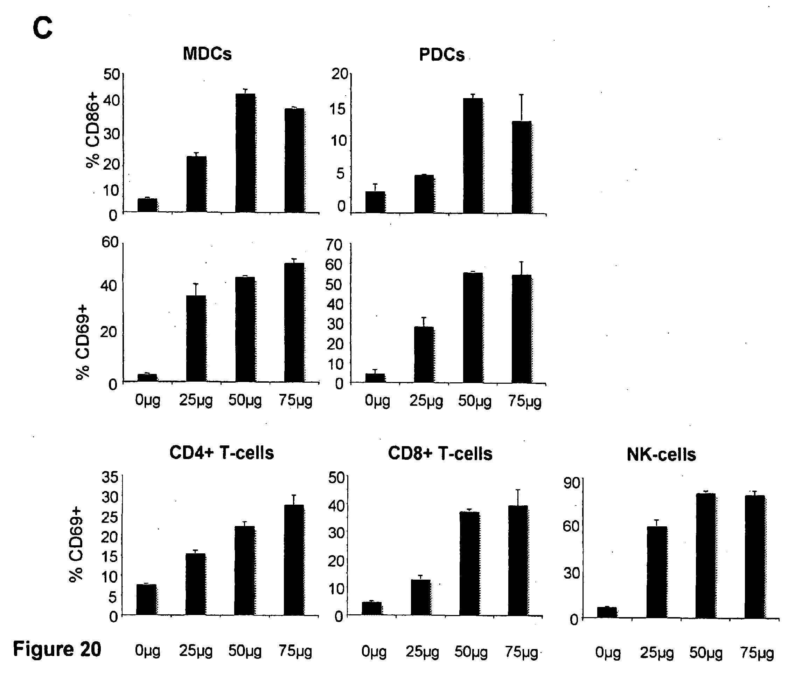

### Figure 20: Dose-dependent cytokine production and activation of distinct immune cell substes by 5' triposphate siRNA in vivo

(A) C57/BL6 mice were injected with 200 µl of 3p-2.2 (25-, 50- or 75 µg/mouse) complexed with jetPEI™ in the retro-orbital vein. Serum was collected after 6 h unless indicated otherwise. Serum cytokine levels of IFN-α, IL-12p40 and IFN-γ were determined by ELISA. Data are shown as means ± SEM of 5 independent experiments.
(B) 48 h after injection with 50 µg 3p-2.2, mice were sacrificed, spleens were excised, and immune cells were analysed by flow cytometry. Surface antigen staining was performed as described previously (http://www.bloodjournal.org/cgi/content/full/103/8/3058#REF4). One representative histogramm of 4 independent experiments is shown.
(C) Dose dependent activation of immune cell subsets *in vivo.* C57/BL6 mice were injected with 200 µl of 3p-2.2 (25-, 50- or 75 µg/mouse) complexed with jetPEI™. 48 h after injection, mice were sacrificed, spleens were excised and distinct immune cells were analysed by flow cytometry. Surface antigen staining was performed as described previously. Data are shown as means ± SEM of 3 independent experiments.

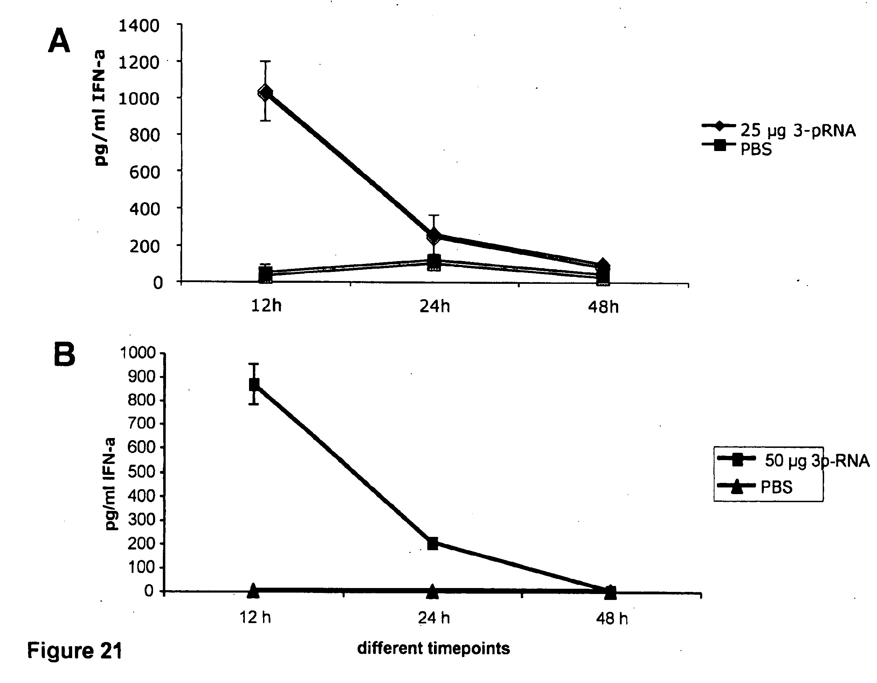

### Figure 21: IFN-α production induced by 5' triposphate siRNA in vivo is time- dependent

C57/BL6 mice were injected with 25µg (A) or 50µg (B) 3p-2.2 complexed with jetPEI™. Serum was' collected after 12 h, 24 h, and 48 h unless indicated otherwise, IFN-a and IL-12p40 concentrations were measured by ELISA. The results for IFN-α is shown. Similary result was obtained for IL-12p40 production (data not shown). Data are shown as means ± SEM of 4 independent experiments.

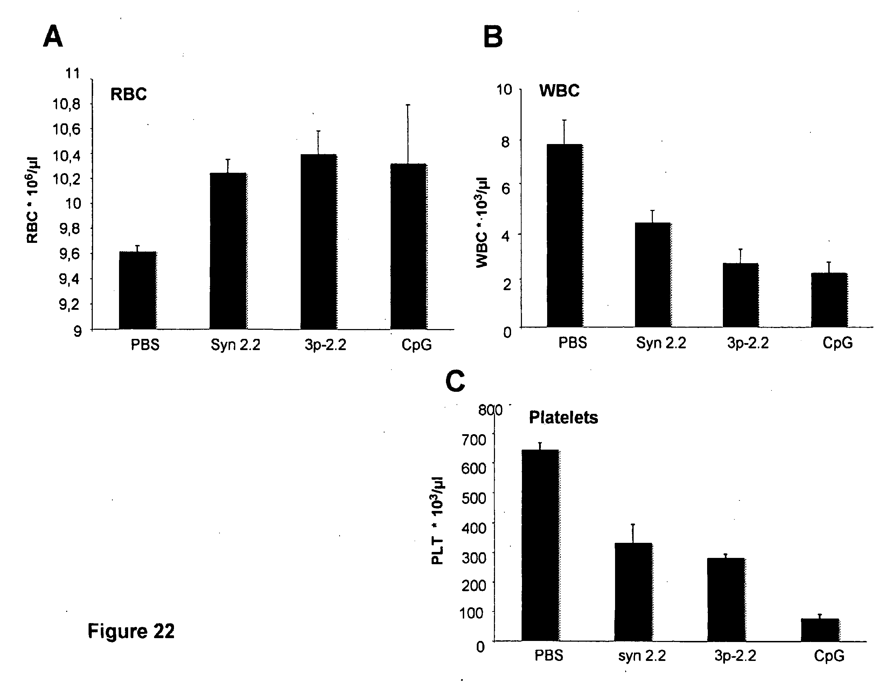

### Figure 22: 5' triposphate siRNA induces moderate thrombocytopenia and leukopenia in vivo.

C57/BL6 mice were injected with 50 µg 3p-2.2 complexed with jetPEI™. Blood was collected after 48 h and processed as EDTA plasma for measurement of platelets, leucocytes, and red blood cells. Blood cell counts were performed at the Central Laboratory of the Department of Internal Medicine, University of Munich, at the indicated time point. Data are shown as means ± SEM of 2 independent experiments.

### Figure 23: 5' triphosphate siRNA induces IFN-α in distinct murine immune cell subsets

Distinct immune cell subpopulations were stimulated as described previously. IFN-α production was measured 24 hours after stimulation by ELISA. Data are shown as means ± SEM of 4 independent experiments.

### Figure 24: Activation of distinct immune cell subsets is independent of TLR7

Conventional dendritic cells (cDC) (A), plasmacytoid dentric cells (PDC) (B), and myloid dendritic cells (MDC) (C) from wildtype C57/BL6 and TLR7-/- mice were stimulated with 200 ng of 5'-triphosphate RNA complexed with Lipofectamine. 24 h after stimulation, IFN-α secretion was measured in the supernatant by ELISA. Data are shown as means ± SEM of 3 independent experiments.

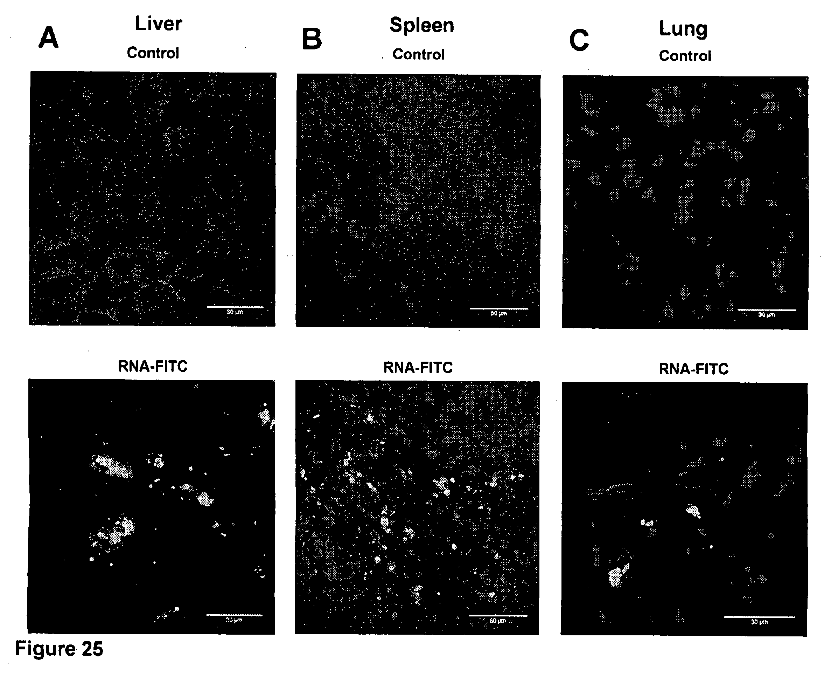

### Figure 25: In vivo distribution of jetPEI-complexed siRNA

C57/BL6 mice were injected intravenously with FITC-labelled siRNA (50 µg) complexed to jetPEI™ (Biomol). 3h after injection, mice were sacrificed and the selected organs were harvested and analyzed for uptake of the RNA complexes using a Zeiss LSM510 confocal mircroscope (Carl Zeiss, Germany) equiped with 488nm-Argon and 633nm-Helium-Neon lasers. Images were obtained taking one optical section of the cells.

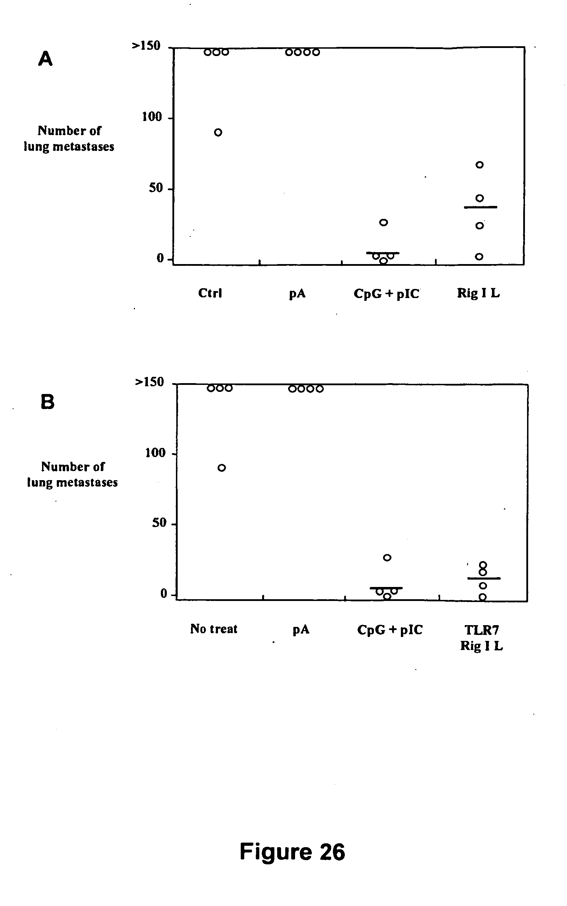

### Figure 26: In vivo delivery of RIG-I ligand with and without TLR-7 *ligand reduces growth of melanoma lung metastases.

Groups of 4 C57BL/6 mice were challenged with 4x10⁵ B16 melanoma cells intravenously to experimentally induce lung metastases. Mice were treated intravenously with 25 µg/ injection of CpG 1826, poly (I:C), poly(A) (a synthetic 19mer RNA), RIG-I L (3p-GA, 5'-TRI-GGGGGGGGGGGAAAAAAAAAAAA-3') and TLR7-RIG-I L (TLR7 and RIG-I double ligand, 3p-2.2) on day 3, 6 and 9 as indicated. Control groups received 5% glucose. Tumor growth was assessed two weeks later by counting the number of metastases on the lung surfaces 14 days after challenge. Shown are the number of lung metastases in individual mice of a representative experiment. The mean number of metastases is indicated by the horizontal bar.

### Detailed Description of the Invention

Detection of viral infection is vital for higher organisms to safeguard the integrity of their genome. TLRs contribute to recognition of viral nucleic acids, but their proper function seems largely dispensable for effective antiviral defense (A. Krug et al., Immunity 21, 107 (Jul, 2004); K. Tabeta et al., Proc Natl Acad Sci U S A 101, 3516 (Mar 9, 2004); T. Delale et al., J Immunol 175, 6723 (Nov 15, 2005); K. Yang et al., Immunity 23, 465 (Nov, 2005)). It was not until recently that it became clear that the two cytoplasmic helicases, MDA-5 and RIG-I (M. Yoneyama et al., Nat Immunol 5, 730 (Jul, 2004)), are essential for controling viral infection.

The present inventors identified RNA with a triphosphate group at the 5'end and an optimal minimal length of 19 nucleotides as a specific ligand for RIG-I. The RIG-I ligand activity of 5'triphosphate RNA is independent of double-stranded RNA formation, since single- and double-stranded 5'triphosphate RNA molecules are equally active. Both exogenous 5' triphosphate RNA transfected into a cell and endogenously formed 5' triphosphate RNA activate RIG-I. Genomic RNA prepared from a negative strand RNA virus and RNA prepared from virus-infected cells but not RNA from non-infected cells triggered a potent IFN-α response in a 5' triphosphate-dependent manner. Binding studies of RIG-I and 5' triphosphate RNA reveal a direct molecular interaction.

Furthermore, the present inventors found that surprisingly, the RIG-I activity of a 5' triphosphate RNA oligonucleotide with an adenosine at the 5' end was higher than the identical molecule with a guanosine at the 5' end. Moreover, the present inventors found that the 3'sequence of a 5' triphosphate RNA had little impact as short 5' triphosphate RNA oligonucleotides with poly A, poly U, poly C or poly G at the 3'end had similar activity. At the same time, the present inventors found that the 5' nucleotide sequence of a 5' triphosphate RNA influences the type I IFN-inducing activity of the oligonucleotide. In contrast to short oligonucleotides, long 5' triphosphate RNA showed different levels of activity. This may be explained by secondary structure formation of long RNA molecules that could affect accessibility of the 5'triphosphate end for RIG-I.

Uncapped, unmodified 5' triphosphate RNA is the first well-defined molecular structure of viral nucleic acids that is detected by eukaryotic cells. Since viruses due to their lifecycle are composed of the same molecular constituents as their host cells, namely protein and nucleic acid, such defined molecular structures that allow discrimination of viral and self RNA are expected to be rare and the presence of such has been questioned. In this regard, viruses are different from bacteria that contain a variety of molecules such as endotoxin which are absent in eukaryotes and which are easily recognized with high confidence by TLRs such as TLR4 located in the cytoplasmic membrane.

Until now, localization of viral nucleic acids in the endosome rather than a specific molecular feature of viral nucleic acids was thought to be the major factor allowing the detection of viruses. Although TLR-mediated recognition of single-stranded RNA (by TLR7 and TLR8) and of short double-stranded RNA (by TLR7) in the endosome was found to be sequence dependent, the frequency of such sequence motifs in viruses and vertebrates is similar (unpublished observation by the present inventors). This applies even to CpG motifs, which are suppressed in both vertebrate and viral but not bacterial DNA (A. M. Krieg, Annu Rev Immunol 20, 709 (2002)). This view is supported by a recent study demonstrating that endosomal localization of TLR9 prevents recognition of self DNA and facilitates detection of viral DNA (G. M. Barton, J. C. Kagan, R. Medzhitov, Nat Immunol 7, 49 (Jan, 2006)). CpG motif independent recognition of DNA by TLR9 has been described by others (J. Vollmer et al., Antisense Nucleic Acid Drug Dev 12, 165 (Jun, 2002)).

Given the fact that all primer-independent RNA transcripts are initially generated as 5' triphosphate RNAs, the question arises how eukaryotic RNA evades the recognition of RIG-I. In the cytosol of eukaryotic cells, most if not all self RNA species do not carry a free 5' triphosphate end. Before self RNA leaves the nucleus and reaches the cytosol, RNA is further processed. This holds true for RNA transcripts of all three RNA polymerases in eukaryotes.

Polymerase I transcribes a large polycistronic precursor ribosomal RNA (rRNA) which contains the sequences for the mature rRNAs (18, 5.8S, 25-28S rRNA), two external transcribed spacers and two internal transcribed spacers. This primary transcript is subjected to many endo- and exonucleolytic-processing steps to produce the mature rRNAs. The net result of this maturing process is a monophosphate group at the 5' end of all polymerase I transcribed rRNAs (M. Fromont-Racine et al., Gene 313, 17 (Aug 14, 2003)).

Messenger RNAs (mRNAs) and small nuclear RNAs (snRNAs), which are transcribed by polymerase II, receive a 7' methyl guanosine group that is attached to the 5' triphosphate of the nascent RNA by a process called capping (A. J. Shatkin, J. L. Manley, Nat Struct Biol 7, 838 (Oct, 2000)). Thus, upon export into the cytoplasm, no free triphosphate groups are found in polymerase II transcripts.

Polymerase III synthesizes transfer RNAs (tRNAs) and rRNA 5S that are both exported in to the cytoplasm, and other small RNAs including U6 RNA. Prior to the export into the cytoplasm, tRNAs are further matured in the nucleus, including the removal of various nucleotides from the 5' end by ribonuclease P. Therefore all mature tRNAs that can be found in the cytoplasm have been processed at the 5' end resulting in a 5' monophosphate (S. Xiao et al. Annual review of biochemistry 71, 165 (2002)). The phosphorylation status of the 5' end of the ribosomal RNA 5S has not been studied and at present is unknown. U6 RNA receives a γ-monomethylphosphate (mpppG) cap structure following transcription (R. Singh, R. Reddy, PNAS 86, 8280 (Nov, 1989)).

In addition to the lack of free 5' triphosphate residues, eukaryotic RNA posttranscriptionally undergoes significant modification of its nucleosides and its ribose backbone. Among all nucleoside modifications, pseudouridinylation is one of the most common posttranscriptional modifications of RNA that appears to be universal among rRNAs and small stable RNAs such as splicing small nuclear RNAs (snRNAs), tRNAs, and small nucleolar RNAs (snoRNAs). However, the frequency and location of pseudouridinilated nucleotides vary phylogenetically. Intriguingly, eukaryotes contain far more nucleoside modifications within their RNA species than prokaryotes. Human ribosomal RNA for example, the major constituent of cellular RNA, contains ten times more pseudouridine (ψ) and 25 times more 2'-O-methylated nucleosides than *E. coli* rRNA (J. Rozenski et al. Nucleic acids research 27, 196 (Jan 1, 1999)). The same applies for eukaryotic tRNAs, the most heavily modified subgroup of RNA with up to 25% of modified nucleosides. The host machinery that carries out nucleoside modifications and 2'-O-methylation of the ribose backbone is located in the nucleolus and consists of RNA-protein complexes containing snoRNAs and several associated proteins (i.e., snoRNPs) (W. A. Decatur, M. J. Fournier, J. Biol. Chem. 278, 695 (January 3, 2003)).

Information on nucleolus specific nucleoside modifications or ribose 2'-O-methylation of viral RNA genomes is limited. Since most RNA viruses do not replicate in the nucleus and modification is tightly confined to the sequence and structure of their target, extensive modification of viral RNA seems unlikely.

Altogether, posttranscriptional modifications of eukaryotic RNA such as 5' processing or capping as well as nucleoside modifications or ribose backbone methylation provide the molecular basis for the distinction of self RNA generated in the nucleus from viral RNA of cytoplasmic origin.

The mRNAs of viruses infecting eukaryotic cells also commonly contain 7-methyl guanosine cap-structures at their 5'ends and poly(A) tails at their 3'ends (Y. Furuichi, A. J. Shatkin, Adv Virus Res 55, 135 (2000)). Some viruses make use of the host transcription machinery to acquire caps and poly(A) tails. RNA viruses that do not rely on the host transcriptional machinery produce their own capping enzymes or utilize other mechanisms such as snatching the 5' -terminal regions of host mRNAs. Despite these adaptations of viruses to the host transcriptional system, viral RNA synthesis leads to transient cytoplasmic RNA intermediates with an uncapped 5'triphosphate end.

With notable exceptions such as the Picornavirus family (see below), viral RNA-dependent RNA polymerases (RdRp) initiate polymerase activity de novo without a specific primer (C. C. Kao, et al., Virology 287, 251 (Sep 1, 2001)). As a consequence, these RdRp-dependent transcripts start with an uncapped 5' triphosphate. This has been studied in great detail for the replication of postive strand RNA viruses of the family of Flaviviridae (including Hepatitis C Virus, Yellow Fever Virus, Japanese Encaphilitis Virus and Dengue Virus); all of these viruses were reported to be recognized via RIG-I (H. Kato et al., Nature 441, 101 (Apr 9, 2006); R. Sumpter, Jr. et al., J. Virol. 79, 2689 (March 1, 2005, 2005); T.-H. Chang et al., Microbes and Infection 8, 157 (2006)). Segmented negative strand RNA virus (NSV) rely on a cap-snatched primer for mRNA transcription, yet initiate genomic and the complementary antigenomic RNA replication by a primer indpendent de novo mechanism resulting in a 5' triphosphate initated transcript (A. Honda, et al., Virus Res 55, 199 (Jun, 1998); G. Neumann, et al., Current topics in microbiology and immunology 283, 121 (2004)). NSV with a nonsegmented genome (Order *Mononegavirales),* including the Paramyxoviruses and Rhabdoviruses, initiate both replication and transcription de novo leading to 5' triphosphate RNA in the cytosol. Both the full length replication products, vRNA and cRNA, and a short leader RNA which is abundantly synthesized during initiation of transcription, maintain their 5' triphosphate (R. J. Colonno, A. K. Banerjee, Cell 15, 93 (1978)), while the virus-encoded mRNA transcripts are further modified at their 5' ends by capping and cap methylation. Consequently, genomic RNA from NSVs per se is expected to trigger an IFN-response without the need for replication and presumed dsRNA formation. Consistent with this notion, not only live virus but also RNA purified from NSV virions, in this case, VSV, has been shown to trigger strong type I interferon responses depending on RIG-I (H. Kato et al., Nature 441, 101 (Apr 9, 2006)).

The present inventors confirmed and extended these observations by demonstrating that dephosphorylation of the viral RNA isolates completely abolished the IFN response, thereby indicating that the 5' triphosphate moiety is strictly required for recognition. In case of RV-infected cells, full length RNAs are permanently enclosed within nucleoprotein (N) to form a linear, helical nucleoprotein-RNA complex (RNP) in which the RNA is not accessible to even small cellular molecules such as RNases. Similarly, leader RNA has been reported to be encapsulated by N (Blumberg BM, Kolakofsky D., J Virol. 1981 Nov;40(2):568-76; Blumberg BM, Leppert M, Kolakofsky D., Cell 1981 Mar;23(3):837-45). The effective recognition of live NSV by RIG-I may suggest that the terminal triphosphates of the linear N-RNA complex are not completely protected by N protein or that in the initial phase of viral transcription, the levels of newly synthesized N protein are insufficient for complete protection. In this respect, it is interesting to note that NSV stocks that contain defective interfering (DI) particle RNAs are potent inducers of IFN (Strahle L. et al. 2006, Virology 351(1):101-11). DIs only contain the terminal promoters for replication and provide plentiful 5' triphosphate ends under conditions of reduced expression of helper virus proteins.

On the other hand, all viruses in the Picornavirus-like supergroup (picoma-, poty-, como-, calici- and other viruses) use a RdRp which exclusively employs a protein as a primer for both positive and negative strand RNA production: this protein primer is part of the precursor RdRp and is cleaved off as elongation of the initial complex occurs, to become a 5'-genome-linked protein, usually known as viral genome-linked protein (VPg) (Y. F. Lee, et al., Proc Natl Acad Sci U S A 74, 59 (Jan, 1977)). Thus during the lifecycle of Picomaviruses, uncapped, triphosphorylated 5' ends are absent. Consequently, RIG-I is expected to be involved in the detection of Flaviviridae and NSV but not picomaviruses, which was confirmed in a recent study (H. Kato et al., Nature 441, 101 (Apr 9, 2006)).

Prior to the present invention, long double-stranded RNA was believed to be the only defined nucleic acid structure that occurs during viral infection but is absent in normal cells. The notion that the long double-stranded RNA mimic poly(I:C) induces type I IFNs dates back to the early days of type I IFN research (M. Absher, W. R. Stinebring, Nature 223, 715 (Aug 16, 1969)). Double-stranded RNA-dependent protein kinase (PKR) was thought to be involved in I FN-α induction (S. D. Der, A. S. Lau, Proc Natl Acad Sci U S A 92, 8841 (Sep 12, 1995)) but Weissmann's group demonstrated that poly(I:C)-induced type I IFN is not impaired in PKR deficient mice (Y. L. Yang et al., Embo J 14, 6095 (Dec 15, 1995)). Others found that poly(I:C)-induced type I IFN was partially dependent on PKR but independent of TLR3 (S. S. Diebold et al., Nature 424, 324 (Jul 17, 2003)). On the other hand, TLR3 was the first receptor proven to specifically bind long dsRNA and to induce type I IFN upon binding (L. Alexopoulou, et al., Nature 413, 732 (Oct 18, 2001)). TLR3 was found to be activated during viral infection (in the case of CMV) (K. Tabeta et al., Proc Natl Acad Sci U S A 101, 3516 (Mar 9, 2004)), but was not required for viral clearance (in the case of RSV) (B. D. Rudd et al., J Immunol 176, 1937 (Feb 1, 2006)).

A number of studies suggested that the helicases MDA-5 and RIG-I recognize dsRNA (M. Yoneyama et al., Nat Immunol 5, 730 (Jul, 2004); S. Rothenfusser et al., J Immunol 175, 5260 (Oct 15, 2005); J. Andrejeva et al., Proc Natl Acad Sci U S A 101, 17264 (Dec 7, 2004)). However, the present inventor found that double-strand formation of RNA is not required for RIG-I-RNA interaction and that dsRNA is not sufficient for RIG-I activation. The present inventors further found that MDA-5 is not involved in 5' triphosphate RNA recognition. Although there is convincing evidence that MDA-5 is activated by the long dsRNA mimic poly(I:C), activation of MDA-5 by natural long dsRNA is still controversial (H. Kato et al., Nature 441, 101 (Apr 9, 2006)). Taken together, TLR3 so far is the only receptor that leads to the production of type I IFN upon binding of the natural long dsRNA molecule, but the contribution of TLR3 to type I IFN induction and viral clearance *in vivo* seems to be weak.

It is widely assumed that replication of both DNA and RNA viruses is associated with the formation of intermediate dsRNA in the cytoplasm. A recent study confirms the formation of intermediate dsRNA for positive strand RNA viruses, dsRNA viruses and DNA viruses but not NSV (F. Weber, et al., J Virol 80, 5059 (May, 2006)). However, formation of endogenous dsRNA occurs physiologically in eukaryotic cells. In healthy eukaryotic cells, dsRNA is present in the form of micro RNAs (miRNA) and precursor-miRNAs. Precursor-miRNA are 70-nucleotide dsRNA stem-loop structures that are constantly exported from the nucleus into the cytosol to be further processed into 22 nucleotides miRNAs which posttranscriptionally regulate a large number of target genes (B. R. Cullen, Mol Cell 16, 861 (Dec 22, 2004)). Therefore, dsRNA is present in normal healthy eukaryotic cells without inducing an type I IFN response. Therefore, dsRNA in the cytoplasm per se is not virus-specific.

There is good evidence that short dsRNA such as siRNA generated by Dicer-mediated cleavage of long dsRNA does not elicit a type I IFN response in non-immune cells (V. Hornung et al., Nat Med 11, 263 (Mar, 2005); D. H. Kim et al., Nat Biotechnol 22, 321 (Mar, 2004); S. M. Elbashir et al., Nature 411, 494 (May 24, 2001)). A recent study suggests that the two nucleotides overhang at the 3' end of dicer cleavage products are essential for the lack of immunorecognition of short dsRNA (J. T. Marques et al., Nat Biotechnol 24, 559 (May, 2006)). In the same study, it was proposed that synthetic blunt end short dsRNA is recognized via RIG-I. The conclusion that RIG-I is the receptor for blunt end short dsRNA is based on experiments using RIG-I overexpressing cells and using RIG-I specific siRNA (short dsRNA with two nucleotides 3' overhangs) on top of stimulation with blunt end short dsRNA. RIG-I deficient cells were not examined in this study.

It is well known that 5' triphosphate independent recognition of short dsRNA as well as ssRNA occurs in the endosomal compartment of a highly specialized subset of immune cells, the plasmacytoid dendritic cell (PDC). PDC carry only two functional TLRs, TLR7 for the detection of RNA, and TLR9 for the detection of DNA. In humans, TLR-induced IFN-α induction is largely confined to PDC. It has been reported that PDC are responsible for the early induction of IFN-α during viral infection (A. Krug et al., Immunity 21, 107 (Jul, 2004)).

However, depleting PDC has no major impact on host survival after viral infection (T. Delale et al., J Immunol 175, 6723 (Nov 15, 2005)). Based on these data, a concept is evolving that PDC contribute to early antiviral immune responses, while the major antiviral activity is based on cytoplasmic recognition of the virus via RIG-I and/or MDA-5. In situations where the virus escapes recognition of RIG-I and/or MDA-5, PDC and TLR-mediated virus recognition may play a more critical role. Thus, PDC serve as sentinels for viral particles before it comes to viral replication in virus-infected cells, and may serve as a backup strategy if the virus escapes RIG-I and/or MDA-5 recognition.

The potency of the 5' triphosphate RNA specific antiviral response is illustrated by the finding of the present inventors that human primary monocytes produce large amounts of IFN-α upon stimulation with 5' triphosphate RNA. Unlike in mice (S. S. Diebold et al., Nature 424, 324 (Jul 17, 2003)), human myeloid cells have not been shown previously to produce considerable amounts of IFN-α upon stimulation with nucleic acids. With 5' triphosphate RNA, now for the first time a molecule is available which is a real mimic of viral infection of cells and consequently is capable of inducing IFN-α in any cell type including immune cells that normally do not make IFN-α, non-immune cells and tumor cells.

Prior to the present invention, the only way to induce a similar type of response was to use attenuated replicating viruses. However, attenuated viruses may cause viral infection and disease in immunosuppressed patients and mutations could eventually revert viruses to become more pathogenic. 5' triphosphate RNA has the potential to mimic attenuated replicating viruses with respect to their potent stimulation of immunity. In this respect, 5' triphosphate RNA seems to be the perfect biologically dead molecule which can be used in the development of vaccines, therapeutic vaccines, or immunotherapies for the prevention and/or treatment of established diseases such as chronic viral infection and tumors.

5' triphosphate RNA induces not only type I IFN production from tumor cells, but also apoptosis of the tumor cells. Tumor cells are more susceptible than non-tumor cells to apoptosis induced by 5' triphosphate RNA. Therefore, 5' triphosphate RNA is an ideal candidate for tumor therapy.

Furthermore, 5' triphosphate RNA is found to be capable of inducing IL-18 and IL-1β production. Without being bound to any theory, it is believed that 5' triphosphate is recognized by the NALP-3 receptor, leading to the production of IL-18 and IL-1β. Therefore, 5' triphosphate RNA may be useful in the treatment of diseases and/or conditions which may be alleviated by the induction of these respective cytokines. The dieases and/or conditions include, but are not limited to, allergies, malignant and benign tumors, viral infections, bacterial infections (in particular, intracellular bacterial infections), immunodeficiencies and Immunosuppression (including bone marrow suppression by cytotoxic chemotherapy).

Another surprising finding of the present inventors is that, in addition to *in vitro* transcribed RNA and viral RNA, bacterial RNA is very potent in inducing a type I IFN response. Similar to *in vitro* transcribed RNA and viral RNA, bacterial RNA contains a 5' triphosphate and lacks the eukaryotic cell-specific modifications. Even more surprisingly, it was found that the IFN-inducing activity of bacterial RNA is not entirely attributable to the presence of the 5' triphosphate, as is the case with *in vitro* transcribed RNA. Therefore, in addition to 5' triphosphate, bacterial RNA contains further molecular features which are responsible for its ability to be recognized by eukaryotic cells and to induce type I IFN production.

This surprising finding of the present inventors opens up a new venue in the development of pharmaceutical compositions which are capable of inducing an anti-viral response and/or an anti-bacterial response and are useful for the treatment of diseases such as viral infections, bacterial infections, (in particular, intracellular bacterial infections), tumors, allergy, autoimmune diseases and immunodeficiencies.

Bacterial RNA is advantageous over attenuated virus and viral RNA as a therapeutic agent because of its safety profile. Whereas attenuated virus may cause viral infection and disease and viral RNA may integrate into the eukaryotic genome causing unwanted genetic alteration, bacterial RNA is inert and does not cause any undesirable diseases or conditions.

In addition, bacterial RNA can be produced in large quantities at very low cost. Therefore, it is a lot more economical to use bacterial RNA as a therapeutic agent than attenuated virus, viral RNA, or *in vitro* transcribed RNA.

### Definitions

As used herein, "a" and "an" refers to not only a single individual, but also a group or species of entities.

### oligonucleotide

As used herein, the term "oligonucleotide" refers to a polynucleotide formed from a plurality of linked nucleoside units; "oligonucleotide" and "polynucleotide" are used synonymously. Such oligonucleotides can be obtained from existing nucleic acid sources, including genomic or cDNA, but are preferably produced by synthetic methods including chemical synthesis, *in vitro* and *in vivo* transcription. In preferred embodiments each nucleoside unit includes a heterocyclic base and a pentofuranosyl, trehalose, arabinose, 2'-deoxy-2'-substituted arabinose, 2'-O-substituted arabinose or hexose sugar group. The nucleoside residues can be coupled to each other by any of the numerous known internucleoside linkages. Such internucleoside linkages include, without limitation, phosphodiester, phosphorothioate, phosphorodithioate, pyrophosphate, alkylphosphonate, alkylphosphonothioate, phosphotriester, phosphoramidate, siloxane, carbonate, carboalkoxy, acetamidate, carbamate, morpholino, borano, thioether, bridged phosphoramidate, bridged methylene phosphonate, bridged phosphorothioate, and sulfone internucleoside linkages. The term "oligonucleotide" also encompasses polynucleosides having one or more stereospecific internucleoside linkage (e.g., (Rₚ)- or (Sₚ)-phosphorothioate, alkylphosphonate, or phosphotriester linkages).

The oligonucleotides of the invention can include naturally occurring nucleosides, modified nucleosides, or mixtures thereof. As used herein, the term "modified nucleoside" is a nucleoside that includes a modified heterocyclic base, a modified sugar moiety, or a combination thereof. In some embodiments, the modified nucleoside is a non-natural pyrimidine or purine nucleoside. In some embodiments, the modified nucleoside is a 2'-substituted ribonucleoside, an arabinonucleoside or a 2'-deoxy-2'-substituted-arabinoside.

As used herein, the term "2'-substituted ribonucleoside" or "2'-substituted arabinoside" includes ribonucleosides or arabinonucleoside in which the hydroxyl group at the 2' position of the pentose moiety is substituted to produce a 2'-substituted or 2'-O-substituted ribonucleoside. Preferably, such substitution is with a lower alkyl group containing 1-6 saturated or unsaturated carbon atoms, or with an aryl group having 6-10 carbon atoms, wherein such alkyl, or aryl group may be unsubstituted or may be substituted, e.g., with halo, hydroxy, trifluoromethyl, cyano, nitro, acyl, acyloxy, alkoxy, carboxyl, carboalkoxy, or amino groups. Examples of 2'-O-substituted ribonucleosides or 2'-O-substituted-arabinosides include, without limitation, 2'-O-methylribonucleosides or 2'-O-methylarabinosides and 2'-O-methoxyethylribonucleosides or 2'-O-methoxyethylarabinosides.

The term "2'-substituted ribonucleoside" or "2'-substituted arabinoside" also includes ribonucleosides or arabinonucleosides in which the 2'-hydroxyl group is replaced with a lower alkyl group containing 1-6 saturated or unsaturated carbon atoms, or with an amino or halo group. Examples of such 2'-substituted ribonucleosides or 2'-substituted arabinosides include, without limitation, 2'-amino, 2'-fluoro, 2'-allyl, and 2'-propargyl ribonucleosides or arabinosides.

The term "oligonucleotide" includes hybrid and chimeric oligonucleotides. A "chimeric oligonucleotide" is an oligonucleotide having more than one type of internucleoside linkage. One preferred example of such a chimeric oligonucleotide is a chimeric oligonucleotide comprising a phosphorothioate, phosphodiester or phosphorodithioate region and non-ionic linkages such as alkylphosphonate or alkylphosphonothioate linkages (see e.g., U.S. Pat. Nos. 5,635,377 and 5,366,878).

A "hybrid oligonucleotide" is an oligonucleotide having more than one type of nucleoside. One preferred example of such a hybrid oligonucleotide comprises a ribonucleotide or 2'-substituted ribonucleotide region, and a deoxyribonucleotide region (see, e.g., U.S. Pat. Nos. 5,652,355, 6,346,614 and 6,143,881).

RNA oligonucleotides discussed herein include otherwise unmodified RNA as well as RNA which have been modified (*e.g.*, to improve efficacy), and polymers of nucleoside surrogates.

Unmodified RNA refers to a molecule in which the components of the nucleic acid, namely sugars, bases, and phosphate moieties, are the same or essentially the same as that which occur in nature, preferably as occur naturally in the human body. The art has referred to rare or unusual, but naturally occurring, RNAs as modified RNAs, see, *e.g*., Limbach et al. 1994, Nucleic Acids Res 22: 2183-2196. Such rare or unusual RNAs, often termed modified RNAs (apparently because these are typically the result of a post-transcriptional modification) are within the term unmodified RNA, as used herein.

Modified RNA as used herein refers to a molecule in which one or more of the components of the nucleic acid, namely sugars, bases, and phosphate moieties, are different from that which occurs in nature, preferably different from that which occurs in the human body. While they are referred to as modified "RNAs," they will of course, because of the modification, include molecules which are not RNAs.

Nucleoside surrogates are molecules in which the ribophosphate backbone is replaced with a non-ribophosphate construct that allows the bases to the presented in the correct spatial relationship such that hybridization is substantially similar to what is seen with a ribophosphate backbone, *e.g*., non-charged mimics of the ribophosphate backbone.

All nucleic acid sequences listed herein are in the 5' to 3' direction unless otherwise indicated.

The RNA oligonucleotide of the invention can be single-stranded (ssRNA), double-stranded (dsRNA), or partially double-stranded (partially dsRNA).

A single-stranded RNA oligonucleotide may contain self-complementary sequences and forms a hairpin. For example, 5'-GACCTAGCCTAAAACTAGGTC-3'. The self-complementary sequence may be a palindromic sequence. For example, 5'AAAGATCCGGATCAAAA-3'.

A double-stranded RNA oligonucleotide may have one- or two-nucleotide overhang at the 5' or 3' end of one or both strands.

A partially double-stranded RNA oligonucleotide may comprise two strands of the same or different length(s), wherein at least one of the strands contains nucleotides outside the complementary sequence. For example,

| | |
|---|---|
| Example 1: | 5'-AAAAGUUCAAAGCUCAAAA-3' |
| | 3'-CAAGUUUCGAG-5' |
| Example 2: | 5'-UCAAAGUCAAAAGCUCAAAGUUGAAAGUUUAAA-3' |
| | 3'-GACUUGAAAAUUUCAGUUUUCGAGUUUAAGUUGAAAACUCG-5' |
| Example 3: | 5'-UCAAAGUCAAAAGCUCAAAGUUGAAA-3' |
| | 3'- UUUCAGUUUUCGAGUUUAAGUUGAAAACUCG-5' |

The length of a single-stranded RNA oligonucleotide is the number of nucleotides contained in the oligonucleotide.

In the case of a double-stranded or partially double-stranded oligonucleotide, the length of the oligonucleotide is the length of the individual strands. In other words, a partially double-stranded oligonucleotide can have two lengths.

### Enhanced Nuclease Resistance

For increased nuclease resistance and/or binding affinity to the target, an oligonucleotide can include, for example, 2'-modified ribose units and/or phosphorothioate linkage(s) and/or pyrophosphate linkage(s). For example, the 2' hydroxyl group (OH) can be modified or replaced with a number of different "oxy" or "deoxy" substituents.

Examples of "oxy"-2' hydroxyl group modifications include alkoxy or aryloxy (OR, e.g., R = H, alkyl, cycloalkyl, aryl, aralkyl, heteroaryl or sugar); polyethyleneglycols (PEG), O(CH₂CH₂O)ₙCH₂CH₂OR; "locked" nucleic acids (LNA) in which the 2' hydroxyl is connected, *e.g*., by a methylene bridge, to the 4' carbon of the same ribose sugar; O-AMINE and aminoalkoxy, O(CH₂)ₙAMINE, (*e.g*., AMINE = NH₂; alkylamino, dialkylamino, heterocyclyl amino, arylamino, diaryl amino, heteroaryl amino, or diheteroaryl amino, ethylene diamine, polyamino). It is noteworthy that oligonucleotides containing only the methoxyethyl group (MOE), (OCH₂CH₂OCH₃, a PEG derivative), exhibit nuclease stabilities comparable to those modified with the robust phosphorothioate modification.

"Deoxy" modifications include hydrogen (*i.e*. deoxyribose sugars, which are of particular relevance to the overhang portions of partially dsRNA); halo (*e.g.*, fluoro); amino (*e.g*.. NH₂; alkylamino, dialkylamino, heterocyclyl, arylamino, diaryl amino, heteroaryl amino, diheteroaryl amino, or amino acid); NH(CH₂CH₂NH)ₙCH₂CH₂-AMINE (AMINE = NH₂; alkylamino, dialkylamino, heterocyclyl amino, arylamino, diaryl amino, heteroaryl amino,or diheteroaryl amino), -NHC(O)R (R = alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with *e.g*., an amino functionality.

Preferred substitutents are 2'-methoxyethyl, 2'-OCH3, 2'-O-allyl, 2'-C- alkyl, and 2'-fluoro.

To maximize nuclease resistance, the 2' modifications can be used in combination with one or more phosphate linker modifications (*e.g*., phosphorothioate). The so-called "chimeric" oligonucleotides are those that contain two or more different modifications.

The inclusion of furanose sugars in the oligonucleotide backbone can also decrease endonucleolytic cleavage. An oligonucleotide agent can be further modified by including a 3' cationic group, or by inverting the nucleoside at the 3'-terminus with a 3'-3' linkage. In another alternative, the 3'-terminus can be blocked with an aminoalkyl group, e.g., a 3' C5-aminoalkyl dT. Other 3' conjugates can inhibit 3'-5' exonucleolytic cleavage. While not being bound by theory, a 3' conjugate, such as naproxen or ibuprofen, may inhibit exonucleolytic cleavage by sterically blocking the exonuclease from binding to the 3'-end of oligonucleotide. Even small alkyl chains, aryl groups, or heterocyclic conjugates or modified sugars (D-ribose, deoxyribose, glucose etc.) can block 3'-5'-exonucleases.

Similarly, 5' conjugates can inhibit 5'-3' exonucleolytic cleavage. While not being bound by theory, a 5' conjugate, such as naproxen or ibuprofen, may inhibit exonucleolytic cleavage by sterically blocking the exonuclease from binding to the 5'-end of oligonucleotide. Even small alkyl chains, aryl groups, or heterocyclic conjugates or modified sugars (D-ribose, deoxyribose, glucose etc.) can block 5'-3'-exonucleases.

Single-stranded RNA oligonucleotides which contain self-complementary sequences and form a hairpin structure have enhanced nuclease resistance compared to single-stranded oligonucleotides which do not.

### Tethered Ligands

The RNA oligonucleotides of the present invention also include those with tethered ligands. The properties of a RNA oligonucleotide, including its pharmacological properties, can be influenced and tailored by the introduction of ligands, e.g. tethered ligands.

The ligands may be coupled, covalently or non-covalently, preferably covalently, either directly or indirectly via an intervening tether, to the RNA oligonucleotide. In preferred embodiments, the ligand is attached to the oligonucleotide via an intervening tether.

In preferred embodiments, a ligand alters the distribution, targeting or lifetime of a RNA oligonucleotide into which it is incorporated. In preferred embodiments, a ligand provides an enhanced affinity for a selected target, e.g., molecule, cell or cell type, a cellular or organ compartment, tissue, organ or region of the body.

Preferred ligands can improve transport, hybridization, and specificity properties and may also improve nuclease resistance of the resultant natural or modified oligoribonucleotide, or a polymeric molecule comprising any combination of monomers described herein and/or natural or modified ribonucleotides.

A wide variety of ligands may be used. Ligands may include agents that allow for the specific targeting of the oligonucleotide; diagnostic compounds or reporter groups which allow for the monitoring of oligonucletotide distribution; cross-linking agents; nuclease-resistance conferring moieties; and natural or unusual nucleobases. General examples include lipophilic moleculeses, lipids, lectins, steroids (e.g.,uvaol, hecigenin, diosgenin), terpenes (e.g., triterpenes, e.g., sarsasapogenin, Friedelin, epifriedelanol derivatized lithocholic acid), vitamins, carbohydrates(e.g., a dextran, pullulan, chitin, chitosan, inulin, cyclodextrin or hyaluronic acid), proteins, protein binding agents, integrin targeting molecules,polycationics, peptides, polyamines, and peptide mimics.

The ligand may be a naturally occurring or recombinant or synthetic molecule, such as a synthetic polymer, e.g., a synthetic poly amino acid. Examples of poly amino acids include, without limitation, poly L-lysine, poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacrylic acid), N-isopropylacrylamide polymers, or polyphosphazine. Example of polyamines include: polyethylenimine, poly lysine, spermine, spermidine, polyamine, pseudopeptide-polyamine, peptidomimetic polyamine, dendrimer polyamine, arginine, amidine, protamine, cationic moieties, e.g., cationic lipid, cationic porphyrin, quaternary salt of a polyamine, or an alpha helical peptide.

Ligands can also include targeting groups, e.g., a cell or tissue targeting agent, e.g., , a thyrotropin, melanotropin, surfactant protein A, Mucin carbohydrate, a glycosylated polyaminoacid, transferrin, bisphosphonate, polyglutamate, polyaspartate, or an RGD peptide or RGD peptide mimetic.

Ligands can be proteins, e.g., glycoproteins, lipoproteins, e.g. low density lipoprotein (LDL), or albumins, e.g. human serum albumin (HSA), or peptides, e.g., molecules having a specific affinity for a co-ligand, or antibodies e.g., an antibody, that binds to a specified cell type such as a cancer cell, endothelial cell, or bone cell. Ligands may also include hormones and hormone receptors. They can also include non-peptidic species, such as cofactors, multivalent lactose, multivalent galactose, N-acetyl-galactosamine, N-acetylglucosamine, multivalent mannose, or multivalent fucose. The ligand can be, for example, a lipopolysaccharide, an activator of p38 MAP kinase, or an activator of NF-κB.

The ligand can be a substance, e.g., a drug, which can increase the uptake of the oligonucleotide agent into the cell, for example, by disrupting the cell's cytoskeleton, e.g., by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

In one embodiment, the ligand is a lipid or lipid-based molecule. Such a lipid or lipid-based molecule preferably binds a serum protein, e.g., human serum albumin (HSA). An HSA binding ligand allows for distribution of the conjugate to a target tissue, e.g., liver tissue, including parenchymal cells of the liver. Other molecules that can bind HSA can also be used as ligands. For example, neproxin or aspirin can be used. A lipid or lipid-based ligand can (a) increase resistance to degradation of the conjugate, (b) increase targeting or transport into a target cell or cell membrane, and/or (c) can be used to adjust binding to a serum protein, e.g., HSA.

A lipid based ligand can be used to modulate the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

In another embodiment, the ligand is a moiety, e.g., a vitamin or nutrient, which is taken up by a target cell, e.g., a proliferating cell. These are particularly useful for treating disorders characterized by unwanted cell proliferation, e.g., of the malignant or non-malignant type, e.g., cancer cells. Exemplary vitamins include vitamin A, E, and K. Other exemplary vitamins include the B vitamins, e.g., folic acid, B12, riboflavin, biotin, pyridoxal or other vitamins or nutrients taken up by cancer cells.

In another embodiment, the ligand is a cell-permeation agent, preferably a helical cell-permeation agent. Preferably, the agent is amphipathic. An exemplary agent is a peptide such as tat or antennapedia. If the agent is a peptide, it can be modified, including a peptidylmimetic, invertomers, non-peptide or pseudo-peptide linkages, and use of D-amino acids. The helical agent is preferably an alpha-helical agent, which preferably has a lipophilic and a lipophobic phase.

In a preferred embodiment, the ligand is an antibody or a fragment thereof which is specific for a moiety present in a cell to be targeted. The moiety may be a protein, a carbohydrate structure, a polynucleotide, or a combination thereof. The moiety may be secreted, associated with the plasma membrane (e.g., on the extracellular or intracellular surface), cytosolic, associated with intracellular organelles (e.g., ER, Golgi complex, mitochondria, endosome, lysosome, secretory vesicle) or nuclear. The antibody may be monoclonal or polyclonal. The antibody may be chemeric or humanized. The antibody may be a single chain antibody. The antibody fragment may be a Fab fragment, a F(ab')_{z} fragment, or any fragments that retain the antigen-binding specificity of the intact antibody.

### immunostimulatory activity

As used herein, "immunostimulatory activity" refers to the capability of an agent, such as a molecule or a composition, to induce an immune response. In one embodiment, the immunostimulatory activity refers to the type I IFN-inducing activity, in particular, the IFN-α-inducing activity.

As used herein, "inducing an immune response" means initiating or causing an increase in one or more of B-cell activation, T-cell activation, natural killer cell activation, activation of antigen presenting cells (e.g., B cells, dendritic cells, monocytes and macrophages), cytokine production, chemokine production, specific cell surface marker expression, in particular, expression of co-stimulatory molecules. In one aspect, such an immune response involves the production of type I IFN (IFN-α and/or IFN-β), in particular, IFN-α, in cells such as PDC (plasmacytoid dendritic cells) and/or monocytes.

As used herein, "type I IFN inducing activity" includes IFN-α-inducing activity and/or IFN-β inducing activity.

As used herein, "IFN-α-inducing activity" refers to the capability of an agent, such as a molecule or composition, to induce IFN-α production from a cell capable of producing IFN-α. Cells capable of producing IFN-α include, but are not limited to, peripheral blood mononuclear cells (PBMC) (e.g., B cells, dendritic cells (myeloid dendritic cells and plasmacytoid dendritic cells), macrophages, monocytes, natural killer cells, granulocytes), endothelial cells, and cell lines.

As used herein, "IFN-β-inducing activity" refers to the capability of an agent, such as a molecule or composition, to induce IFN- β production from a cell capable of producing IFN-β. Any somatic cells, such as PBMC, myeloid dendritic cells, monocytes, PDC, fibroblasts, are capable of producing IFN- p.

### anti-viral response

As used herein, "anti-viral response" refers to the response by a cell, tissue or organism upon infection by a virus with the purpose of eliminating or incapacitating the virus. Typical anti-viral responses include, but are not limited to, type I IFN, MIP1-a, MCP, RANTES, IL-8, IL-6, IP-10, and IFN-γ production.

### anti-bacterial response

An anti-bacterial response is the response by a cell, tissue or organism upon infection by a bacterium with the purpose of eliminating or incapacitating the bacterium. Typical anti-bacterial responses include, but are not limited to, T cell or NK cell-mediated elimination of the infected cell by either receptor-mediated apoptosis or cytokine-mediated apoptosis via TNF or TRAIL, macorphage or monocytes phagocytosis.

An anti-bacterial response, in particular, type I and type II IFN production, may be induced in immune cells or non-immune cells. Immune cells include, but are not limited to, peripheral blood mononuclear cells (PBMC), plasmacytoid dendritric cells (PDC), myeloid dendritic cells (MDC), B cells, macrophages, monocytes, natural killer cells, NKT cells, CD4+ T cells, CD8+ T cells, granulocytes. Non-immune cells include, among others, tumor cells, epithelial cells, endothelial cells, and fibroblasts.

### disorder/disease-related gene and antigen

As used herein, "disorder/disease-related gene" refers to a gene that is expressed or overexpressed in a disease/disorder and that is not expressed or expressed in reduced amount under normal condition. For example, a mutant CF gene is expressed in cystic fibrosis patient but not in an individual without cystic fibrosis; ErbB2 (or Her2) is overexpressed in breast cancer cells compared to normal breast cells; a viral gene is expressed in infected cells but not in uninfected cells. The gene product of the disorder/disease-related gene is referred to herein as the "disorder/disease-related antigen".

### mammal

As used herein, the term "mammal" includes, without limitation, rats, mice, cats, dogs, horses, sheep, cattle, cows, pigs, rabbits, non-human primates, and humans.

### Oligonucleotide and Precursor Thereof

The present invention provides an oligonucleotide capable of inducing an anti-viral response, in particular, type I IFN production, wherein the oligonucleotide comprises a 5' triphosphate, wherein the oligonucleotide comprises at least 1, preferably at least 2, 3, 4, 5, more preferably at least 6, 7, 8, 9, 10, 11, even more preferably at least 12, 13, 14, 15, 16, 17, most preferably at least 18, 19, 20, 21 ribonucleotide(s) at the 5' end, and wherein the oligonucleotide is at least 12, preferably at least 18, more preferably at least 19, even more preferably at least 20, and most preferably at least 21 nucleotides in length.

The oligonucleotide of the invention may be single-stranded, single-stranded containing a self-complementary sequence which can form a hairpin structure, double-stranded, or partially double-stranded.

When the oligonucleotide is single-stranded, single-stranded containing a self-complementary sequence or double-stranded, the length of the oligonucleotide is the length of a single-strand.

When the oligonucleotide is partially double-stranded, the length of the oligonucleotide is the length of the longer strand. Therefore, the oligonucleotide of the present invention include partially double-stranded oligonucleotides wherein at least one of the strands is at least 12, 18, 19, 20 or 21 nucleotides in length.

In the oligonucleotide of the invention, the at least 1 ribonucleotide at the 5' end comprises the 5' triphosphate. In the case of a partially double-stranded oligonucleotide, the at least 1 ribonucleotide at the 5' end which comprises the 5' triphosphate can be on either the long or the short strand, wherein at least the long strand is at least 12, 18, 19, 20, or 21 nucleotides in length.

In the oligonucleotide of the invention, the at least 2, 3, 4, 5, 6, 7, 8, 9, 10, 11, 12, 13, 14, 15, 16, 17, 18, 19, 20, 21 ribonucleotides at the 5' end are on the same strand.

The first ribonucleotide at the 5' end of the oligonucleotide comprises an adenine.

In preferred embodiments, the sequence of the first 4 nucleotides at the 5' end of the oligonucleotide is selected from: AAGU, AAAG, AUGG, AUUA, AACG, AUGA, AGUU, AUUG, AACA, AGAA, AGCA, AACU, AUCG, AGGA, AUCA, AUGC, AGUA, AAGC, AACC, AGGU, AAAC, AUGU, ACUG, ACGA, ACAG, AAGG, ACAU, ACGC, AAAU, ACGG, AUUC, AGUG, ACAA, AUCC, AGUC, wherein all sequences are in the 5'->3' direction.

In more preferred embodiments, the sequence of the first 4 nucleotides at the 5' end of the oligonucleotide is selected from: AAGU, AAAG, AUGG, AUUA, AACG, AUGA, AGUU, AUUG, AACA, AGAA, AGCA, AACU, AUCG, AGGA, AUCA, AUGC, AGUA, AAGC, AACC, wherein all sequences are in the 5'->3' direction.

In most preferred embodiments, , the sequence of the first 4 nucleotides at the 5' end of the oligonucleotide is selected from: AAGU, AAAG, AUGG, AUUA, wherein all sequences are in the 5'->3' direction.

In even more preferred embodiments, the sequence of the first 4 nucleotides at the 5' end of the oligonucleotide is selected from: AAGU, AAAG, AUGG, AUUA, AACG, AUGA, AGUU, AUUG, AACA, wherein all sequences are in the 5'->3' direction.

The oligonucleotide of the invention may be a RNA oligonucleotide, or a chimeric RNA-DNA oligonucleotide. A chimeric RNA-DNA oligonucleotide comprises both ribonucleotides and deoxyribonucleotides. The ribonucleotides and the deoxyribonucleotides may be on the same strand, or may be on different strands.

In one embodiment, the oligonucleotide (RNA or chimeric RNA-DNA) comprises a phosphorothioate backbone. In preferred embodiments, at least 1, preferably at least 2, more preferably at least 3, even more preferably at least 4 nucleotides are phosphorothioate.

In a preferred embodiment, the oligonucleotide of the invention does not contain any of the modifications pseudouridine, 2-thiouridine, 2'-Fluorine-dNTP .

The present invention also provides precursors of the oligonucleotide of the invention.

As used herein, the "precursor of the oligonucleotide" of the invention refers to any molecule which can be processed to generate the oligonucleotide of the invention. The precursors of the oligonucleotide of the invention include, but are not limited to, DNA or RNA molecules which can serve as templates for the synthesis of the RNA oligonucleotides of the invention, RNA or RNA-DNA chimeric molecules which can be enzymatically cleaved to produce the oligonculeotides of the invention.

The oligonucleotide or precursor thereof of the invention may also contain motifs or molecular signatures which are recognized by TLRs. For example, long dsRNA (longer than 30 bases) bearing a 5' triphosphate can serve as a ligand for both RIG-I and TLR3. A chimeric RNA-DNA oligonucleotide comprising a ssRNA bearing a 5' triphosphate and a ssDNA containg CpG can serve as a ligand for both RIG-I and TLR9. ssRNA or dsRNA bearing a 5' triphosphate and defined sequence motifs (S. S. Diebold et al., Science 303, 1529 (Mar 5, 2004); F. Heil et al., Science 303, 1526 (Mar 5, 2004); V. Hornung et al., Nat Med 11, 263 (Mar, 2005); WO 03/086280; European patent application no. 05020020.3) can serve as a ligand for both RIG-I and TLR7. ssRNA bearing a 5' triphosphate and GU-rich motifs (WO 03/086280) can serve as a ligand for both RIG-I and TLR8.

Furthermore, the oligonucleotide of the invention may be a RNA oligonucleotide having gene silencing activity.

In one embodiment, the RNA olignucleotide is an antisense RNA oligonucleotide.

In another embodiment, the RNA olignucleotide is active in RNA interference (RNAi). The RNA oligonucleotide may be an siRNA (small interfering RNA), shRNA (small hairpin RNA) or miRNA (microRNA).

The oligonucleotide or the precursor thereof of the invention can be used to generate a large amount of type I IFN, in particular, IFN-α *in vitro* and/or *in vivo.* The type I IFN, in particular, IFN-α, can be generated at high quantities from different cellular sources, including both immune and non-immune cells, from different species of vertebrates.

The oligonucleotide or the precursor thereof of the invention can also be used to generate a large amount of IL-18 and/or IL-1β *in vitro* and/or *in vivo.* IL-18 and/or IL-1β can be generated at high quantities from different cellular sources, including both immune and non-immune cells, from different species of vertebrates.

The oligonucleotide of the invention may be prepared by synthetic methods including, but not limited to, chemical synthesis, *in vitro* transcription and *in vivo* transcription. In *in vitro* transcription, polymerases including, but not limited to, bacteriophage polymerase such as T7 polymerase, T3 polymerase, Sp6 polymerase, viral polymerases, and *E*. *coli* RNA polymerase may be used. *In vivo* transcription may be achieved in virally infected cells, or bacteria that are either non-infected or infected with a phage.

Furthermore, the oligonucleotides or precursor thereof, in particular, the RNA oligonucleotides, of the invention may be covalently or non-covalently linked to one or more lipophilic groups which enhance the stability and/or the activity and/or facilitate the delivery of the oligonucleotides or precursor thereof.

As used herein, the term "lipophilic" or "lipophilic group" broadly refers to any compound or chemical moiety having an affinity for lipids. Lipophilic groups encompass compounds of many different types, including those having aromatic, aliphatic or alicyclic characteristics, and combinations thereof.

In specific embodiments, the lipophilic group is an aliphatic, alicyclic, or polyalicyclic substance, such as a steroid (e.g., sterol) or a branched aliphatic hydrocarbon. The lipophilic group generally comprises a hydrocarbon chain, which may be cyclic or acyclic. The hydrocarbon chain may comprise various substituents and/or at least one heteroatom, such as an oxygen atom. Such lipophilic aliphatic moieties include, without limitation, saturated or unsatarated fatty acids, waxes (e.g., monohydric alcohol esters of fatty acids and fatty diamides), terpenes (e.g., the C₁₀ terpenes, C₁₅ sesquiterpenes, C₂₀ diterpenes, C₃₀ triterpenes, and C₄₀ tetraterpenes), and other polyalicyclic hydrocarbons.

The lipophilic group may be attached by any method known in the art, including via a functional grouping present in or introduced into the RNA oligonucleotide, such as a hydroxy group (e.g., -CO-CH₂ -OH). Conjugation of the RNA oligonucleotide and the lipophilic group may occur, for example, through formation of an ether or a carboxylic or carbamoyl ester linkage between the hydroxy and an alkyl group R-, an alkanoyl group RCO- or a substituted carbamoyl group KNHCO-. The alkyl group R may be cyclic (e.g., cyclohexyl) or acyclic (e.g., straight-chained or branched; and saturated or unsaturated). Alkyl group R may be a butyl, pentyl, hexyl, heptyl, octyl, nonyl, decyl, undecyl, dodecyl, tridecyl, tetradecyl, pentadecyl, hexadecyl, heptadecyl or octadecyl group, or the like. Preferably, the lipophilic group is conjugated to the 5'-hydroxyl group of the terminal nucleotide. In a preferred embodiment, the liphophilic group is 12-hydroxydodeconoic acid bisdecylamide.

In another embodiment, the lipophilic group is a steroid, such as sterol. Steroids are polycyclic compounds containing a perhydro-1,2-cyclopentanophenanthrene ring system. Steroids include, without limitation, bile acids (e.g., cholic acid, deoxycholic acid and dehydrocholic acid), cortisone, digoxigenin, testosterone, cholesterol and cationic steroids, such as cortisone.

In a preferred embodiment, the lipophilic group is cholesterol or a derivative thereof. A "cholesterol derivative" refers to a compound derived from cholesterol, for example by substitution, addition or removal of substituents. The steroid may be attached to the RNA oligonucleotide by any method known in the art. In a preferred embodiment, the liphophilic group is cholesteryl (6-hydroxyhexyl) carbamate.

In another embodiment, the lipophilic group is an aromatic moiety. In this context, the term "aromatic" refers broadly to mono- and polyaromatic hydrocarbons. Aromatic groups include, without limitation, C₆-C₁₄ aryl moieties comprising one to three aromatic rings, which may be optionally substituted; "aralkyl" or "arylalkyl" groups comprising an aryl group covalently linked to an alkyl group, either of which may independently be optionally substituted or unsubstituted; and "heteroaryl" groups. As used herein, the term "heteroaryl" refers to groups having 5 to 14 ring atoms, preferably 5, 6, 9, or 10 ring atoms; having 6, 10, or 14π electrons shared in a cyclic array; and having, in addition to carbon atoms, between one and about three heteroatoms selected from the group consisting of nitrogen (N), oxygen (O), and sulfur (S).

As used herein, a "substituted" alkyl, cycloalkyl, aryl, heteroaryl, or heterocyclic group is one having between one and about four, preferably between one and about three, more preferably one or two, non-hydrogen substituents. Suitable substituents include, without limitation, halo, hydroxy, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, and ureido groups.

The lipophilic group can be covalently linked directly or indirectly via a linker to the oligonucleotide or precursor thereof. The covalent linkage may or may not comprise a phosphodiester group. And the linker may be of various lengths. The preferred lengths of the linker are known to those skilled in the art and may be determined experimentally.

In one embodiment, the lipophilic group is covalently linked to the 3' end of at least one strand of the oligonucleotide or precursor thereof.

In addition, the oligonucleotide or precursor thereof of the invention may be coupled to a solid support. By "coupled" it is meant that the oligonucleotide or precursor thereof is covalently or non-covalently, directly or indirectly, linked to the solid support. Suitable solid supports include, but are not limited to, silicon wafers, synthetic polymer support such as polystyrene, polypropylene, polyglycidylmethacrylate, substituted polystyrene (e.g., aminated or carboxylated polystyrene, polyacrlamides, polyamides, polyvinylchlorides, etc.), glass, agarose, nitrocellulose, nylon and gelatin nanoparticles. Solid support may enhance the stability and the activity of the oligonucleotide, especially short oligonucleotides less than 16 nucleotides in length.

### Oligonucleotide Conjugates

The present invention also provides an oligonucleotide conjugate which is capable of inducing an anti-viral response, in particular, type I IFN production, comprising an oligonucleotide of the invention and an antigen conjugated to the oligonucleotide. In preferred embodiments, the antigen is conjugated to the oligonucleotide at a position other than its 5' end which carries the 5' triphosphate. In some embodiments, the antigen produces a vaccine effect.

The antigen is preferably selected from disease/disorder-related antigens. The disorder may be, for example, a cancer, an immune disorder, a metabolic disorder, or an infection. The antigen may be a protein, a polypeptide, a peptide, a carbohydrate, or a combination thereof.

The oligonucleotide of the invention may be covalently linked to the antigen, or it is otherwise operatively associated with the antigen. As used herein, the term "operatively associated with" refers to any association that maintains the activity of both the oligonucleotide and the antigen. Non-limiting examples of such operative associations include being part of the same liposome or other such delivery vehicle or reagent. In embodiments wherein the oligonucleotide agent is covalently linked to the antigen, such covalent linkage preferably is at any position on the oligonucleotide that does not interfere with the capability of the oligonucleotide to induce an anti-viral response.

### Pharmaceutical Composition

The present invention provides a pharmaceutical composition comprising one or more of the oligonucleotide(s) or a precursor thereof described above and a pharmaceutically acceptable carrier.

The present disclosure also provides a pharmaceutical composition comprising bacterial RNA and a pharmaceutically acceptable carrier.

As used herein, "bacterial RNA" refers to any RNA species isolated from a bacterium, including, but not limited to, total RNA, mRNA, ribosomal RNA, phage RNA, miRNA, structural RNA, and enzymatic RNA. Bacterial RNA may be endogenous to a bacterium, or may be derived from exogenous DNA that has been introduced into the bacterium. Bacterial RNA can be of any length. Bacterial RNA preparations may contain a single RNA species with a single nucleotide sequence, a single RNA species with more than one nucleotide sequences, or multiple RNA species with more than one nucleotide sequences. Bacterial RNA may comprise any type of nucleotides and bases known in the field, including naturally occurring nucleotides and nucleotides converted inside the cell, such as inosine triphosphate and inosine, any known modifications to the backbone and bases, and a monophosphate, a diphosphate, or a triphosphate group at the 5' end. Bacterial RNA may be single-stranded or double-stranded. Bacterial RNA may comprise a heteroduplex of RNA and DNA. Bacterial RNA may be composed of a mixture of RNAs isolated from different types of bacteria.

Preferably the bacterial RNA does not have a nucleotide sequence that is more than 80%, 85%, 90%, 95%, or 99% complementary or that is 100% to a eukaryotic gene coding sequence. In other words, the bacterial RNA preferably does not have any gene-silencing or RNA interference (RNAi) activity.

The term complementary is well understood by those skilled in the art. For example, A is complementary to T, G is complementary to C, 5'-AG-3' is complementary to 5'-CT-3'.

The degree of complementarity between two nucleotide sequences is the percentage of complementary bases in the overlapping region of the two nucleotide sequences. The degree of complementarily can be determined manually or automatically by various engines such as BLAST. For example, ATCG has 100% complementarity to CGAT and CGATGG, and 75% complementarity to CGTT and CGTTGG. Furthermore, the degree of complementarity between a RNA oligonucleotide or polynucleotide and any sequences present in the public databases (e.g., EMBL, GeneBank) can be determined by the BLAST program.

Preferably the pharmaceutical composition of the invention further comprises an agent which facilitates the delivery of the oligonucleotide or the precursor thereof or the bacterial RNA into a cell, in particular, into the cytosol of the cell.

The delivery agent may be a complexation agent which forms a complex with the oligonucleotide or the precursor thereof and facilitates the delivery of the oligonucleotide or precursor thereof into cells. In one form, the complexation agent is a polymer, preferably a cationic polymer. In a preferred form the complexation agent is a cationic lipid. In another preferred form the complexation agent is polyethylenimine (PEI) (K. Wu et al., Brain Research 1008(2):284-287 (May 22, 2004); B. Urban-Klein et al. Gene Therapy 12(5):461-466 (2005)). Additional examples of complexation agent include, but are not limited to, collagen derivatives (Y. Minakuchi et al. Nucleic Acids Research 32(13):e109 (2004)), and biodegradable microspheres such as liposomes (M. Sioud, D. Sorensen, Biochem Biophys Res Commun 312(4):1220-1225 (2003); PY Chien et al. Cancer Gene Therapy 12(3):321-328 (2005)), virosomes (J de Jonge et al. Gene Therapy, 13:400-411 (2006)), SNALPs (JJ Rossi, Gene Therapy 13:583-584 (2006)), SICOMATRIX^{®} (CSL Limited) (I.D. Davis et al. PNAS 101(29):10697-10702 (July 20, 2004); MJ Pearse, D. Drane, Adv Drug Deliv Rev 57(3):465-474 (Jan 10, 2005)), and poly (D,L-lactide-co-glycolide) copolymer (PLGA) microspheres (A. Khan et al. J Drug Target 12(6):393-404 (2004)).

Polyethylenimine (PEI) can be linear or branched. In a preferred embodiment, PEI is *in vivo*-jetPEI™ which is a linear PEI developed by PolyPlus-transfection for effective and reproducible delivery of anionic oligonucleotides with low toxicity *in vivo.* The preferred *in* vivo routes of administration include, but are not limited to, intravenous, intracerebral and intraperitoneal routes.

Virosomes are reconstituted viral envelopes which are prepared from membrane-enveloped viruses, in particular influenza virus, by solubilization of the viral membrane with a suitable detergent, removal of the nucleocapsids by ultracentrifugation and reconstituion of the viral envelope through extraction of the detergent. Typically, virosomes contain viral lipids and viral glycoproteins (such as hemagglutinin (HA) and neuraminidase (NA) in the case of influenza virosomes), resemble the native virus particles in size and morphology and retain the target specificity and the fusogenic activity of the native viral particles.

SNALPs stand for Stable-Nucleic-Acid-Lipid Particles and contain a lipid bilayer comprised of a mixture of cationic and fusogenic lipid coated with diffusible polyethylene glycol (PEG). The SNALPs are in the 120 nanometer diameter size range, protect the enclosed nucleic acid from serum nucleases and allow cellular endosomal uptake and subsequent cytoplasmic release of the nucleic acid.

ISCOMATRIX^{®} is made from saponin, cholesterol and phospholipids under defined conditions and forms cage like structures typically 40nm in diameter. ISCOMATRIX^{®} has the duel capability of facilitating cargo (e.g., antigen) delivery and stimulating the immune system, both the cellular and humoral immune response.

In another form the delivery agent is a virus, preferably a replication-deficient virus. In one embodiment, the oligonucleotide described in the invention is contained in a viral capsule. In another form the precursor of the oligonucleotide described in the invention is comprised in a viral vector which is contained in a viral capsule. In one embodiment, the viral particle contains an enzyme or a nucleic acid encoding the enzyme required for the processing of the precursor into the oligonucleotide described in the invention. In another form the virus comprising the precursor is administered in conjunction with the enzyme or the nucleic acid encoding the enzyme required for the processing of the precursor into the oligonucleotide described in the invention.

Suitable viruses include, but are not limited to, polymyxoviruses which target upper respiratory tract epithelia and other cells, hepatitis B virus which targets liver cells, influenza virus which targets epithelial cells and other cells, adenoviruses which targets a number of different cell types, papilloma viruses which targets epithelial and squamous cells, herpes virus which targets neurons, retroviruses such as HIV which targets CD4⁺ T cells and dendritic cells and other cells, and modified Vaccinia Ankara which targets a variety of cells. Viruses may be selected based on their target specificity.

In one form the virus is an oncolytic virus. Oncolytic viruses target tumor cells and cause the lysis of the infected tumor cells. Examples of oncolytic viruses include, but are not limited to, naturally occurring wild-type Newcastle disease virus (A. Phuangsab et al. Cancer Lett 172:27-36 (2001)), attenuated strains of reovirus (MC Coffey et al. Science 282:1332-1334 (1998)) and vesicular stomatitis virus (VSV) (DF Stojdl et al. Nat Med 6:821-825 (2000)), genetically engineered mutants of herpes simplex virus type 1 (HSV-1), adenovirus, poxvirus and measles virus (Chiocca EA Nat Rev Cancer 2:938-950 (2002); Russell SJ Cancer Gene Ther 9:961-966 (2002); HJ Zeh, DL Bartlett Cancer Gene Ther 9:1001-1012 (2002)).

In addition to being delivered by a delivery agent, the oligonucleotide or precursor thereof described in the invention or bacterial RNA can be delivered into cells via physical means such as electroporation, shock wave administration (Tschoep K et al., J Mol Med 2001; 79:306-13), ultrasound triggered transfection, and gene gun delivery with gold particles.

The pharmaceutical composition may further comprises another agent such as an agent that stabilizes the oligonucleotide or precursor thereof or bacterial RNA, in particular, RNA oligonucleotide, *e.g*., a protein that complexes with the oligonucleotide agent to form an iRNP. Still other agents include chelators, *e.g*., EDTA (*e.g*., to remove divalent cations such as Mg²⁺), salts, RNAse inhibitors (*e.g*., a broad specificity RNAse inhibitor such as RNAsin) and so forth.

A formulated composition can assume a variety of states. In some examples, the composition is at least partially crystalline, uniformly crystalline, and/or anhydrous (*e.g*., less than 80, 50, 30, 20, or 10% water). In another example, the oligonucleotide agent is in an aqueous phase, *e.g*., in a solution that includes water, this form being the preferred form for administration via inhalation.

The aqueous phase or the crystalline compositions can be incorporated into a delivery vehicle, *e.g*., a liposome (particularly for the aqueous phase), or a particle (e.g., a microparticle as can be appropriate for a crystalline composition). Generally, the oligonucleotide composition is formulated in a manner that is compatible with the intended method of administration.

The pharmaceutical compositions encompassed by the invention may be administered by any means known in the art including, but not limited to, oral or parenteral routes, including intravenous, intramuscular, intraperitoneal, subcutaneous, transdermal, airway (aerosol), ocular, rectal, vaginal, and topical (including buccal and sublingual) administration. In preferred embodiments, the pharmaceutical compositions are administered by intravenous or intraparenteral infusion or injection. The pharmaceutical compositions can also be administered intraparenchymally, intrathecally, and/or by stereotactic injection.

For oral administration, the oligonucleotide or the precursor thereof described in the invention or bacterial RNA will generally be provided in the form of tablets or capsules, as a powder or granules, or as an aqueous solution or suspension.

Tablets for oral use may include the active ingredients mixed with pharmaceutically accept-able excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatin capsules in which the active ingredient is mixed with a solid diluent, and soft gelatin capsules wherein the active ingredient is mixed with water or an oil such as peanut oil, liquid paraffin or olive oil.

For intramuscular, intraperitoneal, subcutaneous and intravenous use, the pharmaceutical compositions of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

The pharmaceutical compositions can also include encapsulated formulations to protect the oligonucleotide or precursor thereof or bacterial RNA against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811; PCT publication WO 91/06309; and European patent publication EP-A-43075.

In general, a suitable dose of an oligonucleotide or precursor thereof or bacterial RNA will be in the range of 0.001 to 500 milligrams per kilogram body weight of the recipient per day (e.g., about 1 microgram per kilogram to about 500 milligrams per kilogram, about 100 micrograms per kilogram to about 100 milligrams per kilogram, about 1 milligrams per kilogram to about 75 milligrams per kilogram, about 10 micrograms per kilogram to about 50 milligrams per kilogram, or about 1 microgram per kilogram to about 50 micrograms per kilogram). The pharmaceutical composition may be administered once per day, or the oligonucleotide or precursor thereof or bacterial RNA may be administered as two, three, four, five, six or more sub-doses at appropriate intervals throughout the day. In that case, the oligonucleotide or precursor thereof or bacterial RNA contained in each sub-dose must be correspondingly smaller in order to achieve the total daily dosage. The dosage unit can also be compounded for delivery over several days, e.g., using a conventional sustained release formulation which provides sustained release of the oligonucleotide agent or bacterial RNA over a several day period. Sustained release formulations are well known in the art. In this embodiment, the dosage unit contains a corresponding multiple of the daily dose.

The skilled artisan will appreciate that certain factors may influence the dosage and timing required to effectively treat a subject, including but not limited to the severity of the infection or disease/disorder, previous treatments, the general health and/or age of the subject, and other diseases/disorders present. Moreover, treatment of a subject with a therapeutically effective amount of a composition can include a single treatment or a series of treatments. Estimates of effective dosages and *in vivo* half-lives for the individual oligonucleotide or precursor thereof described in the invention or bacterial RNA can be made using conventional methodologies or on the basis of *in vivo* testing using an appropriate animal model.

Toxicity and therapeutic efficacy of the oligonucleotide or precursor thereof or bacterial RNA and the pharmaceutical composition of the invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Oligonucleotide agents or bacterial RNA that exhibit high therapeutic indices are preferred.

The data obtained from cell culture assays and animal studies can be used in formulating a range of dosage for use in humans. The dosages of compositions of the invention are preferably within a range of circulating concentrations that include the ED50 with little or no toxicity. The dosage may vary within this range depending upon the dosage form employed and the route of administration utilized. For any oligonucleotide agent or bacterial RNA used in the method of the invention, the therapeutically effective dose can be estimated initially from cell culture assays. A dose may be formulated in animal models to achieve a circulating plasma concentration range of the oligonucleotide agent or bacterial RNA that includes the IC50 (i.e., the concentration of the test oligonucleotide agent which achieves a half-maximal inhibition of symptoms) as determined in cell culture. Such information can be used to more accurately determine useful doses in humans. Levels in plasma may be measured, for example, by high performance liquid chromatography.

The administering physician can adjust the amount and timing of the administration of the pharmaceutical composition of the invention on the basis of results observed using standard measures of efficacy known in the art or described herein.

The pharmaceutical composition of the invention can be used to generate a large amount of type I IFN, in particular, IFN-α, IL-18 and/or IL-1β, *in vitro* and/or *in vivo.* The type I IFN, in particular, IFN-α, IL-18 and/or IL-1β, can be generated at high quantities from different cellular sources, including both immune and non-immune cells, from different species of vertebrates.

### Combined Preparation

The present disclosure provides a combined preparation comprising an oligonucleotide or a precursor thereof described in the invention or a bacterial RNA and a pharmaceutially active agent, wherein the oligonucleotide or a precursor thereof or the bacterial RNA and the agent are for simultaneous, separate or sequential administration.

The pharmaceutically active agents include, but are not limited to, immunostimulatory RNA oligonucleotides, immunostimulatory DNA oligonucleotides, cytokines, chemokines, growth factors, antibiotics, anti-angiogenic factors, chemotherapeutic agents, anti-viral agents, anti-bacterial agents, anti-fungal agents, anti-parasitic agents, and antibodies.

The present disclosure provides a combined preparation comprising an oligonucleotide or a precursor thereof described in the invention or a bacterial RNA and an immunotstimulatory agent, wherein the oligonucleotide or a precursor thereof or the bacterial RNA and the agent are for simultaneous, separate or sequential administration. The combined preparation may further comprise an anti-viral and/or anti-tumor agent.

The present disclosure further provides a combined preparation comprising an oligonucleotide or a precursor thereof described in the invention or a bacterial RNA and an anti-viral and/or anti-bacterial and/or anti-tumor agent, wherein the oligonucleotide or a precursor thereof or the bacterial RNA and the agent are for simultaneous, separate or sequential administration. The combined preparation may further comprise an immunostimulatory agent.

The oligonucleotide or a precursor thereof described in the invention or the bacterial RNA and the immunostimulatory agent, or the anti-viral agent, or the anti-bacterial agent, or the anti-tumor agent may be comprised in the same or in separate compositions. The separate compositions may be administered simultaneously or sequentially.

The combined preparation may further comprise retinoic acid and/or type I IFN. Retinoic acid and/or type I IFN are known to upregulate RIG-I expression in most cell types, including for example endothelial cells, epithelial cells, fibroblasts, immune cells and tumor cells.

An immunostimulatory agent is an agent, such as a molecule or a composition, which is capable of inducing an immune response. Immunostimulatory agents include, but are not limited to, immunostimulatory RNA oligonucleotides such as those capable of inducing IFN-a or IL-12 (Heil F et al. 2004, Science 303: 1526-1529; Sioud M et al. 2005, J Mol Biol 348: 1079-1090; Homung V et al. 2005, Nat Med 11: 263-270; Judge AD et al. 2005, Nat Biotechnol 2005. 23: 457-462; Sugiyama et al. 2005, J Immunol 174:2273-2279; Gitlin L et al. 2006, PNAS 103(22):8459-8464; European patent application nos. 05020020.3 and 05020019.5) (e.g., poly(I:C) and immunostimulatory DNA oligonucleotides such as a CpG-containing or non-CpG-containing DNA oligonucleotide capable of inducing IFN-α (see e.g., WO 01/22990, WO 03/101375), cytokines such as type I IFN and IL-12, chemokines such as IP-10, MIP1-α, MCP, RANTES, IL-8, and growth factors such as IL-3, GMCSF, GSCF, MCSF.

In one embodiment, the immunostimulatory agent is capable of inducing an anti-viral response, such as type I IFN, MIP1-a, MCP, RANTES, IL-8, and IL-6 production.

An anti-viral agent is an agent that is useful in the prevention and the treatment of a viral infection. Anti-viral agents include, but are not limited to nucleoside analogs (such as aciclovir, ganciclovir, ribavirin, lamivudin, etc.), protease inhibitors (such as ritonavir etc), cytotoxic agents (such as taxols, carboplatins, cyclophosphamide, methotrexat, azathiprin, 5-fluoruracil, etc.)

In another embodiment, the immunostimulatory agent is capable of inducing an anti-bacterial response, such as type I and/or type II IFN production.

An anti-bacterial agent is an agent that is useful in the prevention and the treatment of a bacterial infection, in particular, intracellular bacterial infection. Anti-bacterial agents include, but are not limited to, Aminoglycosides, Carbapenems, Cephalosporins, Glycopeptides, Macrolides, Monobactam, Penicillins, Polypeptides, Quinolones, Sulfonamides, Tetracyclines.

An anti-tumor agent is an agent that is useful in the prevention and the treatment of tumor or cancer. Anti-tumor agents include, but are not limited to chemotherapeutic agents (such as cisplatin, doxorubicin, taxols, carboplatins, cyclophosphamide, methotrexat, azathiprin, 5-fluoruracil, etc.), anti-angiogenic factors (such as vasostatin and anti-VEGF antibody), and other anti-cancer agents such as Herceptin^{®}, Rituxan^{®}, Gleevec^{®}, and Iressa^{®}.

In a preferred embodiment, the combined preparation of the invention further comprises an oligonucleotide delivery agent as described previously. In other preferred embodiments, the oligonucleotide or precursor thereof or the bacterial RNA may be delivered by physical means as described previously.

### Pharmaceutical Package

The present invention provides a pharmaceutical package comprising the pharmaceutical composition or the combined preparation of the invention and an instruction for use.

### Use of the Oligonucleotide or Precursor thereof or bacterial RNA for Inducing an Anti-viral Response

The present application provides the use of the oligonucleotide or precursor thereof described in the invention or a bacterial RNA for the preparation of a pharmaceutical composition for inducing an anti-viral response, in particular, type I IFN production, IL-18 production, and/or IL-1β production, in a vertebrate animal, in particular, a mammal.

An anti-viral response is the response by a cell, tissue or organism upon infection by a virus with the purpose of eliminating or incapacitating the virus. Typical anti-viral responses include, but are not limited to, type I IFN, MIP1-a, MCP, RANTES, IL-8, IL-6, IP-10, and IFN-γ production.

An anti-viral response, in particular, type I IFN, IL-18, and/or IL-1β production, may be induced in immune cells or non-immune cells. Immune cells include, but are not limited to, peripheral blood mononuclear cells (PBMC), plasmacytoid dendritric cells (PDC), myeloid dendritic cells (MDC), B cells, CD4+ T cells, CD8+ T cells, macrophages, monocytes, natural killer cells, NKT cells, granulocytes. Non-immune cells include, but are not limited to, fibroblasts, endothelial cells, epithelial cells and tumor cells.

The induction of an anti-viral response, in particular, type I IFN, IL-18, and/or IL-1β production, may aid the prevention and treatment of various disorders and/or diseases such as tumor, infections, and immune disorders.

In one embodiment, the pharmaceutical composition further comprises a delivery agent as described previously. The oligonucleotide or precursor thereof or bacterial RNA may also be delivered by physical means as described previously. In another embodiment, the pharmaceutical composition further comprises another agent such as an agent that stabilizes the oligonucleotide or precursor thereof or bacterial RNA as described previously.

In one embodiment, the oligonucleotide or precursor thereof described in the invention or the bacterial RNA is used in combination with an immunostimulatory agent which is capable of inducing an anti-viral response. In another embodiment, the oligonucleotide or precursor thereof described in the invention or the bacterial RNA is used in combination with an anti-viral agent. In yet another embodiment, the oligonucleotide or precursor thereof described in the invention or the bacterial RNA is used in combination with an immunostimulatory agent which is capable of inducing an anti-viral response and an anti-viral agent. In a further embodiment, the oligonucleotide or precursor thereof described in the invention or the bacterial RNA is used in combination with retinoic acid and/or type I IFN.

Vertebrate animals include, but are not limited to, fish, amphibians, birds, and mammals.

Mammals include, but are not limited to, rats, mice, cats, dogs, horses, sheep, cattle, cows, pigs, rabbits, non-human primates, and humans. In a preferred embodiment, the mammal is human.

### Use of the Oligonucleotide or Precursor thereof or bacterial RNA for Inducing an Anti-bacterial Response

The present application provides the use of the oligonucleotide or precursor thereof described in the invention or a bacterial RNA for the preparation of a pharmaceutical composition for inducing an anti-bacterial response, in particular, a response against intracellular bacteria, in a vertebrate animal, in particular, a mammal.

Intracellular bacteria include, but are not limited to, mycobacteria (tuberculosis), chlamydia, mycoplasma, listeria, and facultative intracellular bacteria such as staphylococcus aureus.

An anti-bacterial response is the response by a cell, tissue or organism upon infection by a bacterium with the purpose of eliminating or incapacitating the bacterium. Typical anti-bacterial responses include, but are not limited to, T cell or NK cell-mediated elimination of the infected cell by either receptor-mediated apoptosis or cytokine-mediated apoptosis via TNF or TRAIL, macorphage or monocytes phagocytosis.

In one form, the anti-bacterial response comprises type I IFN, type II IFN, IL-18 and/or IL-1β production.

An anti-bacterial response, in particular, type I IFN, type II IFN, IL-18, and/or IL-1β production, may be induced in immune cells or non-immune cells. Immune cells include, but are not limited to, peripheral blood mononuclear cells (PBMC), plasmacytoid dendritric cells (PDC), myeloid dendritic cells (MDC), B cells, macrophages, monocytes, natural killer cells, NKT cells, CD4+ T cells, CD8+ T cells, granulocytes. Non-immune cells include, among others, tumor cells, epithelial cells, endothelial cells, and fibroblasts.

The induction of an anti-bacterial response, in particular, type I IFN, type II IFN, IL-18 and/or IL-1β production, may aid the prevention and treatment of various disorders and/or diseases such as tumor, infections, and immune disorders.

In one form, the pharmaceutical composition further comprises a delivery agent as described previously. The oligonucleotide or precursor thereof or bacterial RNA may also be delivered by physical means as described previously. In another form the pharmaceutical composition further comprises another agent such as an agent that stabilizes the oligonucleotide or precursor thereof or bacterial RNA as described previously.

In one form, the oligonucleotide or precursor thereof described in the invention or the bacterial RNA is used in combination with an immunostimulatory agent which is capable of inducing an anti-bacterial response. In another form the oligonucleotide or precursor thereof described in the invention or the bacterial RNA is used in combination with an anti-bacterial agent. In yet another form the oligonucleotide or precursor thereof described in the invention or the bacterial RNA is used in combination with an immunostimulatory agent which is capable of inducing an anti-bacterial response and an anti-bacterial agent. In a further form the oligonucleotide or precursor thereof described in the invention or the bacterial RNA is used in combination with retinoic acid and/or type I IFN.

Vertebrate animals include, but are not limited to, fish, amphibians, birds, and mammals.

Mammals include, but are not limited to, rats, mice, cats, dogs, horses, sheep, cattle, cows, pigs, rabbits, non-human primates, and humans. In a preferred embodiment, the mammal is human.

### Use of the Oligonucleotide or Precursor Thereof or bacterial RNA for Treating Diseases/Disorders

The present invention provides the use of the oligonucleotide or precursor thereof described in the invention or a bacterial RNA for the preparation of a pharmaceutical composition for preventing and/or treating a disease and/or disorder in a vertebrate animal, in particular, a mammal, in medical and/or veterinary practice.

The disease and/or disorder include, but are not limited to infections, tumor, allergy, multiple sclerosis, and immune disorders.

Infections include, but are not limited to, viral infections, bacterial infections, anthrax, parasitic infections, fungal infections and prion infection.

Viral infections include, but are not limited to, infection by hepatitis C, hepatitis B, herpes simplex virus (HSV), HIV-AIDS, poliovirus, encephalomyocarditis virus (EMCV) and small-pox virus. Examples of (+) strand RNA viruses which can be targeted for inhibition include, without limitation, picornaviruses, caliciviruses, nodaviruses, coronaviruses, arteriviruses, flaviviruses, and togaviruses. Examples of picomaviruses include enterovirus (poliovirus 1), rhinovirus (human rhinovirus 1A), hepatovirus (hepatitis A virus), cardiovirus (encephalomyocarditis virus), aphthovirus (foot-and-mouth disease virus O), and parechovirus (human echovirus 22). Examples of caliciviruses include vesiculovirus (swine vesicular exanthema virus), lagovirus (rabbit hemorrhagic disease virus), "Norwalk-like viruses" (Norwalk virus), "Sapporo-like viruses" (Sapporo virus), and "hepatitis E-like viruses" (hepatitis E virus). Betanodavirus (striped jack nervous necrosis virus) is the representative nodavirus. Coronaviruses include coronavirus (avian infections bronchitis virus) and torovirus (Beme virus). Arterivirus (equine arteritis virus) is the representative arteriviridus. Togavirises include alphavirus (Sindbis virus) and rubivirus (Rubella virus). Finally, the flaviviruses include flavivirus (Yellow fever virus), pestivirus (bovine diarrhea virus), and hepacivirus (hepatitis C virus).

In certain forms the viral infections are selected from chronic hepatitis B, chronic hepatitis C, HIV infection, RSV infection, HSV infection, VSV infection, CMV infection, and influenza infection.

In one form the infection to be prevented and/or treated is upper respiratory tract infections caused by viruses and/or bacteria. In another form the infection to be prevented and/or treated is bird flu.

Bacterial infections include, but are not limited to, streptococci, staphylococci, E. coli, pseudomonas.

In one form bacterial infection is intracellular bacterial infection. Intracellular bacterial infection refers to infection by intracellular bacteria such as mycobacteria (tuberculosis), chlamydia, mycoplasma, listeria, and facultative intracellular bacteria such as staphylococcus aureus.

Tumors include both benign and malignant tumors (i.e., cancer).

Cancers include, but are not limited to biliary tract cancer, brain cancer, breast cancer, cervical cancer, choriocarcinoma, colon cancer, endometrial cancer, esophageal cancer, gastric cancer, intraepithelial neoplasm, leukemia, lymphoma, liver cancer, lung cancer, melanoma, myelomas, neuroblastoma, oral cancer, ovarian cancer, pancreatic cancer, prostate cancer, rectal cancer, sarcoma, skin cancer, testicular cancer, thyroid cancer and renal cancer.

In certain forms cancers are selected from hairy cell leukemia, chronic myelogenous leukemia, cutaneous T-cell leukemia, chronic myeloid leukemia, non-Hodgkin's lymphoma, multiple myeloma, follicular lymphoma, malignant melanoma, squamous cell carcinoma, renal cell carcinoma, prostate carcinoma, bladder cell carcinoma, breast carcinoma, ovarian carcinoma, non-small cell lung cancer, small cell lung cancer, hepatocellular carcinoma, basaliom, colon carcinoma, cervical dysplasia, and Kaposi's sarcoma (AIDS-related and non-AIDS related).

Allergies include, but are not limited to, respiratory allergies, contact allergies and food allergies.

Immune disorders include, but are not limited to, autoimmune diseases, immunodeficiency, and immunosuppression.

Autoimmune diseases include, but are not limited to, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, automimmune thyroiditis, dermatitis (including atopic dermatitis and eczematous dermatitis), psoriasis, Sjogren's Syndrome, Crohn's disease, aphthous ulcer, iritis, conjunctivitis, keratocon-junctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing, loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis.

Immunodeficiencies include, but are not limited to, spontaneous immunodeficiency, acquired immunodeficiency (including AIDS), drug-induced immunodeficiency (such as that induced by immunosuppressants used in transplantation and chemotherapeutic agents used for treating cancer), immunosuppression caused by chronic hemodialysis, trauma or surgical procedures.

Immunosuppression includes, but is not limited to, bone marrow suppression by cytotoxic chemotherapy.

The pharmaceutical composition may be a tumor vaccine. The oligonucleotide or precursor thereof described in the invention or the bacterial RNA may induce tumor cell apoptosis through binding to RIG-I, induce type I IFN, IL-18 and/or IL-1β production by the tumor cells, directly and/or indirectly activate effector cells of innate immunity such as NK cells, NKT cells, and γδ T cells, and/or directly and/or indirectly inactivate suppressor T cells, thereby leading to tumor cell growth inhibition and/or destruction.

Tumor cells which have been stimulated with an RIG-I ligand, such as the oligonucleotide or precursor thereof described in the present invention or a bacterial RNA, may also be used as a tumor vaccine.

The oligonucleotide or precursor thereof described in the invention or the bacterial RNA may be used in combination with one or more pharmaceutically active agents such as immunostimulatory agents, anti-viral agents, antibiotics, anti-fungal agents, anti-parasitic agents, anti-tumor agents, cytokines, chemokines, growth factors, anti-angiogenic factors, chemotherapeutic agents, and antibodies.

The oligonucleotide or precursor thereof described in the invention or the bacterial RNA may be used in combination with an anti-viral vaccine or an anti-bacterial vaccine or an anti-tumor vaccine, wherein the vaccine can be prophylactic and/or therapeutic.

The pharmaceutical composition may be for use in combination with one or more prophylactic or therapeutic treatments of diseases and/or disorders such as infection, tumor, multiple sclerosis, and immunodeficiency. For example, treatments of cancer include, but are not limited to, surgery, chemotherapy, radiation therapy, neoadjuvant therapy, thermoablation, and cryoablation.

The oligonucleotide or precursor thereof described in the present invention or a bacterial RNA may be used in combination with retinoic acid and/or type I IFN. Retinoic acid and/or type I IFN are known to upregulate RIG-I expression in most cell types, including for example endothelial cells, epithelial cells, fibroblasts, immune cells and tumor cells.

The pharmaceutical composition may further comprise a delivery agent as described previously. The oligonucleotide or precursor thereof or bacterial RNA may also be delivered by physical means as described previously. The pharmaceutical composition may further comprise another agent such as an agent that stabilizes the oligonucleotide or precursor thereof or bacterial RNA as described previously.

The pharmaceutical composition may be formulated for oral, nasal, ocular, parenteral (including intraveneous, intradermal, intramuscular, intraperitoneal, and subcutaneous), rectal, vaginal or topical (including buccal and sublingual) administration.

Preferably, the pharmaceutical composition is for prophylactic local (e.g., mucosa, skin) or systemic use. For example, a spray (i.e., aerosol) preparation may be used to strengthen the antiviral capability of the nasal and the pulmonary mucosa.

Vertebrate animals include, but are not limited to, fish, amphibians, birds, and mammals.

Mammals include, but are not limited to, rats, mice, cats, dogs, horses, sheep, cattle, cows, pigs, rabbits, non-human primates, and humans. In a preferred embodiment, the mammal is human.

### Use of the Oligonucleotide or Precursor Thereof or bacterial RNA as an Adjuvant

The prevent invention provides the use of the oligonucleotide or precursor thereof described in the invention or a bacterial RNA in combination with at least one antigen for the preparation of a vaccine for inducing an immune response against the at least one antigen in a vertebrate animal, in particular, a mammal.

The at least one antigen may be a protein, a polypeptide, a peptide, a carbohydrate, a nucleic acid, or a combination thereof.

The at least one antigen is preferably a disease/disorder-associated antigen, against which the generation of an immune response is beneficial for the prevention and/or treatment of the disease/disorder.

The oligonucleotide or precursor thereof or the bacterial RNA may be covalently linked to or non-covalently complexed with the at least one antigen. In one form the oligonucleotide or precursor thereof or the bacterial RNA is covalently linked to the at least one antigen. In another form both the oligonucleotide or precursor thereof or the bacterial RNA which is anionic and the protein or peptide antigen which is rendered anionic by N- or C-terminal extension of glutamic acid residues are complexed with cationic polymers. In yet another embodiment, phosphothioates which are incorporated into the oligonucleotide or precursor thereof or the bacterial RNA to increase nuclease resistance complexes with cysteine residues added to the N-terminal of antigenic protein or peptide. In a further form the at least one antigen can be encoded by a vector, in particular, a viral vector, which also comprises the oligonucleotide or precursor thereof. In yet a further form the at least one antigen can be a part of a virosome which encapsulates the oligonucleotide or precursor thereof or the bacterial RNA.

The oligonucleotide or precursor thereof or the bacterial RNA and the at least one antigen may also be comprised in separate compositions which are administered simultaneously.

The vaccine may further comprise a delivery agent as described previously. The oligonucleotide or precursor thereof or the bacterial RNA may also be delivered by physical means as described previously. The pharmaceutical composition may further comprise another agent such as an agent that stabilizes the oligonucleotide or precursor thereof or the bacterial RNA as described previously.

Vertebrate animals include, but are not limited to, fish, amphibians, birds, and mammals.

Mammals include, but are not limited to, rats, mice, cats, dogs, horses, sheep, cattle, cows, pigs, rabbits, non-human primates, and humans. In a preferred embodiment, the mammal is human.

### In Vitro Method for Stimulating an Anti-viral or Anti-bacterial Response

The present invention provides an *in vitro* method for stimulating an anti-viral response in a cell, comprising the steps of:
(a) mixing an oligonucleotide or precursor described in the invention or a bacterial R NA with a complexation agent; and
(b) contacting a cell with the mixture of (a), wherein the cell expresses RIG-I and/or NALP-3.

The present invention also provides an *in vitro* method for stimulating an anti-bacterial response in a cell, comprising the steps of:
(a) mixing an oligonucleotide or precursor described in the invention or a bacterial RNA with a complexation agent; and
(b) contacting a cell with the mixture of (a), wherein the cell expresses RIG-I and/or NALP-3.

Prerferably the anti-viral, response or the anti-bacterial response comprises type I IFN, in particular, IFN-α production, type II IFN production, IL-18 production, and/or IL-1β production.

The cells include, but are not limited to, primary immune cells, primary non-immune cells, and cell lines. Immune cells include, but are not limited to, peripheral blood mononuclear cells (PBMC), plasmacytoid dendritric cells (PDC), myeloid dendritic cells (MDC), B cells, macrophages, monocytes, natural killer cells, granulocytes. CD4+ T cells, CD8+ T cells, NKT cells. Non-immune cells include, but are not limited to, fibroblasts, endothelial cells, and epithelial cells. Cell lines include those that endogenously express RIG-I and/or NAPL-3 and those containing exogenous DNA which directs the expression of RIG-I and/or NALP-3.

### In Vitro Method for Stimulating Th1 Cytokine Production

The present invention provides an *in vitro* method for stimulating the production of a Th1 cytokine in a cell, comprising the steps of:
(a) mixing an oligonucleotide or precursor described in the invention or a bacterial RNA with a complexation agent; and
(b) contacting a cell with the mixture of (a), wherein the cell is capable of producing the Th1 cytokines.

The cell may expresse RIG-I and/or NALP-3.

Preferably, the Th1 cytokine is IL-18 or IL-1β.

The cells include, but are not limited to, immune cells and non-immune cells. Immune cells include, but are not limited to, peripheral blood mononuclear cells (PBMC), plasmacytoid dendritric cells (PDC), myeloid dendritic cells (MDC), B cells, macrophages, monocytes, natural killer cells, granulocytes, CD4+ T cells, CD8+ T cells, NKT cells. In a preferred embodiment, the cell is a macrophage. Non-immune cells include, but are not limited to fibroblasts, endothelial cells, and epithelial cells.

### Method for Preparing RNA for Gene Therapy

The present invention provides a method for preparing RNA for gene therapy, comprising the step of eliminating 5' triphosphate from RNA and/or incorporating modified nucleotides such as pseudouridine, 2-thiouridine, 2'-Fluorine-dNTPs - 2'-O-methylated NTPs, preferably 2'-fluorine-dCTP, 2'-fluorine-dUTP, 2'-O-methylated CTP, 2'-O-methylated UTP, into RNA. The RNA prepared according to the method of the invention lacks immunostimulatory activity and/or capability of inducing an anti-viral response and is therefore suitable for gene transfer in vertebrate cells.

RNA useful in gene therapy include those that upregulate or downregulate the expression/translation of a gene of interest. In the former case, the RNA encodes a protein of interest, the expression of which is of therapeutic value (e.g., a tumor suppressor; the cystic fibrosis protein). In the latter case, the RNA interferes with the expression of a protein of interest, the downregulation of which is of therapeutic value (e.g., an oncogene). In the latter case, the RNA may be an antisense RNA, an siRNA, an shRNA or a miRNA.

The utility of the oligonucleotide or precursor thereof described in the present invention or the bacterial RNA may be extended to other RIG-I and/or NALP-3 ligands.

The present invention is illustrated by the following examples.

### Examples

### Material and Methods

### Cell culture

Human PBMC were prepared from whole blood donated by young healthy donors by Ficoll-Hypaque density gradient centrifugation (Biochrom, Berlin, Germany). PDC were isolated by MACS using the blood dendritic cell Ag (BCDA)-4 dendritic cell isolation kit from Miltenyi Biotec (Bergisch-Gladbach, Germany). Briefly, PDC were labelled with anti-BDCA-4 Ab coupled to colloidal paramagnetic microbeads and passed through a magnetic separation column twice (LS column, then MS column; Miltenyi Biotec). The purity of isolated PDC (lineage-negative, MHC-II-positive and CD123-positive cells) was above 95 %. Before isolation of monocytes, PDC were depleted by MACS (LD column; Miltenyi Biotec) and then monocytes were isolated using the monocyte isolation kit II (Miltenyi Biotec). Murine bone marrow-derived conventional dendritic cells were generated by incubating pooled bone marrow cells in the presence of murine GM-CSF (10 ng/ ml; R&D Systems, Minneapolis, MN). After 7 days, these cultures typically contained more than 90 % cDC (CD11c+, CD11b+, B220-). Viability was above 95 %, as determined by trypan blue exclusion. All cells, except PDC (2.5*10⁶ cells / ml), were cultured at a density of 2*10⁶ cells / ml in RPMI 1640 culture medium (Biochrom, Berlin, Germany) supplemented with 10 % (v/v) FCS (Biochrom), 1.5 mM L-glutamine, 100 U/ ml penicillin, and 100 µg/ ml streptomycin (all from Sigma-Aldrich, Munich, Germany). PDC cultures were additionally supplemented with 10 ng/ ml IL-3 (R&D Systems). HEK 293 cells (human embryonic kindey) were maintained in RPMI 1640 culture medium (Biochrom) supplemented with 10 % (v/v) FCS (Biochrom), 1.5 mM L-glutamine, 100 U/ ml penicillin, and 100 µg/ ml streptomycin (all from Sigma-Aldrich). Vero (African green monkey kidney) and HEK 293T (human embryonic kidney) cells were maintained in Dulbecco's modified Eagle's medium supplemented with antibiotics and 5% or 10% foetal calf serum, respectively. BSR cells were propagated in Glasgow minimal essential medium supplemented with 10% newborn calf serum, phosphate broth, amino acids and antibiotics.

### Mice

TLR7, RIG-I and MDA5 deficient mice have been previously described (Hemmi H et al. Nat. Immunol. 3:196, Feb, 2002; Kato H et al., immunity 23:19, Jul, 2005; Kato H et al. Nature 441(7089):101-105, Apr 9, 2006). Female wildtype C57BU6 mice were purchased from Harlan-Winkelmann (Borchen, Germany). Mice were 6-12 weeks of age at the onset of experiments. Animal studies were approved by the local regulatory agency (Regierung von Oberbayem, Munich, Germany).

### ELISA

Human IFN-α was assessed in cell culture supernatants using the IFN-α module set (Bender MedSystems, Graz, Austria). The murine IP-10 ELISA was from Biosource (Solingen, Germany), the murine IFN-□ ELISA was from PBL Biomedical Laboratories (Piscataway, USA). All ELISA procedures were performed, according to manufacturers' recommendations. Murine IFN-α was measured according to the following protocol: monoclonal rat anti-mouse IFN-α (clone RMMA-1) was used as the capture Ab, and polyclonal rabbit anti-mouse IFN-α serum for detection (both PBL Biomedical Laboratories) together with HRP-conjugated donkey anti-rabbit IgG as the secondary reagent (Jackson ImmunoResearch Laboratories). Mouse rIFN-A (PBL Biomedical Laboratories) was used as the standard (IFN-α concentration in IU/ ml).

### RNAs

Chemically synthesized RNA oligonucleotides were purchased from Eurogentec (Leiden, Belgium). In vitro transcribed RNAs were synthesized using the Silencer siRNA construction Kit (Ambion, Huntingdon, UK) or according to the following protocol: Using partially overlapping single stranded DNA oligonucleotides, a double-stranded DNA template was constructed using Exo⁻ Klenow (Fermentas). The 2500 nucleotides transcript (Fig. 1) was generated using the control template of the Opti mRNA Kit (Curevac, Tübingen, Germany). Templates larger than 40 bp were constructed via PCR using the pBluescript KS as a template (for a detailed list of all in vitro transcription templates see table 1). The obtained templates contained a T7 RNA polymerase consensus promoter followed by the sequence of interest to be transcribed. 20 pmol of the DNA template were incubated with 30 U T7 RNA polymerase, 40 U RNase inhibitor, 0.3 U yeast inorganic pyrophosphatase in a buffer containing 40 mM Tris-HCl pH 8.0, 10 mM DTT, 2 mM spermidine-HCl (Sigma) and 20 mM MgCl₂. Capped RNA was transcribed using the Opti mRNA Kit (Curevac). To transcribe nucleoside modified RNAs, uridine-5'-triphosphate was replaced by either pseudouridine-5'-triphosphate or 2-thiouridine-5'-triphosphate (both TriLink, San Diego, USA) during the in vitro transcription reaction. For the incorporation of 2'-O-methylated UTP (Trilink), T7 R&DNA™ Polymerase (Eipcentre, Madison, USA) was used. This polymerase has single-base active-site mutations that allow the incorporation of NTPs with 2'-substituents such as 2'-O-methyl. In vitro transcription was carried out overnight at 37 °C. The DNA template was digested using DNase I (Fermentas) and subsequently RNAs were purified using the Roche high pure RNA isolation kit (Roche Applied Science, Mannheim, Germany) with the following modifications: Binding buffer was 2.0 M guanidine thiocyanate in 70 % ethanol and wash buffer was substituted by 100 mM NaCl, 4.5 mM EDTA, 10 mM Tris HCl in 70 % ethanol. After elution, excess salts and NTPs were removed by passing the RNAs through a Mini Quick Spin™ Oligo Column (Roche). Size and integrity of RNAs was checked via gel electrophoresis.

**Table 1**

| A: DNA oligonucleotides for the generation of in vitro transcription templates: | | |
|---|---|---|
| Name | Sequence | corres. strand |
| AF6.5-35n | 5'-CAGTAATACGACTCACTATTAGGGAAGCGGGCA-3' | 1 |
| GF6.5-35n | 5'-CAGTAATACGACTCACTATAGGGGAAGCGGGCA-3' | 1 |
| RNA9.2s-0A | 5'-TTGAAGGACAGGTTAAGCTAATAGTGAGTCG-3' | 2 |
| RNA9.2s-1G | 5'-ATTGAAGGACAGGTTAAGCTATAGTGAGTCGTA-3' | 3 |
| RNA9.2s-5A | 5'-GGTAATTGAAGGACAGGTTAATAGTGAGTCG-3' | 2 |
| tri-09-mer | 5'-GGGATCCCCTATAGTGAGTCGTA-3' | 3 |
| tri-12-mer | 5'-GGGTTCATCCCCTATAGTGAGTCGTA-3' | 3 |
| tri-15-mer | 5'-GGGAAGTTCATCCCCTATAGTGAGTCGTA-3' | 3 |
| tri-18-mer | 5'-GGGCTGAAGTTCATCCCCTATAGTGAGTCGTA-3' | 3 |
| tri-21-mer | 5'- GGGACCCTGAAGTTCATCCCCTATAGTGAGTCGTA-3' | 3 |
| tri-24-mer | 5'- GGGCTGACCCTGAAGTTCATCCCCTATAGTGAG- TCGTA-3' | 3 |
| tri-27-mer | 5'- GGGAAGCTGACCCTGAAGTTCATCCCCTATAGT- GAGTCGTA-3' | 3 |
| tri-G-AC-U-Bio | 5'-AAATGTGTGTGTGTGTGTGTGCCTGTCTC-3' | 5 |
| tri-GFPa | 5'- AAGATGAACTTCAGGGTCAGCCCCTATAGT- | 3 |
| | GAGTCGTA-3' | |
| tri-GFPs | 5'- AAGCTGACCCTGAAGTTCATCCCCTATAGTGAGTCG- TA-3' | 3 |
| tri-Poly A | 5'-TTTTTTTTTTTTTTTTTTTTTCCTGTCTC-3' | 5 |
| tri-Poly C | 5'-GGGGGGGGGGGGGGGGGGGGGCCTGTCTC-3' | 5 |
| tri-Poly G | 5'-CCCCCCCCCCCCCCCCCCCCCCCTGTCTC-3' | 5 |
| tri-Poly T | 5'-AAAAAAAAAAAAAAAAAAAAACCTGTCTC-3' | 5 |
| tri-singleG-24mer | 5'- AAAGTGTGTGTGTGTGTGTGTGTGTCTATAGTGAGTCG- TA-3' | 3 |
| tri-27+2s | 5'- AAGTGGTGCAGATGAACTTCAGGGTCAGCTATAGT- GAGTCGTA-3' | 3 |
| tri-27+2a | 5'- AAGCTGACCCTGAAGTTCATCTGCACCACTATAGT- GAGTCGTA-3' | 2 |
| tri-27+0s | 5'- GGTGCAGATGAACTTCAGGGTCAGCTTAATAGT- GAGTCG-3' | 3 |
| tri-27+0a | 5'- AAGCTGACCCTGAAGTTCATCTGCACCTATAGT- GAGTCGTA-3' | 3 |
| RV leader RNA | 5'- ACATTTTTGCTTTGCAATTGA- CAATGTCTGTTTTTTCTTTGAT CTGGTTGTTAAGCGTTATAGTGAGTCGTATTACGCG- 3' | 4 |

### Corresponding strands:

| Name | Sequence |
|---|---|
| 1 | 5'-TGATCGGCTATGGCTGGCCGCATGCCCGCTTCC-3' |
| 2 | 5'-CAGTAATACGACTCACTATTA-3' |
| 3 | 5'-TAATACGACTCACTATA-3' |
| 4 | 5'-AATTCGCGTAATACGACTCACTATA-3' |
| 5 | Ambion T7 Promoter Primer |

B: PCR primer for the generation of in vitro transcription templates using pBKS as the PCR template

### Forward primer:

| Name | Sequence |
|---|---|
| pBKS T7 prom. | 5'-GGATCCTAATACGACTCACTATAGGGCGA-3' |

### Backward primer:

| Name | Sequence |
|---|---|
| pBKS 27-mer | 5'-CACCGCGGTGGAGCTCCAATTCGCCCTAT-3' |
| pBKS 57-mer | 5'-CGGGGGATCCACTAGTTCT-3' |
| pBKS 105-mer | 5'-CCTCGAGGTCGACGGTATC-3' |
| pBKS 204-mer | 5'-CGGATAACAATTTCACACAGGA-3' |
| pBKS 302-mer | 5'-AGTGAGCGCAACGCAATTA-3' |

### RNA isolation

RNA from E. coli strain DH10B and human PBMC was isolated using Trizol® reagent (Invitrogen, Karlsruhe, Germany) according to the manufacturer's protocol. CIAP treatment was performed the following way: 10 µg in vitro transcribed RNA, 15 µg cellular RNA or 1.5 µg viral RNA was treated with 30 U of calf intestine alkaline phosphatase (CIAP) (Stratagene, La Jolla, USA) for 3 hours at 37 °C in a buffer containing 50 mM Tris-HCl, 0.1 mM EDTA in the presence of 10U of RNase inhibitor (RNAguardTM; Amersham-Biosciences). Following CIAP treatment, the RNA was cleaned up using the RNeasy Mini kit.

### Cell extracts

Cell lysates were prepared according to Meister et al. (G. Meister et al., Mol Cell 15, 185 (Jul 23, 2004)) with minor modifications. HEK 293 cells were transfected using high molecular weight (25 kDa) polyethylenimine (PEI; Sigma, 40.872-7). At a confluency of 80-90 %, cells were transfected with a PEI : DNA ratio of 1.5 : 1. 24-36 hours after transfection cells were harvested and the cell pellet was resuspended in five pellet volumes of 10 mM KCI, 1.5 mM MgCl2, 0.5 mM dithiothreitol, 10 mM HEPES-NaOH (pH 7.9), 0.5 mM PMSF and incubated for ten minutes on ice. Subsequently cells were washed and the cell pellet was resuspended in two pellet volumes of the buffer described above and homogenized by douncing. The cell nuclei were removed from the cell lysate by centrifugation at 2.000 g for ten minutes. The supernatant was transferred into microcentrifuge tubes and cleared further by cenrifugation at 2.000 g for ten minutes and further centrifugation for 30 minutes at 20.000 g to obtain the cytoplasmic extract. The concentration of KCI of the extract was subsequently raised to 100 mM by addition of 2 M KCI and glycerol was added to a percentage of 10 %. For purification of FLAG-tagged RIG-IC complexes, cytoplasmic extracts were incubated in FLAG M2 agarose beads (Sigma). FLAG M2 agarose beads were washed once with 0.1 M glycine (pH 3.5) and equilibrated by washing with 1 M Tris-HCl (pH 8.0). The beads were then resuspended in buffer C (0.1 M KCI, 5 mM MgCl2, 10 % glycerol, 10 % Tween20, 10 mM β-mercaptoethanol, 0.2 mM PMSF, and 20 mM Tris-HCl [pH 8.0]) and incubated with cytoplasmic extracts for four hours at 4 °C with rotation. The beads were collected and washed twice in wash buffer (300 mM NaCl, 5 mM MgCl2, 50 mM Tris-HCl [pH 7.5]) supplemented with 0.1 % NP40. Affinity-bound complexes were then eluted by shaking the beads in 0.2 µg/ ml 3xFLAG peptide (Sigma) in wash buffer for two hours at 10 °C and after centrifugation the eluate was collected.

### Ligand binding studies

Whole cell lysate or 25 µl RIG-IC eluate was incubated with 0.375 µg biotinylated RNA in the presence of 40U RNase inhibitor (Fermentas), 0.5 mM PMSF in a final volume of 100 µl in wash buffer for two hours at 4 °C with rotation. 50 µl streptavidin-coated beads (Pierce, Rockford, USA; 20347) were added to the lysate for another hour at room temperature with rotation. Beads were then washed four times with wash buffer supplemented with 0.1 % NP40. Supernatant and beads were lysed in Laemli buffer for further immunoblot analysis.

### Western blotting

For Western blotting, samples were separated by SDS-PAGE and transferred to a nitrocellulose membrane (Amersham-Biosciences, UK) by semi-dry electroblotting. As primary antibody, monoclonal anti-Flag antibody (Sigma) was used. As secondary antibody, peroxidase-conjugated anti-mouse antibody (Amersham-Biosciences) was used. Bound antibodies were visualized by enhanced chemiluminescence system (ECL) according to the manufacturer's protocol (Amersham-Biosciences).

### Reporter assays

12-16 hours prior to transfection, HEK 293 cells were seeded in 48-well plates. At a confluency of 80 %, HEK 293 cells were transfected using PEI with 300 ng of a reporter plasmid (pIFNß-luc), 500 ng of a normalisation plasmid (expressing Rous sarcoma virus ß-galactosidase) and the indicated expression plasmids giving a total of 1,5 µg DNA/ well. 24 hours after transfection culture medium was aspirated and the cells washed once in 0.5ml PBS containing 10 mM EDTA. Then cells were lysed in 50 µl luciferase lysis buffer (10 % glycerol, 1 % Triton-X, 2 mM EDTA, 25 mM TrisHCl [pH 7.8], 2 mM DTT). 20 µl of each sample were mixed with 20 µl of Luciferase Detection Reagent (Promega) and analyzed for luciferase activity with a microplate luminometer (LUMistar, BMGLabtechnologies). To measure beta-galactosidase activity, 10 µl lysate was incubated with 100 µl of solution 1 (1 % Galacton-Plus [TROPIX], 0.1 % 0.1 M MgCl₂, 20 % 0.5 M phosphate [pH 8], 78.9 % H₂O) for 20 minutes and then 50 µl of solution 2 was added (20 % 1 M NaOH, 10 % Emerald [TROPIX] 70 % H₂O). Luciferase activity values were normalized against beta-galactosidase activity of the same extract. Reporter assays for experiments involving viral infection (figure 5) were performed the following way: 12 to18 hours prior to transfection, HEK 293T or Vero cells were seeded in 24-well plates. At a confluency of 80 %, the cells were transfected using Lipofectamine 2000 (Invitrogen) with 400 ng of a reporter plasmid encoding firefly luciferase (p125-Luc) and 2 ng of a plasmid encoding CMV-controlled renilla luciferase (pRL-CMV, Promega) for normalization along with 400 ng of empty vector of RIG-expressing plasmids when indicated. 6 hours after DNA transfection the cells were either infected or transfected with the indicated amounts of RNA using PEI. 48 hours after DNA transfection the cell extracts were prepared and assayed in the Dual Luciferase Reporter System (Promega). Luciferase activity was measured in a Luminometer (Berthold) according to the supplier's instructions.

### Plasmids

pIFN-beta-Luc was kindly provided by T. Maniatis. RIG-1 CARD2 was kindly provided by S. Rothenfusser. p125-Luc. RIG-I full, RIG-IC, RIG-I K270A and the empty control vector were kindly provided by T. Fujita (M. Yoneyama et al., Nat Immunol 5, 730 (Jul, 2004)). RIG-I ΔHelicase_C (AS 655-734) was constructed from RIG-I full via loop out PCR using the following PCR primer pair: 5'- ACTGAGTTTAGGATTTCCTTCAATCC-3', 5'-GGTAGCAAGTGCTTCCTTCTGA-3'. pSC6-T7-NEO was kindly provided by M. Billeter F. (Radecke et al., Embo J 14, 5773 (Dec 1, 1995)). T7 D812N was constructed from pSC6-T7-NEO via site directed mutagenesis using the following PCR primer pair: 5'-GCACTGATTCACGCCTCCTTCGGTACC-3', 5'-GGTACCGAAGGAGGCGTGAATCAGTGC-3'. RIG-I ΔHelicase_C and T7 RNA D812N were confirmed by sequencing.

### Virus stocks

Recombinant RV SAD L16 (Schnell MJ et al., 1994, EMBO J. 13(18):4195-4203) was used as wt RV. Cloning of cDNA, recovery of recombinant SAD ΔPLP virus, which encodes P from the most promoter-distal gene position, and virus propagation, was described previously (K. Brzozka, et al. Journal of virology 79, 7673 (Jun, 2005)).

For isolation of total RNA from non-infected cells or from cells infected with RV at MOI of 1 for 2 days, the RNeasy minikit (QIAGEN, Hilden, Germany) was used according to manufacturer's instructions. For isolation of RV particle RNA, virions were pelleted from cell-free supernatants by ultracentrifugation in SW32Ti for 2h at 4°C and 27,000 rpm. RNA was isolated from pellets with the RNeasy minikit.

### Media and reagents

RPMI 1640 (Biochrom) supplemented with 10% (v/v) heat-inactivated FCS (Invitrogen Life Technologies), 3 mM L-glutamine, 0.01 M HEPES, 100 U/ml penicillin, and 100 µg/ml streptomycin (all from Sigma-Aldrich) and Dulbecco's modified Eagle's medium (PAN, Aidenbach, Germany) supplemented with 10% fetal calf serum (FCS), 3 mM L-glutamine, 100 U/ml penicillin and 100 µg/ml streptomycin was used. CpG ODNs (Coley Pharmaceutical Group) show small letters, phosphorothioate (PT) linkage and capital letters, phosphodiester (PD) linkage 3 of the base:; CpG-A ODN 2216 5'-ggGGGACGATCGTCgggggG-3', CpG-B ODN 1826 (5'-TCCATGACGTTCCTGACGTT-3'). R848 was purchased from InvivoGen.

### RNAs

Chemically synthesized RNA oligonucleotides were purchased from Eurogentec (Leiden, Belgium). *In vitro* transcribed RNAs were synthesized using the megashort script kit (Ambion, Huntingdon, UK). Using partially overlapping single stranded DNA oligonucleotides, a double-stranded DNA template was constructed using Exo⁻ Klenow (Eppicentre) (for a detailed list of all in vitro transcription templates see table 1). The obtained templates contained a T7 RNA Polymerase consensus promoter followed by the sequence of interest to be transcribed. The DNA template was then assembled in a reaction mix containing T7 10x Buffer, the four ribonucleotide solutions and the T7 RNA Polymerase in RNAse free water. *In vitro* transcription was carried out overnight at 37 °C. To generate *in vitro* transcribed double stranded RNA the sense and antisense RNA templates were transcribed for 2 hours in separate reactions. The reactions were then mixed, and the combined reaction was incubated overnight. The DNA template was digested using DNAse I (Ambion) and subsequently RNAs were purified using phenol:chloroform extraction and alcohol precipitation. After elution, excess salts and NTPs were removed by passing the RNAs through a Mini Quick Spin™ Oligo Column (Roche). Integrity of RNAs was checked via gel electrophoresis.

### Isolation of cells

Human PBMC were prepared from whole blood donated by young healthy donors by Ficoll-Hypaque density gradient centrifugation (Biochrom, Berlin, Germany). First, PDCs were depleted by magnetically activated cell sorting (MACS) using the blood dendritic cell Ag (BCDA)-4 dendritic cell isolation kit from Miltenyi Biotec (Bergisch-Gladbach, Germany). The purity of isolated PDC (lineage-negative, MHC-II-positive and CD123-positive cells) was above 95 %. Secondly, human CD3⁺ T cells were isolated with anti-CD3 MicroBeads (Miltenyi Biotec) and passed through a magnetic separation column (LS; Miltenyi Biotec). Memory B cells were positively selected from the negative fraction with anti-CD27 microBeads (Miltenyi Biotec) on a LS column. Naive B cells were isolated from the remaining CD27 negative fraction by positive selection with anti-CD19 MicroBeads (Miltenyi Biotec) on a LS column. The purity of cell fractions yielded 96-99% for memory B cells (IgD⁻CD27⁺) and 95-99% for naïve B cells (IgD⁺CD27⁻) as analyzed by flow cytometry. Then monocytes, myeloid dendritic cells and NK cells were isolated using CD14, BDCA-1 and CD56 microbeads (Miltenyi Biotec). FLT-3 ligand induced mixed cultures of murine myeloid and plasmacytoid dendritic cells were grown as described (3). Plasmacytoid DC from FLT-3 ligand induced bone marrow cultures were sorted with B220 microbeads (Miltenyi Biotec). Conventional dendritic cells (cDC) were generated by incubating pooled bone marrow cells in the presence of murine GM-CSF (10 ng/ml; R&D Systems, Minneapolis, MN). After 7 days, these cultures typically contained more than 90 % cDC (CD11c+, CD11b+, B220-). For isolation of resting murine B cells, NK cells and CD8 T cells spleens were magnetically labeled using anti-B220 microbeads (Miltenyi Biotec) as well as the NK - and CD8 T cell isolation kit. Viability of all cells was above 95 %, as determined by trypan blue exclusion and purity was > 97% as analyzed by FACS Human and murine primary cells were cultivated in RPMI (PAN, Aidenbach, Germany) supplemented with 10% fetal calf serum (FCS), 4mM L-glutamine and 10-5 M mercaptoethanol. Murine B16 melanoma cells were a kind gift of Thomas Tüting and cultivated in Dulbecco's modified Eagle's medium (PAN, Aidenbach, Germany) supplemented with 10% fetal calf serum (FCS), 3 mM L-glutamine, 100 U/ml penicillin and 100 µg/ml streptomycin.

### Cell culture

All cells, except human PDC (2.5*10⁶ cells / ml), were cultured at a density of 2*10⁶ cells /ml. PDC cultures were additionally supplemented with 10 ng/ml IL-3 (R&D Systems).
Cells were seeded in 96-well U-bottom plates (BD Biosciences), respectively. If not indicated otherwise, cells were incubated for 24 hours with 3 µg/ml CpG-B-DN 2006 and/or CpG-ODN 2216, 1 µM R848. We transfected the in vitro transcribed RNA with Lipofectamine 2000 (Invitrogen). If not indicated otherwise, we transfected 200 ng of nucleic acid with 0,5µl of Lipofectamine. After 24 hours the supernatants were collected for analysis of cytokine secretion by enzyme-linked immunosorbent assay (ELISA), and cells were harvested for flow cytometric analysis.

### Cytokine measurement

Concentrations of human and murine IFN-α, IL-12 p40 or IL-6 in the culture supernatants were determined by ELISA according to the manufacture's instructions (BD PharMingen, San Diego, CA). Murine IFN-α was analysed using a PBL Biomedical Laboratories (New Brunswick, NJ) ELISA kit. For some experiments, murine IFN-α was measured according to the following protocol: monoclonal rat anti-mouse IFN-α (clone RMMA-1) was used as the capture Ab, and polyclonal rabbit anti-mouse IFN-α serum for detection (both PBL Biomedical Laboratories) together with HRP-conjugated donkey anti-rabbit IgG as the secondary reagent (Jackson ImmunoResearch Laboratories). Mouse rIFN-α (PBL Biomedical Laboratories) was used as the standard (IFN-α concentration in IU/ml). Human IFN-α was assessed in cell culture supernatants using the IFN-a module set (Bender MedSystems, Graz, Austria).

### Transfection and reporter assay

For monitoring transient IFN-β activation by 5'triphosphate RNA murine B16 cells were seeded in 24-well plates. At a confluency of 70 %, B16 cells were transfected using high PEI with 500 ng of a reporter plasmid (pIFNß-luc DAM/DCM), 50 ng of a normalisation plasmid (expressing Renilla -Luc) and the indicated expression plasmids giving a total of 1,5 µg DNA/ well. B16 cells were transfected using high molecular weight (25 kDa) polyethylenimine (PEI; Sigma, 40.872-7) with a PEI : DNA ratio of 1.5 : 1. 24 hours after transfection culture medium was aspirated and the cells washed once in 0.5ml PBS containing 10mM EDTA. Then cells were lysed in 50µl luciferase lysis buffer (10 % glycerol, 1 % Triton-X, 2mM EDTA, 25mM TrisHCl [pH 7.8], 2mM DTT). 20µl of each sample were mixed with 20µl of Luciferase Detection Reagent (Promega) and analyzed for luciferase activity with a microplate luminometer (LUMistar, BMGLabtechnologies). To measure Renilla luciferase activity, 20µl lysate was incubated with 20µl of Renilla substrate. Luciferase activity values were normalized against Renilla activity of the same extract.

### Plasmids

pIFNβ-Luc was kindly provided by T. Maniatis. RIG-I full, RIG-IC, RIG-I K270A and the empty control vector were kindly provided by T. Fujita (Yoneyama M et al. 2004, Nat. Immunol. 5(7):730-737).

### Western blotting

For Western blotting, samples were separated by SDS-PAGE and transferred to a nitrocellulose membrane (Amersham-Biosciences, UK) by semi-dry electroblotting. As primary antibody, polyclonal anti-BCL-2 antibody (Santa Cruz) was used. As secondary antibody, peroxidase-conjugated anti-mouse antibody (Amersham-Biosciences) was used. Bound antibodies were visualized by enhanced chemiluminescence system (ECL) according to the manufacturer's protocol (Amersham-Biosciences).

### Flow cytometry

At the time points indicated, surface antigen staining was performed as described previously (http://www.bloodjournal.org/cgi/content/full/103/8/3058#REF4). Fluorescence-labelled monoclonal antibodies (mAbs) against human CD27 and IgD, CD14, HLA-DR, CD123, CD11c, BDCA-2, mouse B220, mouse CD11c, mouse NK1.1, mouse CD4, mouse CD8, mouse CD69, mouse CD86 and appropriate isotype control antibodies were purchased from BD Pharmingen (Heidelberg, Germany). Flow cytometric data were acquired on a Becton Dickinson FACSCalibur equipped with 2 lasers (excitation at 488- and 635-nm wavelength). Data were analyzed using Cellquest software (Becton Dickinson, Heidelberg, Germany).

### Confocal microscopy

C57/BL6 mice were injected intravenously with FITC labelled RNA (50 µg) complexed to jetPEI (Biomol) After 3h mice were sacrificed and the desired organs were analysed for uptake of the RNA complexes. Briefly, lung sections were transferred on a poly-L-lysine coated microscope slide and fixed in acetone for 10 min. Nuclear counterstaining was performed using TOPRO-3 (Molecular Probes). Washing steps were done in Tris-buffered saline and cells were mounted in Vectarshield Mounting Medium (Vector Laboratories). Cells were then analysed using a Zeiss LSM510 confocal mircroscope (Carl Zeiss, Germany) equiped with 488nm-Argon and 633nm-Helium-Neon lasers. Images were obtained taking one optical section of the cells.

### Mouse studies

For in vivo studies, we injected C57/BL6 mice with 200 µl containing nucleic acids with prior jetPEI-complexation. Briefly, 10 µl of *in vivo* jetPEI was mixed with 50 µg of nucleic acids at a N/P ration of 10:1 in 5% Glucose solution and incubated for 15 min. Subsequently, the complexes were injected in the retro-orbital vein. Serum was collected after 6 h unless indicated otherwise. Whole blood was obtained by tail clipping at the indicated time points. Serum was prepared from whole blood by coagulation for 30 min at 37 °C and subsequent centrifugation and stored at -20°C. Cytokine levels were determined by ELISA.

### Engraftment of B16 melanoma in the lungs

B16 is a spontaneous murine melanoma and was cultivated in Dulbecco's modified Eagle's medium (PAN, Aidenbach, Germany) supplemented with 10% fetal calf serum (FCS), 3 mM L-glutamine, 100 U/ml penicillin and 100 µg/ml streptomycin. Engraftment of melanoma in the lungs was done by i.v. injection of 4x10⁵ B16 melanoma cells. 14 days after challenge the number of macroscopically visible melanoma metastases on the surface of the lungs was counted with the help of a dissecting microscope. Experiments were done in groups of five mice and repeated two to four times

### Histopathologic and immunohistochemical analyses

Samples of skin, lymph nodes, and lungs were obtained when mice were sacrificed. Tissue specimens were in part fixed in 10% buffered formalin, embedded in paraffin, and routinely stained with H&E. For immunohistopathologic investigations, a zinc-based fixative was used as an alternative to formalin (DAKO, Hamburg, Germany). To confirm the melanocytic origin of tumor cells, sections were immunostained with the TRP2-specific polyclonal rabbit antibody Pep8 (a kind gift from Vincent Hearing, NIH, Bethesda, MD) followed by a biotin-conjugated anti-rabbit secondary antibody and the LSAB-2 color development system (both from DAKO).

### Example 1. In vitro transcribed RNA stimulates IFN-α production in human primary monocytes.

IFN-α production in the human immune system is thought to be largely confined to PDC. IFN-α production in human primary monocytes has not been reported so far. As demonstrated in previous studies (V. Homung et al., J Immunol 168, 4531 (May 1, 2002); I. B. Bekeredjian-Ding et al., J Immunol 174, 4043 (Apr 1, 2005)), monocytes express TLR2, TLR4, TLR6 and TLR8 but no TLR3, TLR7 or TLR9, and produce IL-6 in response to TLR2/6- TLR4- and TLR8-ligands but not to TLR3-, TLR7- or TLR9-ligands (I. B. Bekeredjian-Ding et al., J Immunol 174, 4043 (Apr 1, 2005)). Monocytes failed to produce IFN-α upon stimulation with all TLR ligands tested including CpG-A ODN 2216 (A. Krug et al., Eur J Immunol 31, 2154 (Jul, 2001)) and R848, both of which induce IFN-α in PDC (Fig. 1 and data not shown). We hypothesized that motif patterns or sequences in RNA may exist in long RNA molecules that induce IFN-α in monocytes.

*In vitro* transcription was used to generate long ssRNA molecules as chemical synthesis is impracticable to generate ssRNA larger than 100 nucleotides. RNA transcripts were transfected in monocytes and PDC and IFN-α production was assessed by ELISA.

The present inventors found that a 2500-nucleotide long RNA molecule, but not the TLR9 ligand CpG-A ODN 2216 or the TLR7/8 ligand R848, stimulated a strong IFN-α response in primary human monocytes (Fig. 1A).

The templates that were used to generate the set of ssRNA molecules of different lengths (27-302 nucleotides) were identical at the 5' end, whereas the 3' end was gradually shortened. As a consequence, this set of ssRNA molecules was identical in sequence at the 5' end. IFN-a induction in monocytes was also seen when *in vitro* transcribed RNA molecules of different length (from 27 nucleotides to 302 nucleotides) were used (Figure 1B).

Next, whether the length of the 3' sequence impacts on the IFN-α-inducing activity of 5' phosphate RNA was tested. 5' triphosphate RNA oligonucleotides ranging from 27 to 9 nucleotides were generated by the gradual shortening (in steps of three nucleotides) of a 27-mer oligonucleotide from the 3' end. Whereas RNA oligonucleoties 27, 24 and 21 nucleotides in length were potent inducers of IFN-α in monocytes, a sharp drop of activity was observed for shorter sequences (Fig. 1C). This suggested that *in vitro* transcribed RNA had to have a minimal length of 21 bases to induce IFN-α in monocytes.

Since the results presented in Fig. 1B may be interpreted to suggest that the 3' sequence may influence the IFN-α inducing activity of 5' triphosphate RNA, 31-mer (i.e., 31-nucleotide long) 5' triphosphate RNA oligonucleotides were generated in which the 3' seqeunce (21 nucleotides) was either a poly G (tri-poly. G), a poly A (tri-poly A), a poly C (tri-poly C) or a poly U (tri-poly U) homopolymer. The ten bases at the 5' end were identical for these oligonucleotides. All four RNA oligonucleotides turned out to be equally potent in terms of IFN-α induction in monocytes (Fig. 1 D).

These results indicated that a minimal length is required for the 5' triphosphate RNA to be recognized. Although these results suggested that the 3' sequence of *in vitro* transcribed RNA oligonucleotides had no strong impact on the IFN-α-inducing activity, data with larger RNA molecules (Fig. 1B) pointed to a possible influence of secondary structure formation.

Furthermore, these results indicated that a molecular characteristic shared by all *in vitro* transcribed RNA molecules rather than a specific sequence motif is responsible for IFN-α induction in monocytes.

### Example 2. The 5' triphosphate moiety of in vitro transcribed RNA is responsible for IFN-α induction in human primary monocytes.

In general, for *in vitro* transcription of RNA, the bacteriophage T7 DNA-dependent RNA polymerase is used. Unlike synthetic RNA or eukaryotic mRNA, RNA generated by T7 RNA polymerase contains an uncapped triphosphate group at the 5' end of the RNA molecule. To study the sequence-independent contribution of the 5' triphosphate, IFN-α induction by a synthetic and an *in vitro* transcribed version of an immunostimulatory ssRNA oligonucleotide 9.2s (isRNA9.2s, 19 nucleotides) was compared. isRNA9.2s was identified as a potent stimulus for IFN-α production in PDC in previous studies (V. Homung et al., Nat Med 11, 263 (Mar, 2005)).

Only the *in vitro* transcribed version of isRNA9.2s, but not synthetic isRNA9.2s, strongly induced IFN-α production in monocytes (Fig. 2A upper panel). This difference in IFN-α inducing activity was not due to different transfection efficiency (Fig. 7). In contrast to monocytes, PDC produced IFN-α in response to both *in vitro* transcribed and synthetic isRNA9.2s (Fig. 2A lower panel).

Next, *in vitro* transcription was used to generate a dsRNA oligonucleotide with an overhang of one nucleotide at the 5' position. The two single-stranded oligonucleotides (tri-GFPs, tri-GFPa) and the double-stranded oligonucleotide (tri-GFPds) induced comparable levels of IFN-α in monocytes (Fig. 2B). Cleavage of the 5' overhang (including the 5'triphosphate) of the dsRNA (tri-GFPds) by RNAse T1, an endoribonuclease that specifically degrades single-stranded RNA at G residues, completely abolished the IFN-α inducing activity (Fig. 2B). Moreover, when calf intestine alkaline phosphatase (CIAP) was used to dephosphorylate the 5' end of the *in vitro* transcribed single-stranded RNA oligonucleotides, a complete abrogation of the IFN-α response was observed in monocytes (Figure 2C). In contrast, PDCs, which are known to detect single-stranded RNA oligonucleotides via TLR7, showed no decrease in IFN-α production when oligonucleotides were dephosphorylated (Figure 2C).

Unlike the oligonucleotide with a guanosine-5'-triphosphate, *in vitro* transcribed RNA generated to contain a guanosine-5'-diphosphate, a guanosine-5'-monophosphate or a guanosine-5'-hydroxyl did not induce IFN-α in monocytes (Fig. 8).

Together, these data indicated that the 5'triphosphate is responsible for the IFN-α inducing activity of *in vitro* transcribed RNA in monocytes, and that a 5' triphosphate confers IFN-α-inducing activity to both ssRNA and dsRNA.

### Example 3. 7-methyl-guanosine capping and eukaryote-specifc base modifications abolish IFN-α induction via 5' triphosphate RNA.

In eukaryotic cells, 7'methyl-guanosine is attached to the 5' triphosphate of a nascent mRNA transcript by a process called capping. Capping improves the stability of eukaryotic RNA against nucleases and enhances binding of ribosomal proteins to mRNA.

The influence of capping on the IFN-α inducing activity of 5' triphosphate RNA was examined. Capped RNA can be generated via *in vitro* transcription by including a synthetic cap analog, N-7 methyl GpppG, in the *in vitro* transcription reaction. Since both N-7 methyl GpppG and GTP (typically in a 4:1 mixture of N-7 methyl GpppG:GTP) need to be present during *in vitro* transcription and both are incorporated by T7 RNA polymerase, approximately 80 % of all transcripts are capped after *in vitro* transcription. It was found that RNA of different lengths transcribed in the presence of the synthetic cap analog, which contained approximately 20 % uncapped and 80 % capped RNA, was much less active at inducing IFN-α production in monocytes when compared to uncapped *in vitro* transcribed RNA (100 % uncapped) (Fig. 3A).

Besides 5' capping, eukaryotic RNA undergoes several other posttranscriptional maturation steps including the modification of various nucleosides of the RNA transcript and the methylation of the backbone ribose at the 2'-hydroxyl position. In this respect, it has been previously shown that the incorporation of nucleoside modifications that are abundant in matured eukaryotic, but not in prokaryotic or viral RNA can lead to the complete abrogation of a RNA-triggered inflammatory response mediated via the TLR-system (K. Kariko, et al. Immunity 23, 165 (Aug, 2005). To test whether this phenomenon holds also true for 5' triphosphate RNA triggered IFN-α response, RNA oligonucleotides were generated via *in vitro* transcription with various NTPs substituted with the respective nucleoside- or ribose-modified NTPs.

A significant decrease in IFN-α production was seen when either pseudouridine (Ψ) or 2-thiouridine (s2U) substituted for uridine (U) (Fig. 3B). Analogous results were obtained when 2'-O-methylated UTP was incorporated into the 5' triphosphate RNA oligonucleotides instead of UTP (Fig. 3C). In accordance with these results, transfection of prokaryotic RNA that lacks 5' caps and is low in the respective nucleoside and ribose modifications resulted in a strong IFN-α response in monocytes, whereas eukaryotic RNA was completely inactive in terms of IFN-α induction (Fig. 9).

Lipopolysaccharide (LPS) alone or in combination with synthetic RNA did not contribute to IFN-α production in monocytes (Fig. 9).

Structural features like the presence of a two-nucleotide 3' overhang in a 5' triphosphate RNA duplex, as it occurs in natural cleavage products of the endonuclease dicer, did not interfere with the immunostimulatory activity of the 5' triphosphate RNA oligonucleotides (Fig. 10).

Altogether, these results indicated that posttranscriptional modifications commonly found in mature eukaryotic RNA species suppress the immunostimulatory activity of 5' triphosphate RNA oligonucleotides, thereby providing molecular structures that can be employed for the distinction of self and non-self RNA.

### Example 4. IFN-α induction by 5'triphosphate RNA oligonucleotides is independent of endosomal maturation.

Among the family of TLRs, TLR3, TLR7, TLR8 and TLR9 are known to detect nucleic acids. A number of studies suggest that single-stranded RNA is recognized via TLR7 and TLR8, both located in the endosomal membrane. Similar to CpG-DNA, recognition of single-stranded RNA by TLR7/8 can be blocked by chloroquine, which inhibits endosomal maturation. The present inventors found that in PBMC, increasing concentrations of chloroquine inhibited IFN-α induction by CpG-A but not by 5'triphosphate RNA (Fig. 12A); furthermore, chloroquine did not affect 5' triphosphate RNA induced IFN-α production in isolated monocytes (Fig. 12B). CpG-A is inactive in monocytes with and without chloroquine due to the lack of TLR9 (Fig. 12B).

In analogy to the human system, murine bone marrow cells and myeloid dendritic cells produced vast amounts of IFN-α upon transfection with 5'triphosphate RNA. IFN-α and IP-10 induction in bone marrow-derived myeloid dendritic cells from TLR7-/- mice (Fig. 12C) or LPS2 -/- mice (data not shown) was comparable to the level of IFN-α induction in wild type mice.

Altogether these data suggested that the recognition of 5'triphosphate RNA does not require endosomal maturation, and that TLR3, TLR7/8 or TLR9 are not involved.

### Example 5. Type I IFN induction by exogenous and endogenous 5' triphosphate RNA requires RIG-I but not MDA5.

In previous studies we found that TLR7-mediated recognition of synthetic immunostimulatory RNA requires complexation with a cationic polymer which enables endosomal delivery and confers protection against nuclease degradation, but not transfection of RNA into the cytosol. In contrast to synthetic isRNA, 5' triphosphate RNA induced IFN-α in monocytes only when transfected into the cytosol by cationic lipids, whereas complexation with cationic peptides was not sufficient (data not shown). Consistent with these observations, 5' triphosphate RNA mediated IFN-α induction required neither endosomal maturation nor TLR7 (Fig. 11) or TLR3 (data not shown). These results indicated that the receptor for 5' triphosphate RNA is located in the cytosol and not in the endosomal compartment.

RIG-I and MDA-5 are cytoplasmic proteins involved in the recognition of RNA viruses (H. Kato et al., Nature 441, 101 (Apr 9, 2006)); both RIG-I and MDA-5 are thought to be involved in dsRNA recognition. Although 5' triphosphate RNA in the present invention was active as ssRNA, it remained to be determined whether RIG-I or MDA-5 are involved in 5' triphosphate recognition.

In order to address the effect of dominant negative mutants of RIG-I, HEK 293 cells expressing the reporter luciferase under the control of the IFN-β promoter were used instead of monocytes. As expected, HEK 293 cells transiently transfected with RIG-I did not respond to poly(I:C) or synthetic isRNA (RNA9.2s) (Fig. 4A). However, unexpectedly, single-stranded 5' triphosphate RNA (tri-GFPs and tri-GFPa) strongly activated reporter expression in RIG-I expressing HEK 293 cells. Only HEK 293 cells expressing full length RIG-I responded to 5'triphosphate RNA; HEK293 cells expressing truncated RIG-I which lacked the N terminal CARD domain or RIG-I 270KA mutant devoid of the ATPase activity did not.

To confirm that RIG-I was required for the recognition of 5' triphosphate RNA, the activity of 5' triphosphate RNA in RIG-I-/- MEFs was tested. Whereas wild type MEFs produced large amounts of IFN-β (Fig. 4B) and IL-6 (data not shown) in response to 5' triphosphate RNA stimulation, no response was detected in RIG-I-/- MEFs (Fig. 4B). The response to 5' triphosphate RNA in MDA-5 -/- MEFs was similar to wild type MEFs.

Together, these data provided evidence that RIG-I, but not MDA-5, is required for the recognition of 5' triphosphate RNA and that the recognition of 5' triphosphate RNA is not confined to immune cells such as primary monocytes.

It is hypothesized that since 5' triphosphate RNA is recognized via RIG-I, the formation of endogenous 5' triphosphate RNA via cytoplasmic overexpression of T7 RNA polymerase should trigger the type I IFN pathway. To test this hypothesis, a system, which has been extensively used to generate recombinant negative strand RNA viruses (NSV) from *in vivo* transcribed cDNA in the context of reverse genetics approaches (F. Radecke et al., Embo J 14, 5773 (Dec 1, 1995)), was employed. This system allows template-dependent direct transcription of RNA inside a cell via cytosolically expressed T7 RNA polymerase.

Indeed, coexpression of wild type RIG-I and wild type T7 RNA polymerase, in the absence of exogenously added 5' triphosphate RNA, strongly induced a type I IFN response (Fig. 4C). No type I IFN response was detected when a combination of wild type RIG-I and a mutated form of T7 RNA polymerase (T7 D812N) or a combination of mutant RIG-I (RIG-IC) and wild type T7 RNA polymerase was expressed.

At high levels of expression, a template-independent, T7 RNA polymerase-mediated type I IFN induction was seen (Fig. 4C: no template and X8dT); the presence of a T7 RNA polymerase promoter-containing template was able to enhance the transcription dependent type I IFN induction (Fig. 4C: pBKS). When T7 RNA polymerase was expressed at lower levels, a complete template-dependent type I IFN induction could be seen (Fig. 4D; 100 ng T7 RNA polymerase).

These results demonstrated that not only exogenously added but also endogenously generated 5' triphosphate RNA is recognized via RIG-I, and confirmed that contaminants in the exogenously added 5' triphosphate RNA preparations are not involved in the induction of type I IFN.

### Example 6. RIG-I directly detects genomic triphosphate RNA from a mammalian negative strand RNA virus.

Characteristically, all NSV initiate viral RNA replication in a primer-independent manner, resulting in the presence of a triphosphate moiety at the 5' end of the viral genome (vRNA) or antigenome (cRNA). Moreover, in case of NSV with a nonsegmented genome (Order *Mononegavirales*), including for example the Paramyxoviruses and Rhabdoviruses, RNA transcription yields abundant amounts of short (approximately 60 nt) 5' triphosphate RNAs, known as leader RNAs, which are templated by the 3' end of vRNA (S. P. Whelan, et al. Current topics in microbiology and immunology 283, 61 (2004)). To assess the importance of NSV 5' triphosphate RNAs in the recognition of virus infection by RIG-I, rabies virus (RV), a prototype Rhabdovirus, was used.

Wildtype RV (SAD L16) encodes a potent antagonist of IFN induction, the phosphoprotein P, and therefore does not induce considerable IFN expression upon infection of epithelial cells. In contrast, a RV mutant genetically engineered to express little P (SAD ΔPLP) is an efficient inducer of IFN (K. Brzozka, et al. Journal of virology 79, 7673 (Jun, 2005); K. Brzozka, et al. Journal of virology 80, 2675 (Mar, 2006)). To confirm that RIG-I is involved in the recognition of RV infection, Vero cells were infected with the IFN-inducing RV, SAD ΔPLP, in the absence or presence of transfected RIG-I or RIG-IC (a dominant negative truncation mutant of RIG-I). SAD ΔPLP infection triggered a potent IFN-response which could be further enhanced by the overexpression of RIG-I and strongly suppressed by RIG-IC (Fig. 5A).

These results indicated that RIG-I is required for the initiation of an IFN-response upon RV infection, as has been observed for other NSV, VSV and Flu (H. Kato et al., Nature 441, 101 (Apr 9, 2006)).

To address whether RV RNA itself or viral replication is recognized via RIG-I, RNA was isolated from RV infected BSR cells and subsequently transfected into HEK 293T cells. RNA from RV-infected cells, but not RNA from non-infected cells, induced a potent IFN-β response (Fig. 5B). Moreover, the observed IFN- p production was completely abrogated the isolated RNA was dephosphorylated by CIAP prior to transfection (Fig. 5B), indicating that the 5' triphosphate group was required for recognition.

The RNA of NSV and of NSV-infected cells is not considered infectious and does not allow the initiation of a replicative cycle. The fact that RNA from RV SAD L16-infected cells was equally potent in terms of IFN- p induction as RNA from RV SAD ΔPLP-infected cells indicated that little or no productive translation and replication was initiated via the transfection of the respective RNA isolates.

Nevertheless, to completely rule out that replication of RV was required to trigger a typle I IFN response, full-length RNA from virions was isolated and assessed for its capability of inducing type I IFN expression. Transfection of 200 ng of purified RV RNA effectively stimulated type I IFN induction in HEK 293T cells and dephosphorylation of the genomic RV RNA completely abrogated the IFN response. An *in vitro* transcribed ssRNA corresponding to the 58-nucleotide long RV leader RNA confirmed recognition of and potent type I IFN induction by viral ssRNA.

Altogether, these results demonstrated that RIG-I directly recognizes genomic RNA from RV independent of replication and that this recognition is abolished if the 5' end of the RNA is dephosphorylated.

### Example 7. 5' triphosphate RNA directly binds to RIG-I.

The fact that RIG-I is required for the recognition of 5' triphosphate RNA provides no evidence that RIG-I is the receptor for 5' triphosphate RNA. To identify the receptor for 5' triphosphate RNA, *in vitro* binding assays was carried out to test the ability of 5' triphosphate RNA to pull down RIG-I or RIG-IC, the RNA binding domain of RIG-I.

RNA oligonucleotides with 3' terminal biotin tags were generated and incubated with whole cell lysate from HEK 293 cells overexpressing full length RIG-I, RIG-I CARD2 (the second CARD of RIG-I) or RIG-I Δ Helicase_C (RIG-I devoid of the predicted helicase superfamily c-terminal domain). Subsequently streptavidin beads were used to pull down the biotin tags on the 5' triphosphate RNA oligonucleotides.

Whereas the biotinylated 5' triphosphate oligonucleotide (tri-G-AC-U-Bio) was able to immunoprecipitate full length RIG-I (Fig. 6A, third panel, middle part), it was not very effective at pulling down truncated versions of RIG-I, CARD2 and RIG-I Δ Helicase_C (Fig. 6A, third panel left an right part). Unbiotinylated control RNA oligonucleotide (tri-G-AC-U) did not immunoprecipitate RIG-I. Purified RIG-IC was also efficiently pulled down by 5' triphosphate RNA oligonucleotides (Fig. 6B, second lane). If the initial 5' triphosphate group of the RNA oligonucleotide was enzymatically removed prior to incubation with RIG-I, no coprecipitation was seen (Fig. 6B, fourth lane).

These results indicated that 5'triphosphate RNA directly binds to full length RIG-I or RIG-IC, i.e., RIG-I is the direct receptor responsible for the recognition of 5' triphosphate RNA.

### Example 8. 5' adenosine triphosphate RNA oligonucleotides are superior to 5' guanosine triphosphate RNA oligonucleotides in inducing IFN-α production.

The classical *in vitro* transcription system makes use of the T7 RNA polymerase consensus promoter (J. J. Dunn, F. W. Studier, J Mol Biol 166, 477 (Jun 5, 1983)). Transcription under this promoter is initiated by GTP and usually requires two or more consecutive guanosines at the 5' end of RNA for efficient transcription. Nevertheless, it is possible to use a promoter system for T7 RNA polymerase which initiates with a 5' ATP (F. Huang et al. Biochemistry 39, 15548 (Dec 19, 2000)). Using this system, the role of the initial 5' guanosine in the type I-IFN inudcing activity of 5' triphosphate RNA oligonucleotides was assessed. RNA9.2s (RNA9.2s-0A) was used as a reference oligonucleotide since it starts with a 5' adenosine.

Comparing RNA9.2s-0A (5' ATP) with RNA9.2s-1G (5' GTP), which is shifted one base downstream of the corresponding human TLR9 mRNA, the latter showed a reduction of approximately 25% in IFN-α induction (Fig. 12, upper panel). Four bases further downstream of the human TLR9 mRNA, another 19-mer oligonucleotide could be transcribed which initiated with a 5' adenosine (RNA9.2s-5A). RNA9.2s-5A paralleled RNA9.2-0A in terms of IFN-α induction.

A second set of experiments corroborated these findings: comparison of the *in vitro* transcribed 35-mer RNA oligonucleotide AΦ6.5-35n (5' ATP) with GΦ6.5-35n (5' GTP) revealed a clear superiority of the transcript initiated with an adenosine in inudcing type I IFN, even though these oligonucleotides share more than 97 % homology in sequence (Fig. 12, lower panel).

Together, these findings indicated that RNA transcripts initiated with a 5' adenosine are more potent in terms of IFN-α induction than those initiated with a 5' guanosine.

### Example 9: The IFN-α-inducing activity of adenosine-initiated 5'-triphosphate RNA oligonucleotide depends on its 5' nucleotide sequence.

Adenosine-initiated triphosphate RNA oligonucleotides with all possible base permutations (A, C, G and U) of the 2nd, 3rd and 4th position of the sequence (5'-> 3') (Table 2) were generated via in vitro transcription. Subsequently monocytes from three independent donors were isolated and transfected with the respective RNA oligonucleotides. 36 hours after transfection, supernatants were analyzed for IFN-α production. The obtained IFN-α induction levels of all oligonucleotides were normalized to the mean induction level of all oligonucleotides (= 100%). The obtained normalized induction levels of all three donors were summarized as mean values ± SEM (Figure 13).

It is clear from Figure 13 that adenosine-initiated, *in vitro* transcribed RNA oligonucleotides having identical 3' sequence but different nucleotides at the 2nd, 3rd and 4th positions have different levels of IFN-α-inducing activity. The 5' 4-nucleotide sequences which confer the highest IFN-α-inducing activity include AAGU, AAAG, AUGG, AUUA, AACG, AUGA, AGUU, AUUG, AACA, AGAA, AGCA, AACU, AUCG, AGGA, AUCA, AUGC, AGUA, AAGC, AACC, AGGU, AAAC, AUGU, ACUG, ACGA, ACAG, AAGG, ACAU, ACGC, AAAU, ACGG, AUUC, AGUG, ACAA, AUCC, AGUC.

**Table 2**

| All Oligos share the same sequence except the 2nd, 3rd and 4th position (5'-ANNNGGGGACACACACACACACACACACAC-3') | | |
|---|---|---|
| | IFN-α induction (*100%) | |
| Sequence | mean | SEM |
| AGGG | 0,22 | 0,05 |
| AAUA | 0,40 | 0,07 |
| AGAU | 0,48 | 0,04 |
| AGAG | 0,50 | 0,06 |
| AGCG | 0,52 | 0.01 |
| AGAC | 0,62 | 0,10 |
| ACUA | 0,62 | 0,05 |
| ACUU | 0,66 | 0,01 |
| AAUU | 0,67 | 0,03 |
| AGCU | 0,69 | 0,01 |
| AAAA | 0,73 | 0,09 |
| ACCG | 0,73 | 0,03 |
| AUAG | 0,76 | 0,07 |
| ACCU | 0,76 | 0,01 |
| ACGU | 0,77 | 0,02 |
| ACCA | 0,79 | 0,01 |
| AUAA | 0,82 | 0,13 |
| AGCC | 0,87 | 0,04 |
| AUAU | 0,89 | 0,03 |
| ACCC | 0,89 | 0,01 |
| AGGC | 0,91 | 0,02 |
| AAUC | 0,94 | 0,05 |
| AUCU | 0,94 | 0,03 |
| AAGA | 0,95 | 0,19 |
| ACAC | 0,95 | 0,08 |
| AAUG | 0,96 | 0,07 |
| ACUC | 0,98 | 0,04 |
| AUUU | 0,99 | 0,06 |
| AUAC | 0,99 | 0,07 |
| AGUC | 1,00 | 0,08 |
| AUCC | 1,01 | 0,07 |
| ACAA | 1,01 | 0,08 |
| AGUG | 1,01 | 0,12 |
| AUUC | 1,03 | 0,07 |
| ACGG | 1,03 | 0,05 |
| AAAU | 1,04 | 0,19 |
| ACGC | 1,08 | 0,07 |
| ACAU | 1,11 | 0,09 |
| AAGG | 1,11 | 0,22 |
| ACAG | 1,12 | 0,01 |
| ACGA | 1,14 | 0,02 |
| ACUG | 1,14 | 0,08 |
| AUGU | 1,15 | 0,17 |
| AAAC | 1,15 | 0,09 |
| AGGU | 1,18 | 0,11 |
| AACC | 1,20 | 0,19 |
| AAGC | 1,22 | 0,13 |
| AGUA | 1,22 | 0,12 |
| AUGC | 1,23 | 0,10 |
| AUCA | 1,24 | 0,09 |
| AGGA | 1,27 | 0,05 |
| AUCG | 1,28 | 0,12 |
| AACU | 1,29 | 0,13 |
| AGCA | 1,29 | 0,15 |
| AGAA | 1,29 | 0,14 |
| AACA | 1,30 | 0,19 |
| AUUG | 1,31 | 0,11 |
| AGUU | 1,32 | 0,15 |
| AUGA | 1,32 | 0,01 |
| AACG | 1,34 | 0,15 |
| AUUA | 1,36 | 0,03 |
| AUGG | 1,38 | 0,10 |
| AAAG | 1,40 | 0,15 |
| AAGU | 1,40 | 0,10 |

### Example 10: The IFN-α-inducing activity of bacterial RNA is only partially dependent on the presence of 5' triphosphate.

As shown in Figure 9, total bacterial RNA is capable of inducing IFN-α production from monocytes.

To determine whether the IFN-α-inducing activity of bacterial RNA is due to the presence of the 5' triphosphate, total RNA was isolated from *E. coli* bacteria strain DH10B, either treated or not treated with CIAP to dephosphorylate the 5' end, and subsequently transfected into purified monocytes (200 ng of RNA). IFN-α production was analyzed 24 hours after stimulation.

As controls, Tri-GFPa was prepared via *in vitro* transcription, either treated or not treated with CIAP to dephosphorylate the 5' end, and subsequently transfected into purified monocytes (200 ng of RNA). IFN-α production was analyzed 24 hours after stimulation.

As previously shown in Example 2 and Figure 2C, the removal of 5' triphosphate from *in vitro* transcribed RNA oligonucleotides almost completely abolish the ability of the oligonucleotides to induce IFN-α from monocytes (Figure 14B). In contrast, the removal of 5' triphosphate from total bacterial RNA reduced the amount of IFN-α induced from monocytes by less than 30% (Figure 14A).

Therefore, 5' triphosphate is only one of the molecular features which are responsible for the ability of bacterial RNA to induce IFN-α.

### Example 11. In vitro-transcribed 5' triphosphate siRNA causes target gene knockdown and IFN-β induction.

SiRNAs specific for murine BCL-2 were designed using the algorithm provided by Dharmacon Research (Reynolds et al. 2005) and selected for high immunostimulatory activity according to the method developed by the present inventor (European patent application no. 05 020 020.3; PCT/EP2006/008980) (Figure 15A).

Murine B16 melanoma cells transfected with these BCL-2-specific siRNAs (2.1, 2.2 and 2.3) which were prepared by synthetic means had significantly reduced BCL-2 protein expression (Figure 16A).

The same siRNAs, when prepared by *in vitro* transcription and therefore contained 5' triphosphate (e.g., 3p-2.2), were equally effective at BCL-2 protein knockdown (Figure 16B).

The 5' triphosphate siRNA was not only able to cause target gene knockdown upon introduction into B16 cells, but also activation of the IFN-β promoter either in the presence or the absence of exogenous expression of RIG-I (Figure 16C and 16D). However, the exogenous expression of a truncated version of RIG-I, RIG-IC, which lacks the N-terminal CARD domain, abolished the IFN-β-inducing activity of the 5' triphosphate siRNA (Figure 16C), suggesting that endogenous RIG-I is involved in recognition of 5' triphosphate siRNA in B16 cells and the subsequent activation of the IFN-β promoter.

As shown before, both single-stranded and double-stranded 5' phosphate RNA was able to activate the IFN-β promoter (Figure 16C and 16D). In contrast, poly I:C was not able to activate the IFN-β promoter in B16 cells.

As expected, a positive control plasmid IRF3-5D which drives the expression of a constitutively active IRF3 activates the IFN-β promoter.

### Example 12. 5' triphosphate-siRNA induces apoptosis in tumor cells, but not in primary non-tumor cells.

B16 melanoma cells transfected with *in vitro* transcribed siRNA 2.2 (3p-2.2) underwent apoptosis 24 hours after transfection (Figure 17). In contrast, the transfection of synthetic siRNA 2.2 (Syn 2.2) which does not contain the 5' triphosphate, did not induce apoptosis of B16 cells (Figure 17). Interestingly, a 5' triphosphate-containing control RNA, 3p-GA (5'-TRI-GGGGGGGGGGGAAAA-3'), which does not downregulate BCL-2 expression, also induced apoptosis of B16 cells 24 hours after transfection (Figure 17).

This suggest that the induction of apoptosis in B16 cells by 3p-2.2 was not solely due to its ability to downregulate BCL-2, an anti-apoptotic gene. Rather, the presence of 5' triphosphate alone is capable of inducing apoptosis in tumor cells.

In contrast to B16 melanoma cells, human primary peripheral blood mononuclear cells (PBMCs) were not susceptible to apoptosis induced by RNA oligonucleotides containing 5' triphosphate (Figure 17B).

This suggests tumor cells are more susceptible than non-tumor cells to apoptosis induced by 5' triphosphate RNA, including 5' triphosphate siRNA. Therefore, 5' triphosphate RNA may be useful in the treatment of tumors.

### Example 13. 5' triphophate siRNA induces IFN-α secretion in distinct subsets of human and murine immune cells in vitro.

Transfection of 5' phosphate-containing RNA into isolated primary human immune cells revealed that IFN-α production can be induced in distinct subsets of immune cells, including myeloid dendritic cells (MDC), monocytes, naive B cells and memory B cells, and to a lesser degree, in plasmacytoid dendritic cells (PDC) (Figure 18). In contrast, a small molecule ligand which activates TLR7 and TLR8, R848, and a CpG-containing DNA oligonucleotide, CpG 2216 (5'-ggGGGACGATCGTCgggggG-3') were able to induce IFN-α production in human PDC but not MDC, monocytes, naive B cells or memroy B cells (Figure 18).

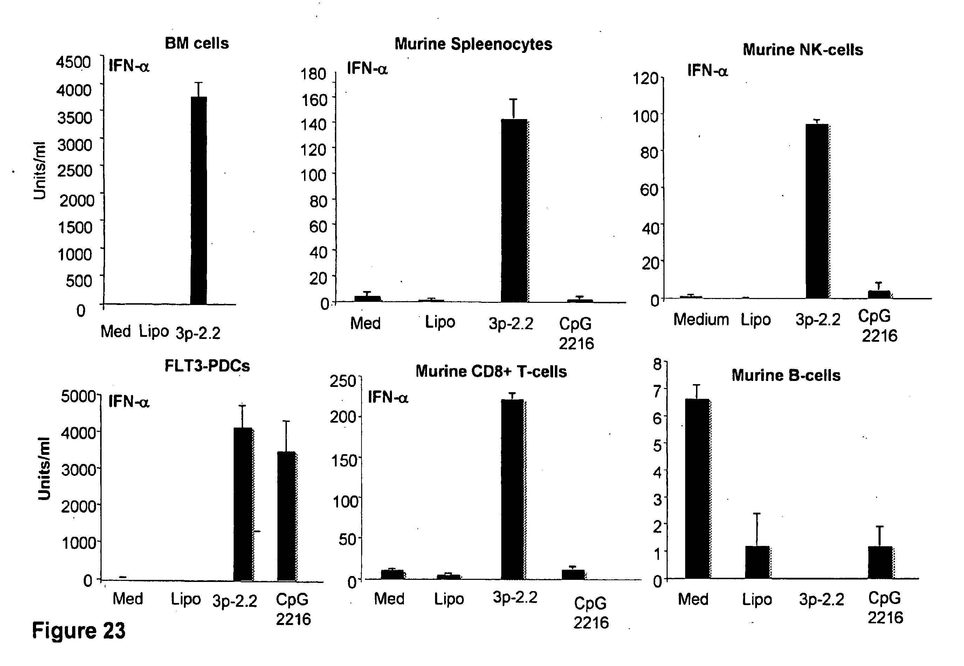

In contrast, trasnfection of 5' phosphate-containing RNA into isolated primary murine immune cells led to IFN-α production in slightly different subsets of immune cells: high amount of IFN-α was induced in PDC, but not at all in B cells (Figure 23). Furthermore, IFN-α induction was seen in bone marrow (BM) cells, splenocytes, NK cells and CD8+ T cells (Figure 23).

This difference in cell specificity in IFN-α induction by 5' triphosphate RNA, long ds RNA or CpG-containing DNA confirms that different cellular receptors are involved in ligand recognition and signal transduction.

### Example 15. 5' triphophate siRNA induces activation of and cytokine production in distinct subsets of murine immune cells in vivo in a dose- and time-dependent manner.

Mice injected with a 5' triphosphate-containing siRNA, 3p-2.2 complexed with jetPEI™ produced a number of cytokines, in particular, Th1 cytokines, including IFN-α, IL-12p40 and IFN-γ as early as 6 hours after injection (Figure 19 A-C). The amount of cytokines produced depended on the amount of 5' triphosphate siRNA injected (Figure 20A). The induced cytokine production can be observed as long as 24 hours after 5' triphosphate RNA injection (Figure 21 and data not shown).

Following the injection of 5' triphosphate-containing siRNA, activation of distinct subsets of immune cells, including MDC, PDC, CD4+ T cells, CD8+ T cells, and NK cells, can be observed as long as 48 hours after injection (Figure 20B). Furthermore, the degree of activation depended on the dose of 5' triphosphate RNA injected (Figure 20C).

This confirms the *in vitro* finding that 5' triphosphate RNA is capable of activating a wide range of immune cells, in contrast to TLR-7 and/or TLR-8 ligands.

### Example 16. Immune activation induced by 5' triphophate siRNA is TLR7-independent.

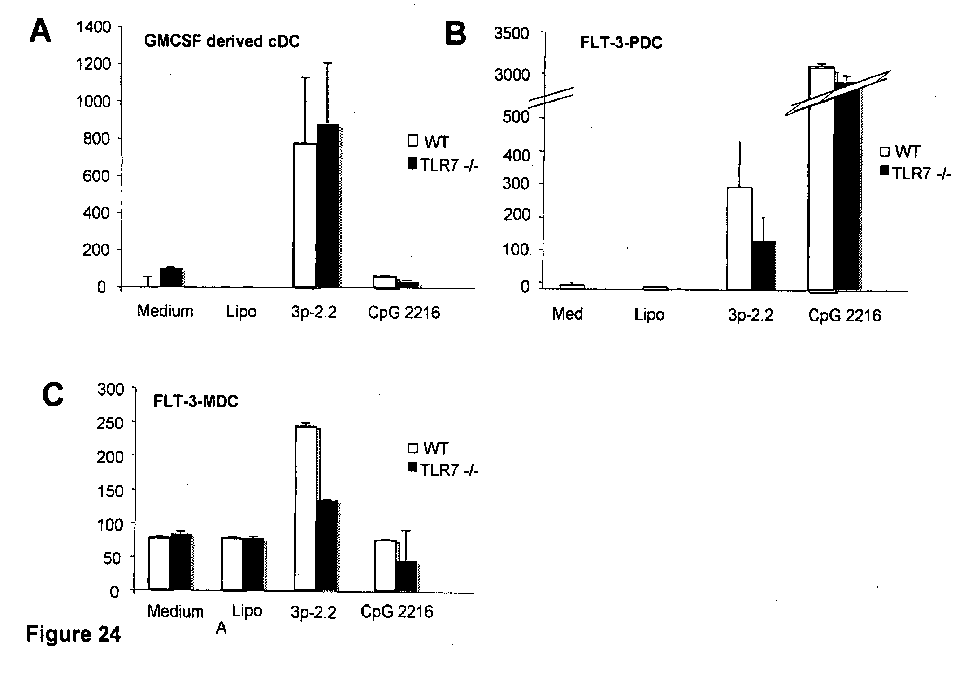

Transfection of 5' triphosphate RNA complexed with Lipofectamine into conventional dendritic cells (cDC), plasmacytoid dendritic cells (PDC), and myeloid dendritic cells (MDC) from wild-type C57/BL6 and TLR7-/- mice revealed that IFN-α production induced by 5' triphosphate RNA *in vitro* was largely TLR7-indpendent (Figure 24).

Furthermore, injection of 5' triposphate siRNA (50 µg) complexed to jetPEI™ in TLR7-/- mice induced IFN-α production, albeit in a reduced amount than in wild-type C57/BL6 control mice (Figure 19D), suggesting that the recognition of 5' triphosphate RNA and the subsequent induction of IFN-α production *in vivo* is at least partially TLR7-independent.

### Example 17. 5' triposphate siRNA induces moderate thrombocytopenia and leukopenia in vivo.

48 hours after injection with 50 µg of 5' triphosphate siRNA (3p-2.2) complexed with jet-PEITM, moderate thrombocytopenia and leukopenia were observed (Figure 22). A similar degree of thrombocytopenia and leukopenia was observed after the injection of a synthetic siRNA, Syn 2.2 (Figure 22).

### Example 18. In vivo distribuition of jetPEI™-complexed siRNA.

As early as 3 hours after Intravenous injection of FITC-labeled siRNA (50 µg) complexed with jetPEI™ (Biomol) into mice, FITC-labeled siRNA could be found in the liver, spleen, and lung of the injected animal (Figure 25).

### Example 19. In vivo delivery of RIG-I ligand alone or in combination with TLR-7 ligand reduces melanoma lung metastases.

B16 is a spontaneous murine melanoma and was cultivated in Dulbecco's modified Eagle's medium (PAN, Aidenbach, Germany) supplemented with 10% fetal calf serum (FCS), 3 mM L-glutamine, 100 U/ml penicillin and 100 µg/ml streptomycin. Engraftment of melanoma in the lungs was done by i.v. injection of 4x10⁵ B16 melanoma cells. On day 3, 6 and 9, tumor-bearing mice were injected with 200 µl of nucleic acids (50µg each) complexed with jetPEI in the retro-orbital vein. Nucleic acids tested included CpG 1826, poly (I:C), poly(A) (a synthetic 19mer RNA), RIG-I L (3p-GA, 5'-TRI-GGGGGGGGGGGAAAAAAAAAAAA-3') and TLR7-RIG-I L (TLR7 and RIG-1 double ligand, 3p-2.2). Control groups received 5% glucose. 14 days after challenge, the number of macroscopically visible melanoma metastases on the surface of the lungs was counted with the help of a dissecting microscope. Experiments were done in groups of five mice and repeated two to four times.

The *in vivo* delivery of CpG containg DNA olignucleotide in combination with poly I:C reduced the number of lung metastases significantly (Figure 26). The *in vivo* delivery of a RIG-I ligand, a 5' triphosphate RNA oligonucleotide, 3p-GA, also reduced the number of lung metastases significantly (Figure 26). The tumor-inhibitory activity of the RIG-I ligand was further enhanced when nucleotide motifs recognized by TLR7 were also present (i.e., when the 5' triphosphate RNA also served as a TLR7 ligand) as in the case of 3p-2.2 (Figure 26).

This confirms the effectivenss of 5' triphosphate RNA as an anti-tumor therapeutic agent.

## Claims

1. A pharmaceutical composition comprising an oligonucleotide wherein the oligonucleotide comprises a 5' triphosphate, wherein the oligonucleotide comprises at least 1, preferably at least 3, more preferably at least 6 ribonucleotide(s) at the 5' end, and wherein the oligonucleotide is at least 12, preferably at least 18, more preferably at least 20, and even more preferably at least 21 nucleotides in length, wherein the first ribonucleotide at the 5' end of the oligonucleotide comprises an adenine, wherein the oligonucleotide is free of modifications, said modifications being 2'-O-methylated NTP.

2. The pharmaceutical composition of claim 1, wherein the sequence of the first 4 nucleotides at the 5' end of the oligonucleotide is selected from: AAGU, AAAG, AUGG, AUUA, AACG, AUGA, AGUU, AUUG, AACA, AGAA, AGCA, AACU, AUCG, AGGA, AUCA, AUGC, AGUA, AAGC, AACC, AGGU, AAAC, AUGU, ACUG, ACGA, ACAG, AAGG, ACAU, ACGC, AAAU, ACGG, AUUC, AGUG, ACAA, AUCC, AGUC, wherein the sequence listing is in the 5' -> 3' direction.

3. The pharmaceutical composition of any of claims 1-2, wherein the oligonucleotide is furthermore free of pseudouridine, 2-thiouridine, and 2'-Fluorine-dNTP modifications.

4. The pharmaceutical composition of any of claims 1-3, further comprising a complexation agent.

5. The pharmaceutical composition of any of claims 1-3, wherein the oligonucleotide is comprised in a viral vector.

6. A combined preparation comprising an oligonucleotide as defined in any of claims 1-3 and an immunostimulatory agent, wherein the oligonucleotide and the agent are for simultaneous, separate or sequential administration.

7. The combined preparation of claim 6, further comprising an anti-viral agent and/or anti-bacterial agent and/or anti-tumor agent.

8. A combined preparation comprising an oligonucleotide as defined in any of claims 1-3 and an anti-viral agent and/or anti-bacterial agent and/or anti-tumor agent, wherein the oligonucleotide and the agent are for simultaneous, separate or sequential administration.

9. The combined preparation of any of claims 6-8, further comprising retinoic acid and/or type I IFN.

10. The combined preparation of any of claims 6-9, further comprising a complexation agent.

11. The combined preparation of any of claims 6-9, wherein the oligonucleotide is comprised in a viral vector.

12. A pharmaceutical package comprising the pharmaceutical composition of any of claims 1-7 or the combined preparation of any of claims 6-11 and an instruction for use.

13. An oligonucleotide defined in any of claims 1-3 for use in stimulating an anti-viral response or an anti-bacterial response in a vertebrate animal.

14. The oligonucleotide use of claim 13, wherein the anti viral response or anti-bacterial response comprises type I IFN production, IL-18 production, and/or IL-1β production.

15. An oligonucleotide as defined in any of claims 1-3 for use in preventing and/or treating diseases and/or disorders selected from the group consisting of viral infection, bacterial infection, tumor, immunosuppression and immunodeficiency in a vertebrate animal.

16. The oligonucleotide for use of claim 15, wherein the oligonucleotide or the precursor thereof is to be used in combination with an anti-viral agent and/or an anti-bacterial agent and/or an anti-tumor agent and/or an anti-tumor therapy.

17. An oligonucleotide defined in any of claims 1-3 and at least one antigen for use in inducing an immune response against the at least one antigen in a vertebrate animal.

18. The oligonucleotide for use of claim 17, wherein the oligonucleotide or the precursor thereof is covalently linked to the at least one antigen.

19. An oligonucleotide, as defined in any of claims 1-3 for use in inducing apoptosis of a tumor cell.

20. The oligonucleotide, for use of any of claims 13-19, wherein the oligonucleotide or the precursor thereof is used in combination with a complexing agent.

21. The oligonucleotide for use of any of claims 13-19, wherein the oligonucleotide or the precursor thereof is comprised in a viral vector.

22. The oligonucleotide for use of any of claims 13-21, wherein the oligonucleotide or precursor thereof is to be used in combination with an immunostimulatory agent.

23. The oligonucleotide for use of any of claims 13-22, wherein the oligonucleotide is to be used in combination with retinoic acid and/or type I IFN.

24. An in vitro method for stimulating an anti-viral or an anti-bacterial response in a cell, comprising the steps of:
(a) mixing an oligonucleotide as defined in any of claims 1-3 with a complexation agent; and
(b) contacting a cell with the mixture of (a), wherein the cell expresses RIG-I and/or NALP-3.

25. The in vitro method of claim 24, wherein the anti-viral response comprises type I IFN production, IL-18 production, and/or IL-1β production.

## Patentansprüche

1. Pharmazeutische Zusammensetzung umfassend: ein Oligonukleotid, worin das Oligonukleotid ein 5'- Triphosphat umfasst, wobei das Oligonukleotid mindestens 1, vorzugsweise mindestens 3, stärker bevorzugt mindestens 6 Ribonukleotid(e) am 5'- Ende umfasst, und wobei das Oligonukleotid mindestens 12, vorzugsweise mindestens 18, stärker bevorzugt mindestens 20, und noch stärker bevorzugt mindestens 21 Nukleotide in der Länge umfasst, wobei das erste Ribonukleotid am 5'- Ende des Oligonukleotids ein Adenin umfasst, wobei das Oligonukleotid frei von Modifikationen ist, wobei die genannte Modifikation 2'-O-methyliertes NTP ist.

2. Pharmazeutische Zusammensetzung nach Anspruch 1, wobei die Sequenz der ersten 4 Nukleotide am 5'- Ende des Oligonukleotids ausgewählt sind aus: AAGU, AAAG, AUGG, AUUA, AACG, AUGA, AGUU, AUUG, AACA, AGAA, AGCA, AACU, AUCG, AGGA, AUCA, AUGC, AGUA, AAGC, AACC, AGGU, AAAC, AUGU, ACUG, ACGA, ACAG, AAGG, ACAU, ACGC, AAAU, ACGG, AUUC, AGUG, ACAA, AUCC, AGUC, wobei das Sequenzprotokoll in der 5' -> 3'-Richtung ist.

3. Pharmazeutische Zusammensetzung nach einem der Ansprüche 1-2, wobei das Oligonukleotid weiterhin frei von Pseudouridin-, 2-Thiouridin- und 2'-Fluorin-dNTP- Modifikationen ist.

4. Pharmazeutische Zusammensetzung nach einem der Ansprüche 1-3, die weiterhin einen Komplexbildner umfasst.

5. Pharmazeutische Zusammensetzung nach einem der Ansprüche 1-3, wobei das Oligonukleotid von einem viralen Vektor umfasst ist.

6. Kombinierte Präparation, die ein Oligonukleotid wie in einem der Ansprüche 1-3 definiert und ein immunstimulierendes Mittel umfasst, wobei das Oligonukleotid und das Mittel gleichzeitig, getrennt oder hintereinander verabreicht werden.

7. Die kombinierte Präparation nach Anspruch 6, die weiterhin ein anti-virales Mittel und/oder ein anti-bakterielles Mittel und/oder anti-Tumormittel umfasst.

8. Kombinierte Präparation, die ein Oligonukleotid wie in einem der Ansprüche 1-3 definiert und ein anti-virales Mittel und/der anti-bakterielles Mittel und/oder anti-Tumormittel umfasst, wobei das Oligonukleotid und das Mittel gleichzeitig, getrennt oder hintereinander verabreicht werden.

9. Kombinierte Präparation nach einem der Ansprüche 6-8, die weiterhin Retinolsäure und/oder Typ I IFN umfasst.

10. Kombinierte Präparation nach einem der Ansprüche 6-9, die weiterhin einen Komplexbildner umfasst.

11. Kombinierte Präparation nach einem der Ansprüche 6-9, wobei das Oligonukleotid von einem viralen Vektor umfasst ist.

12. Pharmazeutische Packung, die die pharmazeutische Zusammensetzung nach einem der Ansprüche 1-7 oder die kombinierte Präparation nach einem der Ansprüche 6-11 und Instruktionen für deren Verwendung umfasst.

13. Oligonukleotid wie in einem der Ansprüche 1-3 definiert, zur Verwendung bei der Stimulierung einer anti-viralen Reaktion oder einer anti-bakteriellen Reaktion in einem Vertebraten.

14. Oligonukleotid zur Verwendung nach Anspruch 13, wobei die anti-virale Reaktion oder antibakterielle Reaktion die Typ I IFN-Produktion, IL-18-Produktion und/oder IL-1β-Produktion umfasst.

15. Oligonukleotid wie in einem der Ansprüche 1-3 definiert, zur Verwendung bei der Prävention und/oder Behandlung von Krankheiten und/oder Funktionsstörungen ausgewählt aus der Gruppe bestehend aus viralen Infektionen, bakteriellen Infektionen, Tumoren, Immunsuppression und Immundefizienz in einem Vertebraten.

16. Oligonukleotid zur Verwendung nach Anspruch 15, wobei das Oligonukleotid oder der Vorläufer davon in Kombination mit einem anti-viralen Mittel und/oder einem anti-bakteriellen Mittel und/oder einem anti-Tumormittel und/oder einer anti-Tumortherapie verwendet werden soll.

17. Oligonukleotid wie in einem der Ansprüche 1-3 definiert und mindestens ein Antigen für die Verwendung bei der Induzierung einer Immunantwort gegen mindestens ein Antigen in einem Vertebraten.

18. Oligonukleotid zur Verwendung nach Anspruch 17, wobei das Oligonukleotid oder der Vorläufer davon kovalent mit mindestens einem Antigen verknüpft ist.

19. Oligonukleotid wie in einem der Ansprüche 1-3 definiert, zur Verwendung bei der Induzierung von Apoptose eines Tumors.

20. Oligonukleotid zur Verwendung nach einem der Ansprüche 13-19, wobei das Oligonukleotid oder der Vorläufer davon in Kombination mit einem Komplexbildner verwendet wird.

21. Oligonukleotid zur Verwendung nach einem der Ansprüche 13-19, wobei das Oligonukleotid oder der Vorläufer davon von einem viralen Vektor umfasst ist.

22. Oligonukleotid zur Verwendung nach einem der Ansprüche 13-21, wobei das Oligonukleotid oder der Vorläufer davon in Kombination mit einem immunstimulierenden Mittel verwendet werden soll.

23. Oligonukleotid zur Verwendung nach einem der Ansprüche 13-22, wobei das Oligonukleotid in Kombination mit Retinolsäure und/oder Typ I IFN verwendet werden soll.

24. *In vitro-* Verfahren zur Stimulierung einer anti-viralen oder einer anti-bakteriellen Reaktion in einer Zelle, umfassend die Schritte:
(a) Mischen eines Oligonukleotid wie in einem der Ansprüche 1-3 definiert mit einem Komplexbildner; und
(b) in Kontakt bringen der Zelle mit der Mischung aus (a), wobei die Zelle RIG-I und/oder NALP-3 exprimiert.

25. *In vitro-* Verfahren nach Anspruch 24, wobei die anti-virale Reaktion Typ I IFN- Produktion, IL-18- Produktion und/oder IL-1β- Produktion umfasst.

## Revendications

1. Composition pharmaceutique comprenant un oligonucléotide, dans laquelle l'oligonucléotide comprend un 5'-triphosphate, dans laquelle l'oligonucléotide comprend au moins un, de préférence au moins trois, plus préférentiellement au moins six ribonucléotides à l'extrémité 5', et dans laquelle l'oligonucléotide a une longueur d'au moins douze, de préférence d'au moins dix-huit, plus préférablement d'au moins vingt, et encore plus préférablement d'au moins vingt et un nucléotides, dans laquelle le premier ribonucléotide à l'extrémité 5' de l'oligonucléotide comprend une adénine, et dans laquelle l'oligonucléotide est exempt de modifications, lesdites modifications étant un NTP 2'-O-méthylé.

2. Composition pharmaceutique selon la revendication 1, dans laquelle la séquence des quatre premiers nucléotides à l'extrémité 5' de nucléotide est choisie parmi : AAGU, AAAG, AUGG, AUUA, AACG, AUGA, AGUU, AUUG, AACA, AGAA, AGCA, AACU, AUCG, AGGA, AUCA, AUGC, AGUA, AAGC, AACC, AGGU, AAAC, AUGU, ACUG, ACGA, ACAG, AAGG, ACAU, ACGC, AAAU, ACGG, AUUC, AGUG, ACAA, AUCC, AGUC, le listing de séquence étant donné dans la direction 5'→3'.

3. Composition selon l'une des revendications 1 et 2, dans laquelle l'oligonucléotide est en outre exempt de modifications pseudo-uridine, 2-thiouridine, et 2'-fluor-dNTP.

4. Composition selon l'une des revendications 1 à 3, comprenant en outre un agent complexant.

5. Composition pharmaceutique selon l'une des revendications 1 à 3, dans laquelle l'oligonucléotide est compris dans un vecteur viral.

6. Préparation combinée comprenant un oligonucléotide tel que défini dans l'une des revendications 1 à 3 et un agent immunostimulant, dans laquelle l'oligonucléotide et l'agent sont destinés à être administrés simultanément, séparément ou séquentiellement.

7. Préparation combinée selon la revendication 6, comprenant en outre un agent antiviral et/ou un agent antibactérien et/ou un agent antitumoral.

8. Préparation combinée comprenant un oligonucléotide tel que défini dans l'une des revendications 1 à 3 et un agent antiviral et/ou un agent antibactérien et/ou un agent antitumoral, dans laquelle l'oligonucléotide et l'agent sont destinés à être administrés simultanément, séparément ou séquentiellement.

9. Préparation combinée selon l'une des revendications 6 à 8, comprenant en outre de l'acide rétinoïque et/ou de l'IFN de type I.

10. Préparation combinée selon l'une des revendications 6 à 9, comprenant en outre un agent complexant.

11. Préparation combinée selon l'une des revendications 6 à 9, dans laquelle l'oligonucléotide est compris dans un vecteur viral.

12. Conditionnement pharmaceutique comprenant la composition pharmaceutique de l'une des revendications 1 à 7 ou la préparation combinée de l'une quelconque des revendications 6 à 11 et des instructions d'utilisation.

13. Un oligonucléotide tel que défini dans l'une des revendications 1 à 3 pour utilisation dans la stimulation d'une réponse antivirale ou d'une réponse antibactérienne chez un animal vertébré.

14. L'utilisation d'un oligonucléotide selon la revendication 13, dans laquelle la réponse antivirale ou la réponse antibactérienne comprend la production d'IFN de type I, la production d'IL-18, et/ou la production d'IL-1β.

15. Un oligonucléotide tel que défini dans l'une quelconque des revendications 1 à 3 pour utilisation dans la prévention et/ou le traitement de maladies et/ou des troubles choisis dans le groupe formé par une infection virale, une infection bactérienne, une tumeur, une immunosuppression et une immunodéficience chez un animal vertébré.

16. L'oligonucléotide pour utilisation selon la revendication 15, ledit oligonucléotide ou son précurseur étant destiné à être utilisé en combinaison avec un agent antiviral et/ou un agent antibactérien et/ou un agent antitumoral et/ou une thérapie antitumorale.

17. Un oligonucléotide selon l'une des revendications 1 à 3 et au moins un antigène pour utilisation dans l'induction d'une réponse immunitaire contre l'au moins un antigène chez un animal vertébré.

18. L'oligonucléotide pour utilisation selon la revendication 17, ledit oligonucléotide ou son précurseur étant lié de manière covalente à l'au moins un antigène.

19. Un oligonucléotide tel que défini selon l'une des revendications 1 à 3 pour utilisation dans l'induction de l'apoptose d'une cellule tumorale.

20. L'oligonucléotide pour utilisation selon l'une quelconque des revendications 13 à 19, ledit oligonucléotide ou son précurseur étant utilisé en combinaison avec un agent complexant.

21. L'oligonucléotide pour utilisation selon l'une quelconque des revendications 13 à 19, ledit oligonucléotide ou son précurseur étant compris dans un vecteur viral.

22. L'oligonucléotide pour utilisation selon l'une quelconque des revendications 13 à 21, ledit oligonucléotide ou son précurseur étant destiné à être utilisé en combinaison avec un agent immunostimulant.

23. L'oligonucléotide pour utilisation selon l'une des revendications 13 à 22, ledit oligonucléotide étant destiné à être utilisé en combinaison avec de l'acide rétinoïque et/ou de l'IFN de type I.

24. Une méthode in vitro pour stimuler une réponse antivirale ou antibactérienne dans une cellule, comprenant les étapes consistant à :
(a) mélanger un oligonucléotide tel que défini dans l'une des revendications 1 à 3 avec un agent complexant ; et
(b) mettre une cellule en contact avec le mélange selon (a), laquelle cellule exprime RIG-I et/ou NALP-3.

25. La méthode in vitro selon la revendication 24, dans laquelle la réponse antivirale comprend la production d'IFN de type I, la production d'IL-18, et/ou la production d'IL-1β.


### SEQUENCE LISTING

<110> Hartmann, Gunther
<120> 5' Triphosphate oligonucleotide Induces Anti-Viral Response
<130> EP44328IFZ121pau
<150> EP 06 016 578.4
   <151> 2006-08-08
<160> 110
<170> PatentIn version 3.3
<210> 1
   <211> 35
   <212> DNA
   <213> Artificial
<220>
   <223> In vitro transcription templates
<400> 1
   cagtaatacg actcactatt aaaaagggga cacac 35
<210> 2
   <211> 35
   <212> DNA
   <213> Artificial
<220>
   <223> In vitro transcription templates
<400> 2
   cagtaatacg actcactatt aaacagggga cacac 35
<210> 3
   <211> 35
   <212> DNA
   <213> Artificial
<220>
   <223> In vitro transcription templates
<400> 3
   cagtaatacg actcactatt aaagagggga cacac 35
<210> 4
   <211> 35
   <212> DNA
   <213> Artificial
<220>
   <223> In vitro transcription templates
<400> 4
   cagtaatacg actcactatt aaatagggga cacac 35
<210> 5
   <211> 35
   <212> DNA
   <213> Artificial
<220>
   <223> In vitro transcription templates
<400> 5
   cagtaatacg actcactatt aacaagggga cacac 35
<210> 6
   <211> 35
   <212> DNA
   <213> Artificial
<220>
   <223> In vitro transcription templates
<400> 6
   cagtaatacg actcactatt aaccagggga cacac 35
<210> 7
   <211> 35
   <212> DNA
   <213> Artificial
<220>
   <223> In vitro transcription templates
<400> 7
   cagtaatacg actcactatt aacgagggga cacac 35
<210> 8
   <211> 35
   <212> DNA
   <213> Artificial
<220>
   <223> In vitro transcription templates
<400> 8
   cagtaatacg actcactatt aactagggga cacac 35
<210> 9
   <211> 35
   <212> DNA
   <213> Artificial
<220>
   <223> In vitro transcription templates
<400> 9
   cagtaatacg actcactatt aagaagggga cacac 35
<210> 10
   <211> 35
   <212> DNA
   <213> Artificial
<220>
   <223> In vitro transcription templates
<400> 10
   cagtaatacg actcactatt aagcagggga cacac 35
<210> 11
   <211> 35
   <212> DNA
   <213> Artificial
<220>
   <223> In vitro transcription templates
<400> 11
   cagtaatacg actcactatt aaggagggga cacac 35
<210> 12
   <211> 35
   <212> DNA
   <213> Artificial
<220>
   <223> In vitro transcription templates
<400> 12
   cagtaatacg actcactatt aagtagggga cacac 35
<210> 13
   <211> 35
   <212> DNA
   <213> Artificial
<220>
   <223> In vitro transcription templates
<400> 13
   cagtaatacg actcactatt aataagggga cacac 35
<210> 14
   <211> 35
   <212> DNA
   <213> Artificial
<220>
   <223> In vitro transcription templates
<400> 14
   cagtaatacg actcactatt aatcagggga cacac 35
<210> 15 .
   <211> 35
   <212> DNA
   <213> Artificial
<220>
   <223> In vitro transcription templates
<400> 15
   cagtaatacg actcactatt aatgagggga cacac 35
<210> 16
   <211> 35
   <212> DNA
   <213> Artificial
<220>
   <223> In vitro transcription templates
<400> 16
   cagtaatacg actcactatt aattagggga cacac 35
<210> 17
   <211> 35
   <212> DNA
   <213> Artificial
<220>
   <223> In vitro transcription templates
<400> 17
   cagtaatacg actcactatt acaaagggga cacac 35
<210> 18
   <211> 35
   <212> DNA
   <213> Artificial
<220>
   <223> In vitro transcription templates
<400> 18
   cagtaatacg actcactatt acacagggga cacac 35
<210> 19
   <211> 35
   <212> DNA
   <213> Artificial
<220>
   <223> In vitro transcription templates
<400> 19
   cagtaatacg actcactatt acagagggga cacac 35
<210> 20
   <211> 35
   <212> DNA
   <213> Artificial
<220>
   <223> In vitro transcription templates
<400> 20
   cagtaatacg actcactatt acatagggga cacac 35
<210> 21
   <211> 35
   <212> DNA
   <213> Artificial
<220>
   <223> In vitro transcription templates
<400> 21
   cagtaatacg actcactatt accaagggga cacac 35
<210> 22
   <211> 35
   <212> DNA
   <213> Artificial
<220>
   <223> In vitro transcription templates
<400> 22
   cagtaatacg actcactatt acccagggga cacac 35
<210> 23
   <211> 35
   <212> DNA
   <213> Artificial
<220>
   <223> In vitro transcription templates
<400> 23
   cagtaatacg actcactatt accgagggga cacac 35
<210> 24
   <211> 35
   <212> DNA
   <213> Artificial
<220>
   <223> In vitro transcription templates
<400> 24
   cagtaatacg actcactatt acctagggga cacac 35
<210> 25
   <211> 35
   <212> DNA
   <213> Artificial
<220>
   <223> In vitro transcription templates
<400> 25
   cagtaatacg actcactatt acgaagggga cacac 35
<210> 26
   <211> 35
   <212> DNA
   <213> Artificial
<220>
   <223> In vitro transcription templates
<400> 26
   cagtaatacg actcactatt acgcagggga cacac 35
<210> 27
   <211> 35
   <212> DNA
   <213> Artificial
<220>
   <223> In vitro transcription templates
<400> 27
   cagtaatacg actcactatt acggagggga cacac 35
<210> 28
   <211> 35
   <212> DNA
   <213> Artificial
<220>
   <223> In vitro transcription templates
<400> 28
   cagtaatacg actcactatt acgtagggga cacac 35
<210> 29
   <211> 35
   <212> DNA
   <213> Artificial
<220>
   <223> In vitro transcription templates
<400> 29
   cagtaatacg actcactatt actaagggga cacac 35
<210> 30
   <211> 35
   <212> DNA
   <213> Artificial
<220>
   <223> In vitro transcription templates
<400> 30
   cagtaatacg actcactatt actcagggga cacac 35
<210> 31
   <211> 35
   <212> DNA
   <213> Artificial
<220>
   <223> In vitro transcription templates
<400> 31
   cagtaatacg actcactatt actgagggga cacac 35
<210> 32
   <211> 35
   <212> DNA
   <213> Artificial
<220>
   <223> In vitro transcription templates
<400> 32
   cagtaatacg actcactatt acttagggga cacac 35
<210> 33
   <211> 35
   <212> DNA
   <213> Artificial
<220>
   <223> In vitro transcription templates
<400> 33
   cagtaatacg actcactatt agaaagggga cacac 35
<210> 34
   <211> 35
   <212> DNA
   <213> Artificial
<220>
   <223> In vitro transcription templates
<400> 34
   cagtaatacg actcactatt agacagggga cacac 35
<210> 35
   <211> 35
   <212> DNA
   <213> Artificial
<220>
   <223> In vitro transcription templates
<400> 35
   cagtaatacg actcactatt agagagggga cacac 35
<210> 36
   <211> 35
   <212> DNA
   <213> Artificial
<220>
   <223> In vitro transcription templates
<400> 36
   cagtaatacg actcactatt agatagggga cacac 35
<210> 37
   <211> 35
   <212> DNA
   <213> Artificial
<220>
   <223> In vitro transcription templates
<400> 37
   cagtaatacg actcactatt agcaagggga cacac 35
<210> 38
   <211> 35
   <212> DNA
   <213> Artificial
<220>
   <223> In vitro transcription templates
<400> 38
   cagtaatacg actcactatt agccagggga cacac 35
<210> 39
   <211> 35
   <212> DNA
   <213> Artificial
<220>
   <223> In vitro transcription templates
<400> 39
   cagtaatacg actcactatt agcgagggga cacac 35
<210> 40
   <211> 35
   <212> DNA
   <213> Artificial
<220>
   <223> In vitro transcription templates
<400> 40
   cagtaatacg actcactatt agctagggga cacac 35
<210> 41
   <211> 35
   <212> DNA
   <213> Artificial
<220>
   <223> In vitro transcription templates
<400> 41
   cagtaatacg actcactatt aggaagggga cacac 35
<210> 42
   <211> 35
   <212> DNA
   <213> Artificial
<220>
   <223> In vitro transcription templates
<400> 42
   cagtaatacg actcactatt aggcagggga cacac 35
<210> 43
   <211> 35
   <212> DNA
   <213> Artificial
<220>
   <223> In vitro transcription templates
<400> 43
   cagtaatacg actcactatt agggagggga cacac 35
<210> 44
   <211> 35
   <212> DNA
   <213> Artificial
<220> .
   <223> In vitro transcription templates
<400> 44
   cagtaatacg actcactatt aggtagggga cacac 35
<210> 45
   <211> 35
   <212> DNA
   <213> Artificial
<220>
   <223> In vitro transcription templates
<400> 45
   cagtaatacg actcactatt agtaagggga cacac 35
<210> 46
   <211> 35
   <212> DNA
   <213> Artificial
<220>
   <223> In vitro transcription templates
<400> 46
   cagtaatacg actcactatt agtcagggga cacac 35
<210> 47
   <211> 35
   <212> DNA
   <213> Artificial
<220>
   <223> In vitro transcription templates
<400> 47
   cagtaatacg actcactatt agtgagggga cacac 35
<210> 48
   <211> 35
   <212> DNA
   <213> Artificial
<220>
   <223> In vitro transcription templates
<400> 48
   cagtaatacg actcactatt agttagggga cacac 35
<210> 49
   <211> 35
   <212> DNA
   <213> Artificial
<220>
   <223> In vitro transcription templates
<400> 49
   cagtaatacg actcactatt ataaagggga cacac 35
<210> 50
   <211> 35
   <212> DNA
   <213> Artificial
<220>
   <223> In vitro transcription templates
<400> 50
   cagtaatacg actcactatt atacagggga cacac 35
<210> 51
   <211> 35
   <212> DNA
   <213> Artificial
<220>
   <223> In vitro transcription templates
<400> 51
   cagtaatacg actcactatt atagagggga cacac 35
<210> 52
   <211> 35
   <212> DNA
   <213> Artificial
<220>
   <223> In vitro transcription templates
<400> 52
   cagtaatacg actcactatt atatagggga cacac 35
<210> 53
   <211> 35
   <212> DNA
   <213> Artificial
<220>
   <223> In vitro transcription templates
<400> 53
   cagtaatacg actcactatt atcaagggga cacac 35
<210> 54
   <211> 35
   <212> DNA
   <213> Artificial
<220>
   <223> In vitro transcription templates
<400> 54
   cagtaatacg actcactatt atccagggga cacac 35
<210> 55
   <211> 35
   <212> DNA
   <213> Artificial
<220>
   <223> In vitro transcription templates
<400> 55
   cagtaatacg actcactatt atcgagggga cacac 35
<210> 56
   <211> 35
   <212> DNA
   <213> Artificial
<220>
   <223> In vitro transcription templates
<400> 56
   cagtaatacg actcactatt atctagggga cacac 35
<210> 57
   <211> 35
   <212> DNA
   <213> Artificial
<220>
   <223> In vitro transcription templates
<400> 57
   cagtaatacg actcactatt atgaagggga cacac 35
<210> 58
   <211> 35
   <212> DNA
   <213> Artificial
<220>
   <223> In vitro transcription templates
<400> 58
   cagtaatacg actcactatt atgcagggga cacac 35
<210> 59
   <211> 35
   <212> DNA
   <213> Artificial
<220>
   <223> In vitro transcription templates
<400> 59
   cagtaatacg actcactatt atggagggga cacac 35
<210> 60
   <211> 35
   <212> DNA
   <213> Artificial
<220>
   <223> In vitro transcription templates
<400> 60
   cagtaatacg actcactatt atgtagggga cacac 35
<210> 61
   <211> 35
   <212> DNA
   <213> Artificial
<220>
   <223> In vitro transcription templates
<400> 61
   cagtaatacg actcactatt attaagggga cacac 35
<210> 62
   <211> 35
   <212> DNA
   <213> Artificial
<220>
   <223> In vitro transcription templates
<400> 62
   cagtaatacg actcactatt attcagggga cacac 35
<210> 63
   <211> 35
   <212> DNA
   <213> Artificial
<220>
   <223> In vitro transcription templates
<400> 63
   cagtaatacg actcactatt attgagggga cacac 35
<210> 64
   <211> 35
   <212> DNA
   <213> Artificial
<220>
   <223> In vitro transcription templates
<400> 64
   cagtaatacg actcactatt atttagggga cacac 35
<210> 65
   <211> 26
   <212> DNA
   <213> Artificial
<220>
   <223> In vitro transcription templates
<400> 65
   gtgtgtgtgt gtgtgtgtgt gtcccc 26
<210> 66
   <211> 32
   <212> DNA
   <213> Artificial
<220>
   <223> In vitro transcription templates
<400> 66
   tatagttcca caaaggcatc tatagtgagt cg 32
<210> 67
   <211> 32
   <212> DNA
   <213> Artificial
<220>
   <223> In vitro transcription templates
<400> 67
   atgcctttgt ggaactatac tatagtgagt cg 32
<210> 68
   <211> 32
   <212> DNA
   <213> Artificial
<220>
   <223> In vitro transcription templates
<400> 68
   tcaaacagag gtcgcatgcc tatagtgagt cg 32
<210> 69
   <211> 32
   <212> DNA
   <213> Artificial
<220>
   <223> In vitro transcription templates
<400> 69
   gcatgcgacc tctgtttgac tatagtgagt cg 32
<210> 70
   <211> 35
   <212> DNA
   <213> Artificial
<220>
   <223> In vitro transcription templates
<400> 70
   tttttttttt ttcccccccc ccctatagtg agtcg 35
<210> 71
   <211> 29
   <212> DNA
   <213> Artificial
<220>
   <223> In vitro transcription templates
<400> 71
   aaaaaaaaaa aaaaaaaaaa acctgtctc 29
<210> 72
   <211> 38
   <212> DNA
   <213> Artificial
<220>
   <223> In vitro transcription templates
<400> 72
   aaagtgtgtg tgtgtgtgtg tgtctatagt gagtcgta 38
<210> 73
   <211> 29
   <212> DNA
   <213> Artificial
<220>
   <223> In vitro transcription templates
<400> 73
   aaatgtgtgt gtgtgtgtgt gcctgtctc 29
<210> 74
   <211> 38
   <212> DNA
   <213> Artificial
<220>
   <223> In vitro transcription templates
<400> 74
   aagatgaact tcagggtcag cccctatagt gagtcgta 38
<210> 75
   <211> 38
   <212> DNA
   <213> Artificial
<220>
   <223> In vitro transcription templates
<400> 75
   aagctgaccc tgaagttcat cccctatagt gagtcgta 38
<210> 76
   <211> 43
   <212> DNA
   <213> Artificial
<220>
   <223> In vitro transcription templates
<400> 76
   aagctgaccc tgaagttcat ctgcaccact atagtgagtc gta 43
<210> 77
   <211> 41
   <212> DNA
   <213> Artificial
<220>
   <223> In vitro transcription templates
<400> 77
   aagctgaccc tgaagttcat ctgcacctat agtgagtcgt a 41
<210> 78
   <211> 43
   <212> DNA
   <213> Artificial
<220>
   <223> In vitro transcription templates
<400> 78
   aagtggtgca gatgaacttc agggtcagct atagtgagtc gta 43
<210> 79
   <211> 19
   <212> DNA
   <213> Artificial
<220>
   <223> In vitro transcription templates
<400> 79
   agtgagcgca acgcaatta 19
<210> 80
   <211> 33
   <212> DNA
   <213> Artificial
<220>
   <223> In vitro transcription templates
<400> 80
   attgaaggac aggttaagct atagtgagtc gta 33
<210> 81
   <211> 29
   <212> DNA
   <213> Artificial
<220>
   <223> In vitro transcription templates
<400> 81
   caccgcggtg gagctccaat tcgccctat 29
<210> 82
   <211> 33
   <212> DNA
   <213> Artificial
<220>
   <223> In vitro transcription templates
<400> 82
   cagtaatacg actcactata ggggaagcgg gca 33
<210> 83
   <211> 21
   <212> DNA
   <213> Artificial
<220>
   <223> In vitro transcription templates
<400> 83
   cagtaatacg actcactatt a 21
<210> 84
   <211> 33
   <212> DNA
   <213> Artificial
<220>
   <223> In vitro transcription templates
<400> 84
   cagtaatacg actcactatt agggaagcgg gca 33
<210> 85
   <211> 29
   <212> DNA
   <213> Artificial
<220>
   <223> In vitro transcription templates
<400> 85
   cccccccccc cccccccccc ccctgtctc 29
<210> 86
   <211> 19
   <212> DNA
   <213> Artificial
<220>
   <223> In vitro transcription templates
<400> 86
   cctcgaggtc gacggtatc 19
<210> 87
   <211> 22
   <212> DNA
   <213> Artificial
<220>
   <223> In vitro transcription templates
<400> 87
   cggataacaa tttcacacag ga 22
<210> 88
   <211> 19
   <212> DNA
   <213> Artificial
<220>
   <223> In vitro transcription templates
<400> 88
   cgggggatcc actagttct 19
<210> 89
   <211> 41
   <212> DNA
   <213> Artificial
<220>
   <223> In vitro transcription templates
<400> 89
   gggaagctga ccctgaagtt catcccctat agtgagtcgt a 41
<210> 90
   <211> 29
   <212> DNA
   <213> Artificial
<220>
   <223> In vitro transcription templates
<400> 90
   gggaagttca tcccctatag tgagtcgta 29
<210> 91
   <211> 35
   <212> DNA
   <213> Artificial
<220>
   <223> In vitro transcription templates
<400> 91
   gggaccctga agttcatccc ctatagtgag tcgta 35
<210> 92
   <211> 23
   <212> DNA
   <213> Artificial
<220>
   <223> In vitro transcription templates
<400> 92
   gggatcccct atagtgagtc gta 23
<210> 93
   <211> 32
   <212> DNA
   <213> Artificial
<220>
   <223> In vitro transcription templates
<400> 93
   gggctgaagt tcatccccta tagtgagtcg ta 32
<210> 94
   <211> 38
   <212> DNA
   <213> Artificial
<220>
   <223> In vitro transcription templates
<400> 94
   gggctgaccc tgaagttcat cccctatagt gagtcgta 38
<210> 95
   <211> 29
   <212> DNA
   <213> Artificial
<220>
   <223> In vitro transcription templates
<400> 95
   gggggggggg gggggggggg gcctgtctc 29
<210> 96
   <211> 26
   <212> DNA
   <213> Artificial
<220>
   <223> In vitro transcription templates
<400> 96
   gggttcatcc cctatagtga gtcgta 26
<210> 97
   <211> 31
   <212> DNA
   <213> Artificial
<220>
   <223> In vitro transcription templates
<400> 97
   ggtaattgaa ggacaggtta atagtgagtc g 31
<210> 98
   <211> 39
   <212> DNA
   <213> Artificial
<220>
   <223> In vitro transcription templates
<400> 98
   ggtgcagatg aacttcaggg tcagcttaat agtgagtcg 39
<210> 99
   <211> 17
   <212> DNA
   <213> Artificial
<220>
   <223> In vitro transcription templates
<400> 99
   taatacgact cactata 17
<210> 100
   <211> 33
   <212> DNA
   <213> Artificial
<220>
   <223> In vitro transcription templates
<400> 100
   tgatcggcta tggctggccg catgcccgct tcc 33
<210> 101
   <211> 31
   <212> DNA
   <213> Artificial
<220>
   <223> In vitro transcription templates
<400> 101
   ttgaaggaca ggttaagcta atagtgagtc g 31
<210> 102
   <211> 29
   <212> DNA
   <213> Artificial
<220>
   <223> In vitro transcription templates
<400> 102
   tttttttttt tttttttttt tcctgtctc 29
<210> 103
   <211> 19
   <212> RNA
   <213> Artificial
<220>
   <223> SiRNA oligonucleotide
<400> 103
   augccuuugu ggaacuaua 19
<210> 104
   <211> 19
   <212> RNA
   <213> Artificial
<220>
   <223> SiRNA oligonucleotide
<400> 104
   uauaguucca caaaggcau 19
<210> 105
   <211> 19
   <212> RNA
   <213> Artificial
<220>
   <223> SiRNA oligonucleotide
<400> 105
   gcaugcgacc ucuguuuga 19
<210> 106
   <211> 19
   <212> RNA
   <213> Artificial
<220>
   <223> SiRNA oligonucleotide
<400> 106
   ucaaacagag gucgcaugc 19
<210> 107
   <211> 19
   <212> RNA
   <213> Artificial
<220>
   <223> SiRNA oligonucleotide
<400> 107
   ggaugacuga guaccugaa 19
<210> 108
   <211> 19
   <212> RNA
   <213> Artificial
<220>
   <223> SiRNA oligonucleotide
<400> 108
   uucagguacu cagucaucc 19
<210> 109
   <211> 19
   <212> RNA
   <213> Artificial
<220>
   <223> control oligonucleotide
<400> 109
   aaaaaaaaaa aaaaaaaaa 19
<210> 110
   <211> 19
   <212> RNA
   <213> Artificial
<220>
   <223> SiRNA oligonucleotide
<400> 110
   agcuuaaccu guccuucaa 19